# EUROPÄISCHE PATENTSCHRIFT

(11) **EP 0 903 353 B1**
(45) Veröffentlichungstag und Bekanntmachung des Hinweises auf die Patenterteilung: **25.11.2009**
(21) Anmeldenummer: 98117231.5
(22) Anmeldetag: 11.09.1998
(51) Int. Cl.: C07K 5/02, A61K 38/06, A61K 38/07, C07K 5/10, C07K 5/08

(54) **Imidazolidinderivate, ihre Herstellung, ihre Verwendung und sie enthaltende pharmazeutische Präparate**
Imidazolidine derivates, their preparation and use, and pharmaceutical compositions containing them
Dérivés d'imidazoline, leur préparation et leur utilisation et compositions pharmaceutiques les contenant

(30) Priorität: 18.09.1997 DE 19741235
(43) Veröffentlichungstag der Anmeldung: 24.03.1999
(73) Patentinhaber: Sanofi-Aventis Deutschland GmbH, 65929 Frankfurt am Main (DE)
(72) Erfinder: Wehner, Volkmar Dr., 97657 Sandberg (DE); Stilz, Hans Ulrich Dr., 65929 Frankfurt (DE); Schmidt, Wolfgang Dr., 65929 Frankfurt (DE); Seiffge, Dirk Dr., 55246 Mainz-Kostheim (DE)

(56) Entgegenhaltungen:
- EP-A- 0 530 505
- EP-A- 0 566 919
- EP-A- 0 580 008
- EP-A- 0 584 694
- EP-A- 0 842 943
- EP-A- 0 842 944
- EP-A- 0 842 945
- WO-A-95/14008
- WO-A-95/15973
- DE-A- 19 515 177

## Beschreibung

Die vorliegende Erfindung betrifft neue Imidazolidinderivate der Formel I gemäß Anspruch 1, 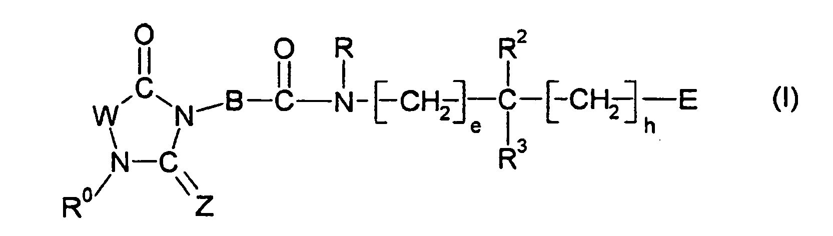 in der B, E, W, Z, R, R⁰, R², R³, e und h die unten angegebenen Bedeutungen haben. Die Verbindungen der Formel I sind wertvolle Arzneimittelwirkstoffe, die sich zum Beispiel zur Therapie und Prophylaxe von Entzündungserkrankungen, beispielsweise der rheumatoiden Arthritis, oder von allergischen Erkrankungen eignen. Die Verbindungen der Formel I sind Inhibitoren der Adhäsion und Migration von Leukozyten und/oder Antagonisten des zur Gruppe der Integrine gehörenden Adhäsionsrezeptors VLA-4. Sie eignen sich generell zur Therapie oder Prophylaxe von Krankheiten, die durch ein unerwünschtes Ausmaß an Leukozytenadhäsion und/oder Leukozytenmigration verursacht werden oder damit verbunden sind, oder bei denen Zell-Zell- oder Zell-Matrix-Interaktionen eine Rolle spielen, die auf Wechselwirkungen von VLA-4-Rezeptoren mit ihren Liganden beruhen. Die Erfindung betrifft weiterhin Verfahren zur Herstellung der Verbindungen der Formel I, ihre Verwendung in der Therapie und Prophylaxe der genannten Krankheitszustände und pharmazeutische Präparate, die Verbindungen der Formel I enthalten.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine αβ-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertetern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen-Rezeptor oder Vitronectin-Rezeptor ist VLA-4 kein typisches RGDbindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osborn et al., Cell 1989, 59, 1203). VCAM-1 ist, wie ICAM-1, ein Mitglied der Immunglobulin-Gen-Überfamilie. Identifiziert wurde VCAM-1 (zuerst bekannt als INCAM-110) als ein Adhäsionsmolekül, das auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1NLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/ VLA-4-Mechanismus die Funktion zu, derartige Zellen aus dem Blutstrom in Infektionsgebiete und Entzündungsherde zu rekrutieren (Elices et al., Cell 1990, 60, 577; Osbom, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertern Endothel unter anderem noch von den folgenden Zellen exprimiert: Myoblasten, lymphoiden dendritischen Zellen und Gewebsmakrophagen, rheumatoidem Synovium, Zytokin-stimutierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osbom et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen. Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-α4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (USSN 07/821,768; EP-A-626 861). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176, 1183), Arteriosklerose (O'Brien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osborn, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4/VCAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93/15764, WO-A-94/16094, WO-A-94/17828 und WO-A-95119790 beschrieben. In den Patentanmeldungen WO-A-94/15958, WO-A-95/15973, WO-A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung. Es besteht somit Bedarf nach VLA-4-Antagonisten mit einem günstigen Eigenschaftsprofil für einen Einsatz in der Therapie und Prophylaxe.

In der WO-A-95/14008, der WO-A-94121607, der WO-A-93118057, der EP-A-449 079, der EP-A-530 505 (US-A-5 389 614), der EP-A-566 919 (US-A-5 397 796), der EP-A-580 008 (US-A-5 424 293) und der EP-A-584 694 (US-A-5 554 594) sind substituierte 5-Ring-Heterocyclen beschrieben, die am N-terminalen Ende des Moleküls eine Amino-, Amidino- oder Guanidinofunktion aufweisen und die thrombozytenaggregationshemmende Wirkungen zeigen. In der EP-A-796 855 (europäische Patentanmeldung 97103712.2) sind weitere Heterocyclen beschrieben, die Inhibitoren der Knochenresorption sind. In der EP-A-842 943, EP-A-842 945 und EP-A-842 944 (deutsche Patentanmeldungen 19647380.2, 19647381.0 und 19647382.9) wird beschrieben; daß bestimmte Verbindungen aus dieser Reihe und bestimmte weitere Verbindungen überraschenderweise auch die Leukozytenadhäsion hemmen und VLA-4-Antagonisten sind. Nicht konkret offenbart werden in den genannten Anmeldungen aber die ausgewählten Verbindungen der Formel I, die sich durch ihren VLA-4-Antagonismus und/oder ihre hemmende Wirkung auf die Leukozytenadhäsion und Leukozytenmigration auszeichnen und die Gegenstand der vorliegenden Erfindung sind.

Die vorliegende Erfindung betrifft somit Verbindungen der Formel I gemäß Anspruch 1, 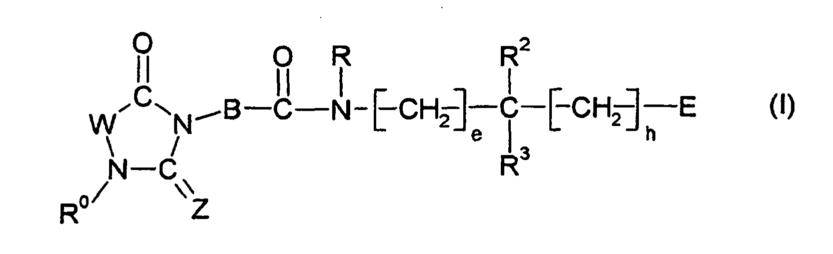 worin
- W: für R¹-A-C(R¹³) oder R¹-CH=C steht;
- Z: für Sauerstoff oder Schwefel steht;
- A: für eine direkte Bindung oder (C₁-C₂)-Alkylen steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
- E: Tetrazolyl, (R⁸O)₂P(O), HOS(O)₂, R⁹NHS(O), oder R¹⁰CO bedeutet;
- R: Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁- C₈)-alkyl bedeutet;
- R⁰: für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₄)-Aryl-(C₁-C₈)- alkyl, gegebenenfalls substituiertes Heteroaryl, im Heteroarylrest gegebenenfalls substituiertes Heteroaryl-(C₁-C₈)-alky), H-CO, (C₁-C₈)-Alkyl-CO, (C₃-C₁₂)- Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)- Tricycloalkyl-CO, (C₆-C₁₂)- Tricycoalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)- Cycloalkyl-S(O)ₙ, (C₃-C₁₂)-Cycloalkyl-(C₁-C₈)-alkyl-S(O)ₙ, (C₈-C₁₂)-Bicycloalkyl- S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆- C₁₂)-Tricycloalkyl-(C₁-C₈)-alkyl-S(O)ₙ, gegebenenfalls substituiertes (C₆-C₁₄)- Aryl-S(O)ₙ, im Arylrest gegebenenfalls substituiertes (C₆-C₁₄)-Aryl-(C₁-C₈)-alkyl- S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: für einen gegebenenfalls substituierten Rest aus der Reihe Phenyl, Furyl, Thienyl, Pyrrolyl, Imidazolyl und Pyridyl steht, wobei jeder dieser Reste auch benzanelliert sein kann;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, (C₃-C₈)-Cycloalkyl, (C₃-C₈)-Cycloalkyl-(C₁-C₈)-alkyl, (C₆- C₁₂)-Bicycloalkyl, (C₆-C₁₂)-Bicycloalkyl-(C₁-C₈)-alkyl, (C₆-C₁₂)- Tricycloalkyl, (C₆- C₁₂)-Tricycloalkyl-(C₁-C₈)-alkyl, (C₂-C₈)-Alkenyl, (C₂-C₈)-Alkinyl, R¹¹NH, CON(CH₃)R⁴, CONHR⁴, COOR¹⁵, CON(CH₃)R¹⁵ oder CONHR¹⁵ bedeutet;
- R⁴: Wasserstoff oder (C₁-C₁₀)-Alkyl bedeutet, das gegebenenfalls einfach oder mehrfach durch gleiche oder verschiedene Reste aus der Reihe Hydroxy, (C₁- C₈)-Alkoxy, R⁵, gegebenenfalls substituiertes (C₃-C₈)-Cycloalkyl, Hydroxycarbonyl, Aminocarbonyl, Mono- oder Di-((C₁-C₁₈)-aikyl)-aminocarbonyl, (C₆-C₁₄)-Aryl-(C₁-C₈)-alkoxycarbonyl, das im Arylrest auch substituiert sein kann, (C₁-C₈)-Alkoxycarbonyl, Het-CO, R⁶-CO, Tetrazolyl und Trifluormethyl substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl oder einen gegebenenfalls substituierten monocyclischen oder bicyclischen 5-gliedrigen bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, bedeutet;
- R⁶: den Rest einer natürlichen oder unnatürlichen Aminosäure, Iminosäure, gegebenenfalls N-(C₁-C₈)-alkylierten oder N-((C₆-C₁₄)-Aryl-(C₁-C₈)-alkylierten) Azaaminosäure, der im Arylrest auch substituiert kann, oder den Rest eines Dipeptids bedeutet, sowie deren Ester und Amide, wobei freie funktionelle Gruppen durch in der Peptidchemie übliche Schutzgruppen geschützt sein können;
- R⁸: Wasserstoff, (C₁-C₁₈)-Alkyl, gegebenenfalls substituiertes (C₆-C₁₄)-Aryl oder (C₆- C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, (C₁-C₈)- Alkylcarbonyloxy-(C₁-C₆)-alkoxy, (C₆-C₁₄)-Arylcarbonyloxy-(C₁-C₆)-alkoxy, Amino oder Mono- oder Di-((C₁C₁₈)-alkykl)-amino bedeutet;
- R¹¹: für Wasserstoff, R^{12a}, R^{12a}-CO, H-CO, R¹²-O-CO, R^{12b}-CO, R^{12b}-CS, R^{12a}-S(O)₂ oder R^{12b}-S(O)₂ steht;
- R^{12a}: (C₁-C₁₈)-Alkyl, (C₂-C₈)-Alkenyl, (C₂-C₈)-Alkinyl, (C₃-C₁₂)-Cycloalkyl, (C₃-C₁₂)- Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₈)-alkyl)-amino oder R^{12a}-NH bedeutet;
- R¹³: Wasserstoff, (C₁-C₆)-Alkyl, gegebenenfalls substituiertes (C₆-C₁₄)-Aryl, im Arylrest gegebenenfalls substituiertes (C₆-C₁₄)-Aryl-(C₁-C₆)-alkyl, (C₃-C₈)- Cycloalkyl oder (C₃-C₈)-Cyclo-(C₁-C₆)-alkyl bedeutet;
- R¹⁵: für R¹⁶-(C₁-C₆)-alkyl oder für R¹⁶ steht;
- R¹⁶: für einen 6-gliedrigen bis 24-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- Het: für den Rest eines über ein Ring-Stickstoffatom gebundenen 5-gliedrigen bis 10-gliedrigen, gesättigten monocyclischen oder polycyclischen Heterocyclus steht, der ein, zwei, drei oder vier gleiche oder verschiedene zusätzliche Ring- Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring- Stickstoffatomen gleiche oder verschiedene Reste aus der Reihe Wasserstoff, R^{h}, HCO, R^{h}CO und R^{h}O-CO als Substituenten stehen können und R^{h} für (C₁- C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆- C₁₄)-Aryl-(C₁-C₈)-alkyl steht;
- e und h: unabhängig voneinander für 0 oder 1 stehen;

in allen ihren stereoisomeren Formen und Mischungen davon in allen Verhältnissen, und ihre physiologisch verträglichen Salze.

Alkylreste können geradkettig oder verzweigt sein. Dies gilt auch, wenn sie Substituenten tragen oder als Substituenten anderer Reste auftreten, beispielsweise in Alkoxyresten, Alkoxycarbonylresten oder Arylalkylresten. Entsprechendes gilt für Alkylenreste. Beispiele für geeignete (C₁-C₁₈)-Alkylreste sind Methyl, Ethyl, n-Propyl, n-Butyl, n-Pentyl, n-Hexyl, n-Heptyl, n-Octyl, n-Decyl, n-Undecyl, n-Dodecyl, n-Tridecyl, n-Pentadecyl, n-Hexadecyl, n-Heptadecyl, n-Octadecyl, Isopropyl, Isobutyl, Isopentyl, Isohexyl, 3-Methylpentyl, Neopentyl, Neohexyl, 2,3,5-Trimethylhexyl, sec-Butyl, tert-Butyl, tert-Pentyl. Bevorzugte Alkylreste sind Methyl, Ethyl, n-Propyl, Isopropyl, n-Butyl, Isobutyl, sec-Butyl, tert-Butyl, n-Pentyl, Isopentyl, n-Hexyl und Isohexyl. Beispiele für Alkylenreste sind Methylen, Ethylen, Tri-, Tetra-, Penta- und Hexamethylen oder durch einen Alkylrest substituiertes Methylen oder Ethylen, zum Beispiel Methylen, das durch eine Methylgruppe, eine Ethylgruppe, eine Isopropylgruppe, eine Isobutylgruppe, eine tert-Butylgruppe, eine n-Pentylgruppe, eine Isopentylgruppe oder eine n-Hexylgruppe substituiert ist, oder zum Beispiel Ethylen, das sowohl an dem einem als auch an dem anderen Kohlenstoffatom oder auch an an beiden Kohlenstoffatomen substituiert sein kann.

Auch Alkenylreste und Alkenylenreste sowie Alkinylreste können geradkettig oder verzweigt sein. Beispiele für Alkenylreste sind Vinyl, 1-Propenyl, Allyl, Butenyl, 3-Methyl-2-butenyl, Beispiele für Alkenylenreste sind Vinylen oder Propenylen, für Alkinylreste Ethinyl, 1-Propinyl oder Propargyl.

Cycloalkylreste sind insbesondere Cyclopropyl, Cyclobutyl, Cyclopentyl, Cyclohexyl, Cycloheptyl, Cyclooctyl, Cyclononyl, Cyclodecyl, Cycloundecyl und Cyclododecyl, die aber auch beispielsweise durch (C₁-C₄)-Alkyl substituiert sein können. Als Beispiele für substituierte Cycloalkylreste seien 4-Methylcyclohexyl und 2,3-Dimethylcyclopentyl genannt. Analoges gilt für Cycloalkylenreste.

Bicycloalkylreste, Tricycloalkylreste und die für R¹⁶ stehenden 6-gliedrigen bis 24-gliedrigen bicyclischen und tricyclischen Reste werden formal durch Abstraktion eines Wasserstoffatoms aus Bicyclen bzw. Tricyclen erhalten. Die zugrunde liegenden Bicyclen und Tricyclen können als Ringglieder nur Kohlenstoffatome enthalten, es kann sich also um Bicycloalkane oder Tricycloalkane handeln, sie können aber im Falle der für R¹⁶ stehenden Reste auch ein bis vier gleiche oder verschiedene Heteroatome aus der Reihe Stickstoff, Sauerstoff und Schwefel enthalten, es kann sich also um Aza-, Oxa- und Thiabicyclo- und -tricycloalkane handeln. Sind Heteroatome enthalten, so sind bevorzugt ein oder zwei Heteroatome, insbesondere Stickstoffatome oder Sauerstoffatome, enthalten. Die Heteroatome können beliebige Positionen im bicyclischen bzw. tricyclischen Gerüst einnehmen, sie können sich in den Brücken oder im Falle von Stickstoffatomen auch an den Brückenköpfen befinden. Sowohl die Bicycloalkane und Tricycloalkane als auch ihre Hetero-Analoga können vollständig gesättigt sein oder eine oder mehrere Doppelbindungen enthalten; bevorzugt enthalten sie eine oder zwei Doppelbindungen oder sind insbesondere vollständig gesättigt. Sowohl die Bicycloalkane und Tricycloalkane als auch die Hetero-Analoga und sowohl die gesättigten als auch die ungesättigten Vertreter können unsubstituiert sein oder in beliebigen geeigneten Positionen durch eine oder mehrere Oxogruppen und/oder eine oder mehrere gleiche oder verschiedene (C₁-C₄)-Alkylgruppen, zum Beispiel Methylgruppen oder lsopropylgruppen, bevorzugt Methylgruppen, substituiert sein. Die freie Bindung des bicyclischen oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo-Position oder einer endo-Position.

Beispiele für Grundkörper bicyclischer Ringsysteme, von denen sich ein bicyclischer Rest ableiten kann, sind das Norbornan (= Bicyclo[2.2.1]heptan), das Bicyclo[2.2.2]octan und das Bicyclo[3.2.1]octan, Beispiele für Heteroatome enthaltende, für ungesättigte oder für substituierte Ringsysteme sind das 7-Azabicylo[2.2.1]heptan, das Bicyclo[2.2.2]oct-5-en und der Campher (= 1,7,7-Trimethyl-2-oxobicyclo[2.2.1]heptan).

Beispiele für Systeme, von denen sich ein tricyclischer Rest ableiten kann, sind das Twistan (= Tricyclo[4.4.0.0^{3.8}]decan), das Adamantan (= Tricyclo[3.3.1.1^{3,7}]decan), das Noradamantan (= Tricyclo[3.3.10^{3,7}]nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Bevorzugt leiten sich bicyclische oder tricyclische Reste von verbrückten Bicyclen bzw. Tricyclen ab, also von Systemen, in denen Ringe zwei oder mehr als zwei Atome gemeinsam haben. Bevorzugt sind, soweit nicht anders angegeben, weiterhin auch bicyclische oder tricyclische Reste mit 6 bis 18 Ringgliedern, besonders bevorzugt solche mit 6 bis 14 Ringgliedern, ganz besonders bevorzugt solche mit 7 bis 12 Ringgliedern.

Im einzelnen besonders bevorzugte bicyclische und tricyclische Reste sind der 2-Norbornylrest, sowohl derjenige mit der freien Bindung in der exo-Position als auch derjenige mit der freien Bindung in der endo-Position, der 2-Bicyclo[3.2.1]octylrest, der Adamantylrest, sowohl der 1-Adamantylrest als auch der 2-Adamantylrest, der Homoadamantylrest und der Noradamantylrest, zum Beispiel der 3-Noradamantylrest. Darüber hinaus bevorzugt sind der 1- und der 2-Adamantylrest.

(C₆-C₁₄)-Arylgruppen sind beispielsweise Phenyl, Naphthyl, zum Beispiel 1-Naphthyl und 2-Naphthyl, Biphenylyl, zum Beispiel 2-Biphenylyl, 3-Biphenylyl und 4-Biphenylyl, Anthryl oder Fluorenyl, (C₆-C₁₀)-Arylgruppen beispielsweise 1-Naphthyl, 2-Naphthyl und insbesondere Phenyl. Arylreste, insbesondere Phenylreste, können einfach oder mehrfach, bevorzugt einfach, zweifach oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Hydroxy-(C₁-C₄)-alkyl wie zum Beispiel Hydroxymethyl oder 1-Hydroxyethyl oder 2-Hydroxyethyl, Methylendioxy, Ethylendioxy, Formyl, Acetyl, Cyan, Hydroxycarbonyl, Aminocarbonyl, (C₁-C₄)-Alkoxycarbonyl, Phenyl, Phenoxy, Benzyl, Benzyloxy, Tetrazolyl substituiert sein. Entsprechendes gilt beispielsweise für Reste wie Arylalkyl oder Arylcarbonyl. Arylalkylreste sind insbesondere Benzyl sowie 1- und 2-Naphthylmethyl, 2-, 3- und 4-Biphenylylmethyl und 9-Fluorenylmethyl, die auch substituiert sein können. Substituierte Arylalkylreste sind beispielsweise durch einen oder mehrere (C₁-C₈)-Alkylreste, insbesondere (C₁-C₄)-Alkylreste, im Arylteil substituierte Benzylreste und Naphthylmethylreste, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7-und 8-Methyl-2-naphthylmethyl, durch einen oder mehrere (C₁-C₈)-Alkoxyreste, insbesondere (C₁-C₄)-Alkoxyreste, im Arylteil substituierte Benzylreste und Naphthylmethylreste, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, Nitrobenzylreste, zum Beispiel 2-, 3-und 4-Nitrobenzyl, Halobenzylreste, zum Beispiel 2-, 3- und 4-Chlorbenzyl und 2-, 3- und 4-Fluorbenzyl, 3,4-Dichlorbenzyl, Pentafluorbenzyl, Trifluormethylbenzylreste, zum Beispiel 3- und 4-Trifluormethylbenzyl oder 3,5-Bis(trifluormethyl)benzyl. Substituierte Arylalkylreste können aber auch unterschiedliche Substituenten aufweisen.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Zweifach substituiertes Phenyl kann also in der 2,3-Position, der 2,4-Position, der 2,5-Position, der 2,6-Position, der 3,4-Position oder der 3,5-Position, bezogen auf die Verknüpfungstelle, substituiert sein. Bevorzugt sind in zweifach substituierten Phenylresten die beiden Substituenten in der 3-Position und der 4-Position, bezogen auf die Verknüpfungsstelle, angeordnet. In dreifach substituierten Phenylresten können sich die Substituenten beispielsweise in der 2,3,4-Position, der 2,3,5-Position, der 2,4,5-Position, der 2,4,6-Position, der 2,3,6-Position oder der 3,4,5-Position befinden. Entsprechendes gilt für Phenylenreste, die zum Beispiel als 1,4-Phenylen oder als 1,3-Phenylen vorliegen können.

Phenylen-(C₁-C₃)-alkyl ist insbesondere Phenylenmethyl (-C₆H₄-CH₂-) und Phenylenethyl, (C₁-C₃)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Heteroaryl steht für einen monocyclischen oder polycyclischen aromatischen Rest mit 5 bis 14 Ringgliedern, der 1, 2, 3, 4 oder 5 Heteroatome als Ringglieder enthält. Beispiele für Heteroatome sind N, O und S. Sind mehrere Heteroatome enthalten, können diese gleich oder verschieden sein. Heteroarylreste können ebenfalls einfach oder mehrfach, bevorzugt einfach, zweifach oder dreifach, durch gleiche oder verschiedene Reste aus der Reihe (C₁-C₈)-Alkyl, insbesondere (C₁-C₄)-Alkyl, (C₁-C₆)-Alkoxy, insbesondere (C₁-C₄)-Alkoxy, Halogen, Nitro, Amino, Trifluormethyl, Hydroxy, Hydroxy-(C₁-C₄)-alkyl wie zum Beispiel Hydroxymethyl oder 1-Hydroxyethyl oder 2-Hydroxyethyl, Methylendioxy, Formyl, Acetyl, Cyan, Hydroxycarbonyl, Aminocarbonyl, (C₁-C₄)-Alkoxycarbonyl, Phenyl, Phenoxy, Benzyl, Benzyloxy, Tetrazolyl substituiert sein. Bevorzugt steht Heteroaryl für einen monocyclischen oder bicyclischen aromatischen Rest, der 1, 2, 3 oder 4, insbesondere 1, 2 oder 3, gleiche oder verschiedene Heteroatome aus der Reihe N, O und S enthält und der durch 1, 2, 3 oder 4, insbesondere 1, 2 oder 3, gleiche oder verschiedene Substituenten aus der Reihe (C₁-C₆)-Alkyl, (C₁-C₆)-Alkoxy, Fluor, Chlor, Nitro, Amino, Trifluormethyl, Hydroxy, Hydroxy-(C₁-C₄)-alkyl, (C₁-C₄)-Alkoxycarbonyl, Phenyl, Phenoxy, Benzyloxy und Benzyl substituiert sein kann. Besonders bevorzugt steht Heteroaryl für einen monocyclischen oder bicyclischen aromatischen Rest mit 5 bis 10 Ringgliedern, insbesondere für einen 5-gliedrigen bis 6-gliedrigen monocyclischen aromatischen Rest, der 1, 2 oder 3, insbesondere 1 oder 2, gleiche oder verschiedene Heteroatome aus der Reihe N, O und S enthält und durch 1 oder 2 gleiche oder verschiedene Substituenten aus der Reihe (C₁-C₄)-Alkyl, (C₁-C₄)-Alkoxy, Phenyl, Phenoxy, Benzyloxy und Benzyl substituiert sein kann.

Heterocyclen, die für monocylische oder bicyclische 5-gliedrige bis 12-gliedrige heterocyclische Ringe stehen, können aromatisch oder teilweise oder vollständig gesättigt sein. Sie können unsubstituiert sein oder an einem oder mehreren Kohlenstoffatomen oder an einem oder mehreren Stickstoffatomen durch gleiche oder verschiedene Substituenten substituiert sein, wie dies für den Rest Heteroaryl angegeben ist. Insbesondere kann der heterocyclische Ring an Kohlenstoffatomen einfach oder mehrfach durch gleiche oder verschiedene Reste aus der Reihe (C₁-C₈)-Alkyl, zum Beispiel (C₁-C₄)-Alkyl, (C₁-C₈)-Alkoxy, zum Beispiel (C₁-C₄)-Alkoxy wie Methoxy, Phenyl-(C₁-C₄)-alkoxy, zum Beispiel Benzyloxy, Hydroxy, Oxo, Halogen, Nitro, Amino oder Trifluormethyl substituiert sein, und/oder können Ring-Stickstoffatome in heterocyclischen Ringen wie auch in Heteroarylresten durch (C₁-C₈)-Alkyl, zum Beispiel (C₁-C₄)-Alkyl wie Methyl oder Ethyl, durch gegebenenfalls substituiertes Phenyl oder Phenyl-(C₁-C₄)-alkyl, zum Beispiel Benzyl, substituiert sein.

Beispiele für Heterocyclen, die dem Heteroarylrest oder dem Rest des monocyclilschen oder bicyclischen 5-gliedrigen bis 12-gliedrigen heterocyclischen Ring zugrunde liegen können, sind Pyrrol, Furan, Thiophen, Imidazol, Pyrazol, Oxazol, Isoxazol, Thiazol, Isothiazol, Tetrazol, Pyridin, Pyrazin, Pyrimidin, Indol, Isoindol, Indazol, Phthalazin, Chinolin, Isochinolin, Chinoxalin, Chinazolin, Cinnolin, β-Carbolin oder benzanellierte, cyclopenta-anellierte, cyclohexa-anellierte oder cyclohepta-anellierte Derivate dieser Heterocyclen.

Stickstoffheterocyclen können auch als N-Oxide vorliegen.

Reste, die für Heteroaryl oder den Rest eines monocyclischen oder bicyclischen 5-gliedrigen bis 12-gliedrigen heterocyclischen Ringes stehen können, sind beispielsweise 2- oder 3-Pyrrolyl, Phenylpyrrolyl, zum Beispiel 4- oder 5-Phenyl-2-pyrrolyl, 2-Furyl, 3-Furyl, 2-Thienyl, 3-Thienyl, 4-Imidazolyl, Methylimidazolyl, zum Beispiel 1-Methyl-2-, -4- oder -5-imidazolyl, 1,3-Thiazol-2-yl, 2-Pyridyl, 3-Pyridyl, 4-Pyridyl, N-Oxido-2-, -3- oder -4-Pyridyl, 2-Pyrazinyl, 2-, 4- oder 5-Pyrimidinyl, 2-, 3-oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3-oder4-Isochinolyl, 1-Oxo-1,2-dihydro-3-isochinolyl, 2-Chinoxalinyl, 2-Benzofuranyl, 2-Benzothienyl, 2-Benzoxazolyl oder 2-Benzothiazolyl oder, als Reste von teilhydrierten oder vollständig hydrierten heterocyclischen Ringen, beispielsweise auch Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl, Benzodioxolanyl.

Für den Rest Het stehende heterocylische Reste können an Kohlenstoffatomen und/oder Ring-Stickstoffatomen unsubstituiert oder einfach oder mehrfach, zum Beispiel zweifach, dreifach, vierfach oder fünffach, durch gleiche oder verschiedene Substituenten substituiert sein. Kohlenstoffatome können zum Beispiel durch (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Oxo, Cyan, Hydroxycarbonyl, Aminocarbonyl, (C₁-C₄)-Alkoxycarbonyl, Phenyl, Phenoxy, Benzyl, Benzyloxy, Tetrazolyl substituiert sein, insbesondere durch (C₁-C₄)-Alkyl, zum Beispiel Methyl, Ethyl oder tert-Butyl, (C₁-C₄)-Alkoxy, zum Beispiel Methoxy, Hydroxy, Oxo, Phenyl, Phenoxy, Benzyl, Benzyloxy. Schwefelatome können zum Sulfoxid oder zum Sulfon oxidiert sein. Beispiele für den Rest Het sind 1-Pyrrolidinyl, 1-Piperidinyl, 1-Piperazinyl, 4-substituiertes 1-Piperazinyl, 4-Morpholinyl, 4-Thiomorpholinyl, 1-Oxo-4-thiomorpholinyl, 1,1-Dioxo-4-thiomorpholinyl, Perhydroazepin-1-yl, 2,6-Dimethyl-1-piperidinyl, 3,3-Dimethyl-4-morpholinyl, 4-Isopropyl-2,2,6,6-tetramethyl-1-piperazinyl, 4-Acetyl-1-piperazinyl, 4-Ethoxycarbonyl-1-piperazinyl.

Die für R¹ stehenden heteroaromatischen Reste Furyl, Thienyl, Pyrrolyl, Imidazolyl und Pyridyl können über jedes der Kohlenstoffatome gebunden sein, es können also die Reste 2-Furyl, 3-Furyl, 2-Thienyl, 3-Thienyl, 2-Pyrrolyl, 3-Pyrrolyl, 2-Imidazolyl, 4-Imidazolyl, 5-Imidazolyl, 2-Pyridyl, 3-Pyridyl und 4-Pyridyl vorliegen. Der für R¹ stehende Phenylrest und die heteroaromatischen Reste können auch benzanelliert sein, R¹ kann also auch für Naphthyl, Benzo[b]furyl (= Benzofuryl), Benzo[c]furyl, Benzo[b]thienyl (= Benzothienyl), Benzo[c]thienyl, Indolyl, Benzimidazolyl, Chinolyl und Isochinolyl stehen, insbesondere für Naphthyl, Benzofuryl, Benzothienyl, Indolyl, Benzimidazolyl, Chinolyl und Isochinolyl. Die für R¹ stehenden benzanellierten Reste sind bevorzugt über ein Kohlenstoffatom im heterocyclischen Ring gebunden, wobei sie wiederum über jedes dieser Kohlenstoffatome gebunden sein können. Beispiele für solche für R¹ stehenden benzanellierten Reste sind 1-Naphthyl, 2-Naphthyl, 2-Benzofuryl, 3-Benzofuryl, 2-Benzothienyl, 3-Benzothienyl, 2-Indolyl, 3-Indolyl, 4-Indolyl, 5-Indolyl, 6-Indolyl, 7-Indolyl, 2-Benzimidazolyl, 2-Chinolyl, 3-Chinolyl, 4-Chinolyl, 1-Isochinolyl, 3-Isochinolyl oder 4-Isochinolyl.

Die für R¹ stehenden Reste können unsubstituiert sein oder in beliebigen Positionen durch einen oder mehrere, zum Beispiel einen, zwei, drei oder vier, gleiche oder verschiedene Substituenten substituiert sein. Dabei gelten die obigen Erläuterungen, zum Beispiel zu den Substituentenpositionen in Phenylresten und heterocyclischen Resten, entsprechend auch für die für R¹ stehenden Reste. Als Substituenten an Kohlenstoffatomen kommen also zum Beispiel (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Cyan, Formyl, Acetyl, Hydroxycarbonyl, Aminocarbonyl, (C₁-C₄)-Alkoxycarbonyl, Phenyl, Phenoxy, Benzyl, Benzyloxy und Tetrazolyl in Betracht, wobei diese Substituenten an Kohlenstoffatomen im heterocyclischen Ring und/oder an Kohlenstoffatomen in einem anellierten Benzolring stehen können. Stickstoffatome in Pyrrolylresten, Imidazolylresten und ihren benzanellierten Analogen können unsubstituiert sein oder insbesondere zum Beispiel durch (C₁-C₈)-Alkyl, zum Beispiel (C₁-C₄)-Alkyl wie Methyl oder Ethyl, durch gegebenenfalls substituiertes Phenyl oder Phenyl-(C₁-C₄)-alkyl, zum Beispiel Benzyl, oder zum Beispiel durch (C₁-C₄)-Alkyl-CO substituiert sein.

Der Substituent an einem für B stehenden substituierten Alkylenrest kann zum einen einen Cyclus enthalten, wenn es sich um einen Substituenten aus der Reihe (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆) handelt, und er kann zum anderen acyclisch sein, wenn es sich um einen Substituenten aus der Reihe (C₁-C₈)-Alkyl, (C₂-C₈)-Alkenyl und (C₂-C₈)-Alkinyl handelt. Die acyclischen Substituenten können 2, 3, 4, 5, 6, 7 oder 8 Kohlenstoffatome oder im Falle des gesättigten Alkylrests auch 1 Kohlenstoffatom enthalten. Im Falle der Alkenylreste und Alkinylreste kann sich die Doppelbindung oder Dreifachbindung in einer beliebigen Position befinden und im Falle der Doppelbindung cis-Konfiguration oder trans-Konfigurätion aufweisen. Wie oben erläutert, können diese Alkylreste, Alkenylreste und Alkinylreste geradkettig oder verzweigt sein.

Als Beispiele für Substituenten, die der für B stehende (C₁-C₆)-Alkylenrest tragen kann, seien insbesondere genannt Methyl, Ethyl, n-Propyl, n-Butyl, n-Pentyl, n-Hexyl, n-Heptyl, n-Octyl, Isopropyl, Isobutyl, Isopentyl, Isohexyl, sec-Butyl, tert-Butyl, tert-Pentyl, Neopentyl, Neohexyl, 3-Methylpentyl, 2-Ethylbutyl, Vinyl, Allyl, 1-Propenyl, 2-Butenyl, 3-Butenyl, 3-Methyl-2-butenyl, Ethinyl, 1-Propinyl, 2-Propinyl, 6-Hexinyl, Phenyl, Benzyl, 1-Phenylethyl, 2-Phenylethyl, 3-Phenylpropyl, 4-Biphenylylmethyl, Cyclopropyl, Cyclopropylmethyl, Cyclopentyl, Cyclohexyl, Cyclohexylmethyl, 2-Cyclohexylethyl, 3-Cyclooctylpropyl, 2-Pyridyl, 3-Pyridyl, 4-Pyridyl, 4-Pyridylmethyl, 2-(4-Pyridyl)ethyl, 2-Furylmethyl, 2-Thienylmethyl, 3-Thienylmethyl oder 2-(3-Indolyl)ethyl.

Halogen steht für Fluor, Chlor, Brom oder Iod, insbesondere für Fluor oder Chlor.

Der Rest einer Aminosäure, Iminosäure oder Azaaminosäure oder eines Dipeptids wird wie in der Peptidchemie üblich aus der entsprechenden Aminosäure, Iminosäure oder Azaaminosäure oder dem Dipeptid erhalten, indem von der N-terminalen Aminogruppe oder von der Iminogruppe formal ein Wasserstoffatom entfernt wird. Über die so entstehende freie Bindung an der Aminogruppe oder der Iminogruppe ist diese Gruppe dann peptidartig durch eine Amidbindung mit der CO-Gruppe in der Gruppe R⁶-CO verknüpft.

Die natürlichen und unnatürlichen Aminosäuren können in allen stereochemischen Formen vorliegen, beispielsweise in der D-Form, der L-Form oder in Form einer Mischung von Stereoisomeren, zum Beispiel in Form eines Racemats. Bevorzugte Aminosäuren sind α-Aminosäuren und β-Aminosäuren, besonders bevorzugt sind α-Aminosäuren. Als in Betracht kommende Aminosäuren seien beispielsweise genannt (vgl. Houben-Weyl, Methoden der organischen Chemie, Band 15/1 und 15/2. Georg Thieme Verlag, Stuttgart, 1974):
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure.

Steht R⁶ für den Rest einer natürlichen oder unnatürlichen α-Aminosäure, die am α-Kohlenstoffatom nicht verzweigt ist, das heißt, die am α-Kohlenstoffatom ein Wasserstoffatom trägt, dann liegt der Rest -N(R^{b})-CH(SC)-CO-L vor, in dem CO-L für die Säuregruppe der Aminosäure oder ein Derivat davon, zum Beispiel eine Estergruppe oder eine Amidgruppe, steht, R^{b} zum Beispiel für Wasserstoff und SC für die Seitenkette der α-Aminosäure steht, also zum Beispiel für einen der Substituenten, die in der α-Position der vorstehend genannten, in der α-Position unverzweigten α-Aminosäuren enthalten sind. Beispiele für Seitenketten sind Alkylreste, zum Beispiel die Methylgruppe im Alanin oder die Isopropylgrupppe im Valin, der Benzylrest im Phenylalanin, der Phenylrest im Phenylglycin, der 4-Aminobutylrest im Lysin oder die Hydroxycarbonylmethylgruppe in der Asparaginsäure. Solche Seitenketten und damit die Aminosäuren können im Sinne der vorliegenden Erfindung außer durch ihre chemische Struktur zum Beispiel auch aufgrund ihrer physikochemischen Eigenschaften zu einer Gruppe zusammengefaßt werden, beispielsweise können lipophile Seitenketten von hydrophilen Seitenketten, die polare Gruppen enthalten, unterschieden werden. Beispiele für lipophile Seitenketten, die in für R⁶ stehenden Aminosäuren enthalten sein können, sind Alkylreste, Arylalkylreste oder Arylreste.

Azaaminosäuren sind natürliche oder unnatürliche Aminosäuren, in denen eine CH-Einheit durch ein Stickstoffatom ersetzt ist, beispielsweise in α-Aminosäuren der Zentralbaustein 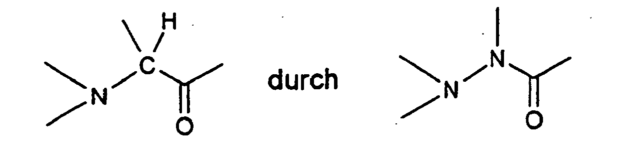 ersetzt ist.

Als Rest einer Iminosäure kommen insbesondere Reste von Heterocyclen aus der folgenden Gruppe in Betracht: Pyrrolidin-2-carbonsäure; Piperidin-2-carbonsäure; 1,2,3,4-Tetrahydroisochinotin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1^{6.9}]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

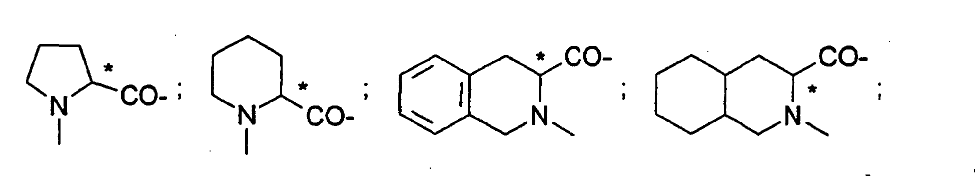

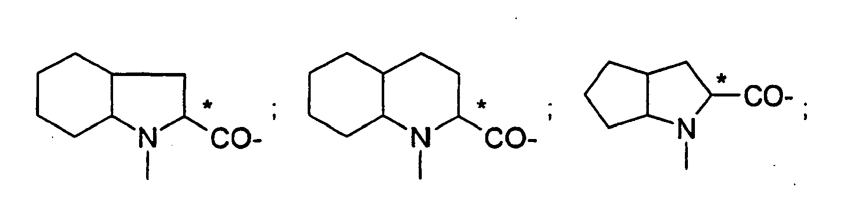

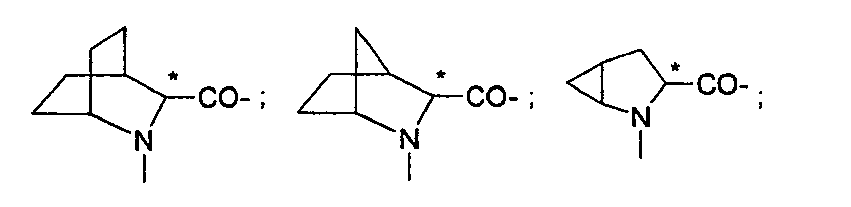

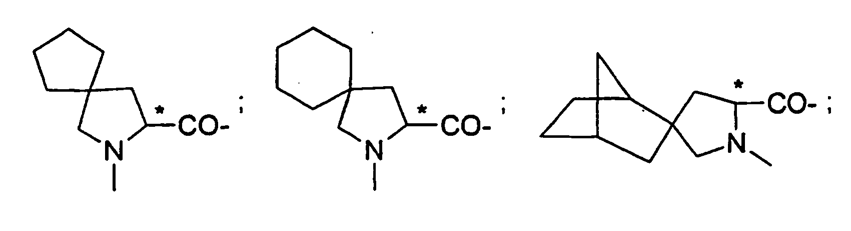

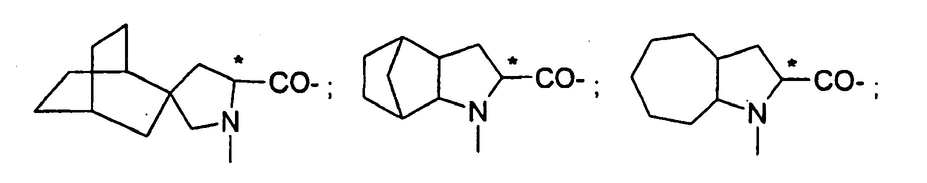

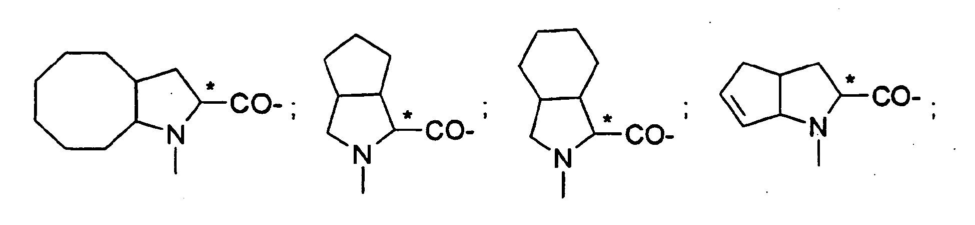

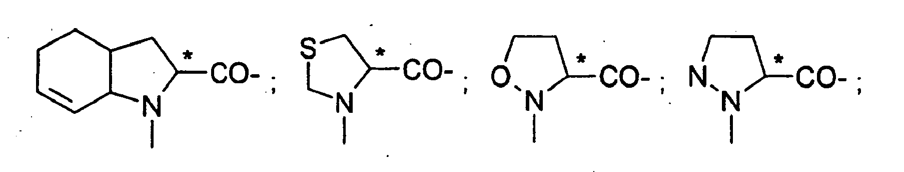

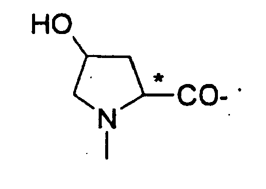

Die oben genannten Resten zugrundeliegenden Heterocyclen sind beispielsweise bekannt aus US-A-4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A271,865 und EP-A344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch in Form von Derivaten der Carbonsäuregruppe vorliegen, zum Beispiel als Ester oder Amide, wie zum Beispiel als Methylester, Ethylester, n-Propylester, Isopropylester, Isobutylester, tert-Butylester, Benzylester, unsubstituierte Amide, Methylamide, Ethylamide, Semicarbazide oder ω-Amino-(C₂-C₈)-alkylamide.

Funktionelle Gruppen in Resten von Aminosäuren, Iminosäuren, Azaaminosäuren und Dipeptiden sowie in anderen Teilen der Moleküle der Formel I können in geschützter Form vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ); Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Solche Salze sind beispielsweise bei Verbindungen der Formel I, welche saure Gruppen, zum Beispiel Carbonsäuregruppen, enthalten, Alkalimetallsalze oder Erdalkalimetallsalze sowie Salze mit Ammoniak und physiologisch verträglichen organischen Aminen. Solche Verbindungen der Formel I können also zum Beispiel als Natriumsalze, Kaliumsalze, Calciumsalze, Magnesiumsalze oder als Säureadditionssalze mit Aminen wie zum Beispiel Triethylamin, Ethanolamin, Tris-(2-hydroxy-ethyl)-amin oder Aminosäuren, insbesondere basischen Aminosäuren, vorliegen.

Verbindungen der Formel I, welche basische Gruppen, zum Beispiel eine Aminogruppe oder eine Guanidinogruppe, enthalten, bilden mit anorganischen Säuren, wie zum Beispiel Salzsäure, Schwefelsäure oder Phosphorsäure, und mit organischen Carbonsäuren oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure Salze. Enthalten die Verbindungen der Formel I gleichzeitig saure und basische Gruppen im Molekül, so gehören neben den geschilderten Salzformen auch innere Salze oder Betaine zu der Erfindung.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die Verbindungen der Formel I können in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Mischungen dieser Formen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe einer Ausgangssubstanz oder eines Zwischenprodukts im Verlaufe der Synthese.

Die erfindungsgemäßen Verbindungen der Formel I können darüber hinaus bewegliche Wasserstoffatome enthalten, also in verschiedenen tautomeren Formen vorliegen. Auch diese Tautomeren sind Gegenstand der vorliegenden Erfindung. Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und aktive Metabolite.

Die einzelnen Strukturelemente in der Formel I haben bevorzugt unabhängig voneinander die folgenden Bedeutungen.

W steht bevorzugt für R¹-A-C(R¹³).

Z steht bevorzugt für Sauerstoff.

A steht bevorzugt für eine direkte Bindung oder Methylen, besonders bevorzugt für eine direkte Bindung.

B steht bevorzugt für einen zweiwertigen Rest aus der Reihe Methylen, Ethylen, Trimethylen, Tetramethylen, Vinylen, Phenylen oder für einen substituierten (C₁-C₄)-Alkylenrest. Besonders bevorzugt steht B für einen zweiwertigen Methylenrest oder Ethylenrest (= 1,2-Ethylen), insbesondere für einen Methylenrest, wobei jeder dieser Reste unsubstituiert oder substituiert sein kann. Ganz besonders bevorzugt steht B für einen substituierten Methylenrest oder Ethylenrest, insbesondere für einen substituierten Methylenrest. Ist ein für B stehender zweiwertiger Alkylenrest, insbesondere ein Methylenrest oder Ethylenrest (= 1,2-Ethylen), substituiert, so ist er bevorzugt substituiert durch einen Rest aus der Reihe (C₁-C₈)-Alkyl, (C₂-C₈)-Alkenyl, (C₂-C₈)-Alkinyl, (C₃-C₇)-Cycloalkyl, insbesondere (C₅-C₆)-Cycloalkyl, (C₃-C₇)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl oder im Heteroarylrest gegebenenfalls substituiertes Heteroaryl-(C₁-C₄)-alkyl. Besonders bevorzugt ist ein für B stehender substituierter Alkylenrest substituiert durch (C₁-C₈)-Alkyl, also durch einen geradkettigen oder verzweigten Alkylrest mit 1, 2, 3, 4, 5, 6, 7 oder 8 Kohlenstoffatomen.

E steht bevorzugt für Tetrazolyl oder R¹⁰CO, besonders bevorzugt für R¹⁰CO.

R steht bevorzugt für Wasserstoff, (C₁-C₈)-Alkyl oder Benzyl, besonders bevorzugt für Wasserstoff oder (C₁-C₈)-Alkyl, ganz besonders bevorzugt für Wasserstoff oder (C₁-C₄)-Alkyl, insbesondere für Wasserstoff, Methyl oder Ethyl.

R⁰ steht bevorzugt für (C₁-C₈)-Alkyl, (C₃-C₁₂)-Cycloalkyl, (C₃-C₁₂)-Cycloalkyl-(C₁-C₈)-alkyl, (C₆-C₁₂-Bicycloalkyl, (C₆-C₁₂)-Bicycloalkyl-(C₁-C₈)-alkyl, (C₆-C₁₂)- Tricycloalkyl, (C₆-c₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl, besonders bevorzugt für (C₁-C₈)-Alkyl, (C₃-C₁₂)-Cycloalkyl, (C₃-C₁₂)-Cycloalkyl-(C₁-C₄)-alkyl, gegebenenfalls substituiertes (C₆-C₁₄)-Aryl, im Arylrest gegebenenfalls substituiertes (C₆-C₁₄)-Aryl-(C₁-C₄)-alkyl, gegebenenfalls substituiertes Heteroaryl oder im Heteroarylrest gegebenenfalls substituiertes Heteroaryl-(C₁-C₄)-alkyl, ganz besonders bevorzugt für gegebenenfalls substituiertes (C₆-C₁₄)-Aryl, im Arylrest gegebenenfalls substituiertes (C₆-C₁₄)-Aryl-(C₁-C₄)-alkyl, gegebenenfalls substituiertes Heteroaryl oder im Heteroarylrest gegebenenfalls substituiertes Heteroaryl-(C₁-C₄)-alkyl, darüber hinaus bevorzugt für im Arylrest gegebenenfalls substituiertes (C₆-C₁₄)-Aryl-(C₁-C₄)-alkyl oder im Heteroarylrest substituiertes Heteroaryl-(C₁-C₄)-alkyl. Speziell bevorzugt ist es, wenn R⁰ für im Arylrest gegebenenfalls substituiertes (C₆-C₁₄)-Aryl-(C₁-C₄)-alkyl, insbesondere für unsubstituiertes oder im Arylrest einfach oder mehrfach substituiertes Biphenylylmethyl, Naphthylmethyl oder Benzyl steht.

R¹ steht bevorzugt für einen Rest aus der Reihe Phenyl, Furyl, Thienyl, Pyrrolyl, Imidazolyl und Pyridyl, der nicht benzanelliert ist. Besonders bevorzugt steht R¹ für einen Phenylrest, einen 2-Furylrest, einen 3-Furylrest, einen 2-Thienylrest, einen 3-Thienylrest, einen 3-Pyrrolylrest, einen 4-Imidazolylrest, einen 3-Pyridylrest oder einen 4-Pyridylrest, ganz besonders bevorzugt für einen Phenylrest, einen 2-Furylrest, einen 3-Furylrest, einen 2-Thienylrest, einen 3-Thienylrest, einen 4-Imidazolylrest oder einen 4-Pyridylrest, darüber hinaus bevorzugt für einen Phenylrest oder einen 4-Pyridylrest. Bevorzugt ist ein für R¹ stehender Rest unsubstituiert oder durch einen, zwei oder drei, insbesondere durch einen oder durch zwei, gleiche oder verschiedene derjenigen Reste substituiert, die oben als in Betracht kommende Substituenten an Kohlenstoffatomen und Stickstoffatomen in R¹ angegeben sind. Besonders bevorzugt ist ein für R¹ stehender Rest unsubstituiert. Bevorzugte Substituenten an Kohlenstoffatomen in dem Rest R¹ sind (C₁-C₄)-Alkyl, (C₁-C₄)-Alkoxy, Halogen, Amino, Trifluormethyl, Hydroxy, Hydroxy-(C₁-C₄)-alkyl, Methylendioxy, Ethylendioxy, Phenyl, Phenoxy, Benzyl und Benzyloxy, insbesondere als Substituenten an Kohlenstoffatomen eines für R¹ stehenden Heteroarylrests. Besonders bevorzugte Substituenten an Kohlenstoffatomen in R¹, insbesondere an Kohlenstoffatomen eines für R¹ stehenden Phenylrests, sind (C₁-C₄)-Alkyl, (C₁-C₄)-Alkoxy, Halogen, Trifluormethyl, Hydroxy, Hydroxy-(C₁-C₄)-alkyl, Methylendioxy, Ethylendioxy, Phenyl, Phenoxy, Benzyl und Benzyloxy.

R² steht bevorzugt für Wasserstoff oder (C₁-C₈)-Alkyl, besonders bevorzugt für Wasserstoff oder (C₁-C₄)-Alkyl.

R³ steht bevorzugt für (C₁-C₈)-Alkyl, gegebenenfalls substituiertes (C₆-C₁₄)-Aryl, im Arylrest gegebenenfalls substituiertes (C₆-C₁₄)-Aryl-(C₁-C₄)-alkyl, (C₃-C₈)-Cycloalkyl, (C₃-C₈)-Cycloalkyl-(C₁-C₄)-alkyl, (C₆-C₁₂)-Bicycloalkyl, (C₆-C₁₂)-Bicycloalkyl-(C₁-C₄)-alkyl, (C₆-C₁₂)-Tricycloalkyl, (C₆-C₁₂)-Tricycloalkyl-(C₁-C₄)-alkyl, (C₂-C₈)-Alkenyl, (C₂-C₈)-Alkinyl, gegebenenfalls substituiertes Heteroaryl, im Heteroarylrest gegebenenfalls substituiertes Heteroaryl-(C₁-C₄)-alkyl, R¹¹NH, CON(CH₃)^{R}, CONHR⁴, CON(CH₃)R¹⁵ oder CONHR¹⁵, besonders bevorzugt für gegebenenfalls substituiertes (C₆-C₁₄)-Aryl, insbesondere gegebenenfalls substituiertes (C₆-C₁₀)-Aryl, gegebenenfalls substituiertes 5-gliedriges oder 6-gliedriges Heteroaryl mit einem oder zwei gleichen oder verschiedenen Heteroatomen aus der Reihe Stickstoff, Sauerstoff und Schwefel, insbesondere Pyridyl, R¹¹NH, CON(CH₃)R⁴, CONHR⁴, CON(CH₃)R¹⁵ oder CONHR¹⁵, ganz besonders bevorzugt für gegebenenfalls substituiertes (C₆-C₁₀)-Aryl, R¹¹NH, CON(CH₃)R⁴, CONHR⁴, CON(CH₃)R¹⁵ oder CONHR¹⁵

R⁴ steht bevorzugt für (C₁-C₈)-Alkyl, das gegebenenfalls wie oben in der Definition von R⁴ angegeben substituiert sein kann, besonders bevorzugt für (C₁-C₈)-Alkyl, insbesondere (C₁-C₆)-Alkyl, das durch einen oder zwei der in der obigen Definition von R⁴ angegebenen Substituenten substituiert ist. Ganz besonders bevorzugt ist es, wenn einer der Substituenten in der 1-Position der Alkylgruppe gebunden ist, also an dasjenige Kohlenstoffatom der Alkylgruppe, an das auch das Stickstoffatom in der Gruppe CONHR⁴ oder in der Gruppe CON(CH₃)R⁴ gebunden ist, und wenn dieser Substituent in der 1-Position einer der Reste Hydroxycarbonyl, Aminocarbonyl, Mono- oder Di-((C₁-C₁₈)-alkyl)-aminocarbonyl, (C₆-C₁₄)-Aryl-(C₁-C₈)-alkoxycarbonyl, das im Arylrest auch substituiert sein kann, Het-CO, R⁶-CO, (C₁-C₈)-Alkoxycarbonyl oder Tetrazolyl ist. In diesem ganz besonders bevorzugten Fall steht der Rest -NHR⁴ bzw. der Rest -N(CH₃)R⁴ somit für den Rest einer α-Aminosäure bzw. einer N-Methyl-α-aminosäure oder eines Derivats davon, wobei formal dieser Rest durch Abstraktion eines Wasserstoffatoms von der Aminogruppe der Aminosäure erhalten wird. Speziell bevorzugte α-Aminosäuren sind dabei solche mit einer lipophilen Seitenkette, zum Beispiel Phenylglycin, Phenylalanin, Valin, Leucin, Isoleucin und Homologe davon, sowie Derivate dieser Aminosäuren wie Ester, Amide oder die Derivate, in denen die Carbonsäuregruppe in den Rest Het-CO überführt ist.

R¹¹ steht bevorzugt für Wasserstoff, R^{23a}, R^{12a}-CO, H-CO, R^{12a}-O-CO, R^{12b}-CO, R^{12b}-CS oder R^{12a}-S(O)₂, besonders bevorzugt für Wasserstoff, R^{12a}, R^{12a}-CO, R^{12a}-O-CO, R^{12b}-CO, R^{12b}-CS oder R^{12a}S(O)₂, ganz besonders bevorzugt für R^{12a}, R^{12a}-CO, R^{12a}-O-CO, R^{12b}-CO, R^{12b}-CS oder R^{12a}-S(O)₂, darüber hinaus bevorzugt für R¹², R¹²-CO, R^{12a}-O-CO, R^{12b}-CO oder R^{12a}-S(O)₂.

R^{12a} steht bevorzugt für (C₁-C₁₀)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵.

R^{12b} steht bevorzugt für R^{12a}-NH.

R¹³ steht bevorzugt für Wasserstoff, (C₁-C₆)-Alkyl, (C₃-C₈)-Cycloalkyl oder Benzyl, besonders bevorzugt für Wassserstoff oder (C₁-C₆)-Alkyl, ganz besonders bevorzugt für Wasserstoff oder (C₁-C₄)-Alkyl, insbesondere für (C₁-C₄)-Alkyl, wobei ein bevorzugter Alkylrest, für den R¹³ steht, der Methylrest ist.

R¹⁵ steht bevorzugt für R¹⁶-(C₁-C₃)-alkyl oder für R¹⁶, besonders bevorzugt für R¹⁶-(C₁)-alkyl oder R¹⁶. Darüber hinaus bevorzugt steht R¹⁵ dann, wenn R³ für COOR¹⁵ steht, für den exo-2-Norbornylrest, den endo-2-Norbornylrest oder den Bicyclo[3.2.1]octylrest, und steht R¹⁵ dann, wenn R³ für CONHR¹⁵ steht, für den exo-2-Norbornylrest, den endo-2-Norbornylrest, den 3-Noradamantylrest und insbesondere den 1-Adamantylrest, den 2-Adamantylrest, den 1-Adamantylmethylrest oder den 2-Adamantylmethylrest.

R¹⁶ steht bevorzugt für einen 6-gliedrigen bis 14-gliedrigen, insbesondere einen 7-gliedrigen bis 12-gliedrigen, verbrückten bicyclischen oder tricyclischen Rest, der gesättigt oder teilweise ungesättigt ist und der auch ein bis vier, insbesondere ein, zwei oder drei, speziell ein oder zwei, gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann.

Het steht bevorzugt für den Rest eines über ein Ring-Stickstoffatom gebundenen 5-gliedrigen bis 10-gliedrigen, gesättigten monocyclischen oder polycyclischen Heterocyclus, der ein oder zwei gleiche oder verschiedene zusätzliche Ring-Heteratome aus der Reihe Sauerstoff, Stickstoff und Schwefel enthalten kann und an Kohlenstoffatomen und an Ring-Stickstoffatomen gegebenenfalls substituiert sein kann, wobei an zusätzlichen Ring-Stickstoffatome gleiche oder verschiedene Reste aus der Reihe Wasserstoff, R^{h}, HCO, R^{h}CO oder R^{h}O-CO als Substituenten stehen können. Besonders bevorzugt steht Het für einen derartigen Heterocyclus, der kein zusätzliches Ring-Heteroatom enthält oder der ein zusätzliches Ring-Heteroatom aus der Reihe Stickstoff, Sauerstoff und Schwefel enthält, ganz besonders bevorzugt steht Het für den Rest eines über ein Stickstoffatom gebundenen 5-gliedrigen, 6-gliedrigen oder 7-gliedrigen, gesättigten monocyclischen Heterocyclus, der kein zusätzliches Ring-Heteroatom enthält oder der ein zusätzliches Ring-Heteroatom aus der Reihe Stickstoff, Sauerstoff und Schwefel enthält, wobei auch in diesen Fällen der Rest Het unsubstituiert sein kann oder an Kohlenstoffatomen und/oder an zusätzlichen Ring-Stickstoffatomen substituiert sein kann.

Steht R³ für einen der Reste (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, (C₃-C₈)-Cycloalkyl, (C₃-C₈)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, (C₂-C₈)-Alkenyl, (C₂-C₈)-Alkinyl, CON(CH₃)R⁴, CONHR⁴, COOR¹⁵, CON(CH₃)R¹⁵ oder CONHR¹⁵, so steht bevorzugt e für 0 und h für 1. Steht R³ für R¹¹NH, so steht bevorzugt e für 1 und h für 0.

Bevorzugte Verbindungen der Formel I sind solche Verbindungen, in denen einer oder mehrere der Reste bevorzugte Bedeutungen haben, wobei alle Kombinationen von bevorzugten Substituentenbedeutungen Gegenstand der vorliegenden Erfindung sind. Besonders bevorzugte Verbindungen der Formel 1 sind solche, worin gleichzeitig
- W: für R¹-A-C(R¹³) steht;
- Z: für Sauerstoff oder Schwefel steht;
- A: für eine direkte Bindung oder Methylen steht;
- B: für einen zweiwertigen Methylenrest oder Ethylenrest steht, die beide unsubstituiert sein können oder substituiert sein können durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl;
- E: Tetrazolyl oder R¹⁰CO bedeutet;
- R: Wasserstoff oder (C₁-C₈)-Alkyl bedeutet;
- R⁰: für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, H-CO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆- C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl- S(O)ₙ steht, wobei n für 1 oder 2 steht;
- R¹: für einen gegebenenfalls substituierten Rest aus der Reihe Phenyl, Furyl, Thienyl, Pyrrolyl, Imidazolyl und Pyridyl steht, wobei jeder dieser Reste auch benzanelliert sein kann;
- R²: Wasserstoff oder (C₁-C₈)-Alkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, (C₃-C₈)-Cycloalkyl, (C₃-C₈)-Cycloalkyl-(C₁-C₈)-alkyl, (C₆- C₁₂)-Bicycloalkyl, (C₆-C₁₂)-Bicycloalkyl-(C₁-C₈)-alkyl, (C₆-C₁₂)-Tricycloalkyl, (C₆- C₁₂)-Tricycloalkyl-(C₁-C₈)-alkyl, (C₂-C₈)-Alkenyl, (C₂-C₈)-Alkinyl, R¹¹NH, CON(CH₃)R⁴, CONHR⁴, COOR¹⁵, CON(CH₃)R¹⁵ oder CONHR¹⁵ bedeutet;
- R⁴: (C₁-C₈)-Alkyl bedeutet, das gegebenenfalls einfach oder mehrfach durch gleiche oder verschiedene Reste aus der Reihe Hydroxy, (C₁-C₈)-Alkoxy, R⁵, gegebenenfalls substituiertes (C₃-C₈)-Cycloalkyl, Hydroxycarbonyl, Aminocarbonyl, Mono- oder Di-((C₁-C₁₈)-alkyl)-aminocarbonyl, (C₆-C₁₄)-Aryl-(C₁- C₈)-alkoxycarbonyl, das im Arylrest auch substituiert sein kann, (C₁-C₈)- Alkoxycarbonyl, Het-CO, R⁶-CO, Tetrazolyl und Trifluormethyl substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl oder einen gegebenenfalls substituierten monocyclischen oder bicyclischen 5-gliedrigen bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, bedeutet;
- R⁶: den Rest einer natürlichen oder unnatürlichen Aminosäure, Iminosäure, gegebenenfalls N-(C₁-C₈)-alkylierten oder N-((C₆-C₁₄)-Aryl-(C₁-C₈)-alkylierten) Azaaminosäure, der im Arylrest auch substituiert kann, oder den Rest eines Dipeptids bedeutet, sowie deren Ester und Amide, wobei freie funktionelle Gruppen durch in der Peptidchemie übliche Schutzgruppen geschützt sein können;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, (C₁-C₈)- Alkylcarbonyloxy-(C₁-C₆)-alkoxy, (C₆-C₁₄)-Arylcarbonyloxy-(C₁-C₆)-alkoxy,
- R¹¹: Amino oder Mono- oder Di-((C₁-C₁₈)-alkyl)-amino bedeutet; für Wasserstoff, R^{12a}, R^{12a}-CO, R^{12a}-O-CO, R^{12b}-CO, R^{12b}-CS oder R^{12a}-S(O)₂ steht;
- R^{12a}: (C₁-C₁₈)-Alkyl, (C₂-C₈)-Alkenyl, (C₂-C₈)-Alkinyl, (C₃-C₁₂)-Cycloalkyl, (C₃-C₁₂)- Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₈)-alkyl)-amino oder R^{12a}-NH bedeutet;
- R¹³: Wasserstoff oder (C₁-C₆)-Alkyl bedeutet;
- R¹⁵: für R¹⁶-(C₁-C₆)-alkyl oder für R¹⁶ steht;
- R¹⁶: für einen 6-gliedrigen bis 14-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- Het: für den Rest eines über ein Ring-Stickstoffatom gebundenen 5-gliedrigen bis 10-gliedrigen, gesättigten monocyclischen oder polycyclischen Heterocyclus steht, der ein, zwei, drei oder vier gleiche oder verschiedene zusätzliche Ring- Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring- Stickstoffatomen gleiche oder verschiedene Reste aus der Reihe Wasserstoff, R^{h}, HCO, R^{h}CO oder R^{h}O-CO als Substituenten stehen können und R^{h} für (C₁- C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆- C₁₄)-Aryl-(C₁-C₈)-alkyl steht;
- e und h: unabhängig voneinander für 0 oder 1 stehen;
in allen ihren stereoisomeren Formen und Mischungen davon in allen Verhältnissen, und ihre physiologisch verträglichen Salze.

Ganz besonders bevorzugte Verbindungen der Formel I sind solche, worin gleichzeitig
- W: für R¹-A-C(R¹³) steht;
- Z: für Sauerstoff steht;
- A: für eine direkte Bindung oder Methylen steht;
- B: für einen zweiwertigen Methylenrest oder Ethylenrest steht, die beide unsubstituiert sein können oder substituiert sein können durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl;
- E: R¹⁰CO bedeutet;
- R: Wasserstoff oder (C₁-C₄)-Alkyl bedeutet;
- R⁰: für (C₁-C₈)-Alkyl, (C₃-C₁₂)-Cycloalkyl, (C₃-C₁₂)-Cycloalkyl-(C₁-C₈)-alkyl, (C₆-C₁₂)- Bicycloalkyl, (C₆-C₁₂)-Bicycloalkyl-(C₁-C₈)-alkyl, (C₆-C₁₂)-Tricycloalkyl, (C₆-C₁₂)- Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl steht;
- R¹: für einen gegebenenfalls substituierten Rest aus der Reihe Phenyl, Furyl, Thienyl, Pyrrolyl, Imidazolyl und Pyridyl steht;
- R²: Wasserstoff oder (C₁-C₄)-Alkyl bedeutet;
- R³: (C₁-C₈)-Alkyl, gegebenenfalls substituiertes (C₆-C₁₄)-Aryl, im Arylrest gegebenenfalls substituiertes (C₆-C₁₄)-Aryl-(C₁-C₄)-alkyl, gegebenenfalls substituiertes Heteroaryl, im Heteroarylrest gegebenenfalls substituiertes Heteroaryl-(C₁-C₄)-alkyl, (C₃-C₈)-Cycloalkyl, (C₃-C₈)-Cycloalkyl-(C₁-C₄)-alkyl, (C₆- C₁₂)-Bicycloalkyl, (C₆-C₁₂)-Bicycloalkyl-(C₁-C₄)-alkyl, (C₆-C₁₂)-Tricycloalkyl, (C₆- C₁₂)-Tricycloalkyl-(C₁-C₄)-alkyl, R¹¹NH, CON(CH₃)R⁴, CONHR⁴, COOR¹⁵, CON(CH₃)R¹⁵ oder CONHR¹⁵ bedeutet;
- R⁴: (C₁-C₈)-Alkyl bedeutet, das gegebenenfalls einfach oder mehrfach durch gleiche
- R⁵: oder verschiedene Reste aus der Reihe Hydroxy, (C₁-C₈)-Alkoxy, R⁵, gegebenenfalls substituiertes (C₃-C₈)-Cycloalkyl, Hydroxycarbonyl, Aminocarbonyl, Mono- oder Di-((C₁-C₈)-alkyl)-aminocarbonyl, (C₆-C₁₄)-Aryl-(C₁- C₈)-alkoxycarbonyl, das im Arylrest auch substituiert sein kann, (C₁-C₈)- Alkoxycarbonyl, Het-CO, R⁶-CO, Tetrazolyl und Trifluormethyl substituiert sein kann; gegebenenfalls substituiertes (C₆-C₁₄)-Aryl, im Arylrest gegebenenfalls substituiertes (C₆-C₁₄)-Aryl-(C₁-C₈)-alkyl oder einen gegebenenfalls substituierten monocyclischen oder bicyclischen 5-gliedrigen bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, bedeutet;
- R⁶: den Rest einer natürlichen oder unnatürlichen Aminosäure, Iminosäure oder gegebenenfalls N-(C₁-C₈)-alkylierten oder N-((C₆-C₁₄)-Aryl-(C₁-C₈)-alkylierten) Azaaminosäure, der im Arylrest auch substituiert kann, bedeutet, sowie deren Ester und Amide, wobei freie funktionelle Gruppen durch in der Peptidchemie übliche Schutzgruppen geschützt sein können;
- R¹⁰: Hydroxy, (C₁-C₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, (C₁-C₈)- Alkylcarbonyloxy-(C₁-C₆)-alkoxy, (C₆-C₁₄)-Arylcarbonyloxy-(C₁-C₆)-alkoxy, Amino oder Mono- oder Di-((C₁-C₈)-alkyl)-amino bedeutet;
- R¹¹: für R^{12a}, R^{12a}-CO, R^{12a}-O-CO, R^{12b}-CO oder R^{12a}-S(O)₂ steht;
- R^{12a}: (C₁-C₁₀)-Alkyl, (C₂-C₈)-Alkenyl, (C₂-C₈)-Alkinyl; (C₃-C₁₂)-Cycloalkyl, (C₃-C₁₂)- Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₀)-alkyl)-amino oder R^{12a}-NH bedeutet;
- R¹³: Wasserstoff oder (C₁-C₄)-Alkyl bedeutet;
- R¹⁵: für R¹⁶-(C₁-C₃)-alkyl oder für R¹⁶ steht;
- R¹⁶: für einen 7-gliedrigen bis 12-gliedrigen bicyclischen oder tricyclischen Rest
- Het: steht, der gesättigt oder teilweise ungesättigt ist und der auch ein oder zwei gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann; für den Rest eines über ein Ring-Stickstoffatom gebundenen 5-gliedrigen bis 10-gliedrigen, gesättigten monocyclischen oder polycyclischen Heterocyclus steht, der ein oder zwei gleiche oder verschiedene zusätzliche Ring- Heteratome aus der Reihe Sauerstoff, Stickstoff und Schwefel enthalten kann und der an Kohlenstoffatomen und zusätzlichen Ring-Stickstoffatomen gegebenenfalls substituiert sein kann, wobei an zusätzlichen Ring- Stickstoffatomen gleiche oder verschiedene Reste aus der Reihe Wasserstoff, R^{h}, HCO, R^{h}CO oder R^{h}O-CO als Substituenten stehen können und R^{h} für (C₁- C₆)-Alkyl, (C₃-C₈)-Cycloalkyl, (C₃-C₈)-Cycloalkyl-(C₁-C₄)-alkyl, gegebenenfalls substituiertes (C₆-C₁₄)-Aryl oder im Arylrest gegebenenfalls substituiertes (C₆- C₁₄)-Aryl-(C₁-C₄)-alkyl steht;
- e und h: unabhängig voneinander für 0 oder 1 stehen;
in allen ihren stereoisomeren Formen und Mischungen davon in allen Verhältnissen, und ihre physiologisch verträglichen Salze.

Darüber hinaus bevorzugte Verbindungen der Formel I sind solche, worin gleichzeitig
- W: für R¹-A-C(R¹³) steht;
- Z: für Sauerstoff steht;
- A: für eine direkte Bindung oder Methylen steht;
- B: für einen unsubstituierten Methylenrest oder für einen Methylenrest steht, der durch einen Rest aus der Reihe (C₁-C₈)-Alkyl, (C₂-C₈)-Alkenyl, (C₂-C₈)-Alkinyl, (C₃-C₇)-Cycloalkyl, (C₃-C₇)-Cycloalkyl-(C₁-C₄)-alkyl, gegebenenfalls substituiertes (C₆-C₁₀)-Aryl, im Arylrest gegebenenfalls substituiertes (C₆-C₁₀)- Aryl-(C₁-C₄)-alkyl, gegebenenfalls substituiertes Heteroaryl und im Heteroarylrest gegebenenfalls substituiertes Heteroaryl-(C₁-C₄)-alkyl substituiert ist;
- E: R¹⁰CO bedeutet;
- R: Wasserstoff oder (C₁-C₄)-Alkyl bedeutet;
- R⁰: für im Arylrest gegebenenfalls substituiertes (C₆-C₁₄)-Aryl-(C₁-C₄)-alkyl oder im Heteroarylrest gegebenenfalls substituiertes Heteroaryl-(C₁-C₄)-alkyl steht;
- R¹: für einen gegebenenfalls substituierten Rest aus der Reihe Phenyl, Furyl, Thienyl, Pyrrolyl, Imidazolyl und Pyridyl steht;
- R²: Wasserstoff oder (C₁-C₄)-Alkyl bedeutet;
- R³: für einen unsubstituierten Phenylrest oder Naphthylrest oder einen durch einen, zwei oder drei gleiche oder verschiedene Reste aus der Reihe (C₁-C₄)-Alkyl, (C₁- C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy, Benzyl und Benzyloxy substituierten Phenylrest oder Naphthylrest steht oder R³ für Pyridyl, (C₁-C₄)-Alkyl, (C₂-C₄)-Alkenyl, (C₂-C₄)- Alkinyl, (C₅-C₆)-Cycloalkyl, R¹¹NH, CON(CH₃)R⁴, CONHR⁴, CON(CH₃)R¹⁵ oder CONHR¹⁵ steht;
- R⁴: (C₁-C₈)-Alkyl bedeutet, das durch einen oder zwei gleiche oder verschiedene Reste aus der Reihe Hydroxy, (C₁-C₈)-Alkoxy, R⁵, gegebenenfalls substituiertes (C₃-C₈)-Cycloalkyl, Hydroxycarbonyl, Aminocarbonyl, (C₆-C₁₀)-Aryl-(C₁-C₄)- alkoxycarbonyl, das im Arylrest auch substituiert sein kann, (C₁-C₆)- Alkoxycarbonyl, Het-CO, R⁶-CO, Tetrazolyl und Trifluormethyl substituiert ist;
- R⁵: gegebenenfalls substituiertes (C₆-C₁₀)-Aryl, im Arylrest gegebenenfalls substituiertes (C₆-C₁₀)-Aryl-(C₁-C₄)-alkyl oder einen gegebenenfalls substituierten monocyclischen oder bicyclischen 5-gliedrigen bis 10-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, bedeutet;
- R¹⁰: Hydroxy, (C₁-C₈)-Alkoxy, (C₆-C₁₀)-Aryl-(C₁-C₄)-alkoxy, das im Arylrest auch substituiert sein kann, gegebenenfalls substituiertes (C₆-C₁₀)-Aryloxy, (C₁-C₈)- Alkylcarbonyloxy-(C₁-C₄)-alkoxy, (C₈-C₁₀)-Arylcarbonyloxy-(C₁-C₄)-alkoxy, Amino oder Mono- oder Di-((C₁-C₈)-alkyl)-amino bedeutet;
- R¹¹: für R^{12a}, R^{12a}-CO, R^{12a}-O-CO, R^{12b}-CO oder R^{12a}-S(O)₂ steht;
- R^{12a}: (C₁-C₁₀)-Alkyl, (C₂-C₈)-Alkenyl, (C₂-C₈)-Alkinyl, (C₃-C₁₂)-Cycloalkyl, (C₃-C₁₂)- Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₀)-alkyl)-amino oder R^{12a}-NH bedeutet;
- R¹³: Wasserstoff oder (C₁-C₄)-Alkyl bedeutet;
- R¹⁵: für R¹⁶-(C₁-C₃)-alkyl oder für R¹⁶ steht;
- R¹⁶: für einen 7-gliedrigen bis 12-gliedrigen bicyclischen oder tricyclischen Rest steht, der gesättigt ist und der auch ein oder zwei gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- Het: für den Rest eines über ein Ring-Stickstoffatom gebundenen 5-gliedrigen bis 7-gliedrigen, gesättigten monocyclischen Heterocyclus steht, der ein oder zwei gleiche oder verschiedene zusätzliche Ring-Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring-Stickstoffatomen gleiche oder verschiedene Reste aus der Reihe Wasserstoff, R^{h}, HCO, R^{h}CO oder R^{h}O-CO als Substituenten stehen können und R^{h} (C₁-C₆)-Alkyl, (C₃-C₈)- Cycloalkyl, (C₃-C₈)-Cycloalkyl-(C₁-C₄)-alkyl, gegebenenfalls substituiertes (C₆- C₁₀)-Aryl oder im Arylrest gegebenenfalls substituiertes (C₆-C₁₀)-Aryl-(C₁-C₄)- alkyl steht;
- e und h: unabhängig voneinander für 0 oder 1 stehen;
in allen ihren stereoisomeren Formen und Mischungen davon in allen Verhältnissen, und ihre physiologisch verträglichen Salze.

Speziell bevorzugte Verbindungen der Formel I sind zum einen solche, worin B für unsubstituiertes Methylen steht oder für Methylen steht, das durch einen (C₁-C₈)-Alkylrest substituiert ist, in allen ihren stereoisomeren Formen und Mischungen davon in allen Verhältnissen, und ihre physiologisch verträglichen Salze. Besonders speziell bevorzugte Verbindungen der Formel I sind solche, worin B für Methylen steht, das durch einen (C₁-C₈)-Alkylrest substituiert ist, in allen ihren stereoisomeren Formen und Mischungen davon in allen Verhältnissen, und ihre physiologisch verträglichen Salze.

Speziell bevorzugte Verbindungen der Formel I sind zum anderen solche, worin R¹ für einen Rest aus der Reihe Phenyl, Furyl, Thienyl, Pyrrolyl, Imidazolyl und Pyridyl steht, der unsubstituiert oder durch einen, zwei oder drei gleiche oder verschiedene Substituenten aus der Reihe (C₁-C₄)-Alkyl, (C₁-C₄)-Alkoxy, Halogen, Amino, Trifluormethyl, Hydroxy, Hydroxy-(C₁-C₄)-alkyl, Methylendioxy, Ethylendioxy, Phenyl, Phenoxy, Benzyl und Benzyloxy substituiert ist, in allen ihren stereoisomeren Formen und Mischungen davon in allen Verhältnissen, und ihre physiologisch verträglichen Salze.

Besonders speziell bevorzugte Verbindungen der Formel I sind solche, worin R¹ für einen Rest aus der Reihe Phenyl, 2-Furyl, 3-Furyl, 2-Thienyl, 3-Thienyl, 3-Pyrrolyl, 4-Imidazolyl und 3-Pyridyl und 4-Pyridyl steht, wobei der Phenylrest unsubstituiert ist oder durch einen oder zwei gleich oder verschiedene Reste aus der Reihe (C₁-C₄)-Alkyl, (C₁-C₄)-Alkoxy, Halogen, Trifluormethyl, Hydroxy, Hydroxy-(C₁-C₄)-alkyl, Methylendioxy, Ethylendioxy, Phenyl, Phenoxy, Benzyl und Benzyloxy substituiert ist und wobei die heteroaromatischen Reste unsubstituiert sind oder durch einen oder zwei gleiche oder verschiedene Reste aus der Reihe (C₁-C₄)-Alkyl, (C₁-C₄)-Alkoxy, Halogen, Amino, Trifluormethyl, Hydroxy, Hydroxy-(C₁-C₄)-alkyl, Methylendioxy, Ethylendioxy, Phenyl, Phenoxy, Benzyl und Benzyloxy substituiert sind, in allen ihren stereoisomeren Formen und Mischungen davon in allen Verhältnissen, und ihre physiologisch verträglichen Salze.

Ganz besonders speziell bevorzugte Verbindungen der Formel I sind solche, worin R¹ für einen unsubstituierten Rest aus der Reihe Phenyl, 2-Euryl 3-Furyl, 2-Thienyl, 3-Thienyl, 3-Pyrrolyl, 4-Imidazolyl, 3-Pyridyl und 4-Pyridyl steht, in allen ihren stereoisomeren Formen und Mischungen davon in allen Verhältnissen, und ihre physiologisch verträglichen Salze.

Noch spezieller bevorzugte Verbindungen der Formel I sind solche, worin R¹ für einen unsubstituierten Rest aus der Reihe Phenyl, 2-Furyl, 3-Furyl, 2-Thienyl, 3-Thienyl, 4-Imidazolyl und 4-Pyridyl steht, in allen ihren stereoisomeren Formen und Mischungen davon in allen Verhältnissen, und ihre physiologisch verträglichen Salze.

Generell sind Verbindungen der Formel I bevorzugt, die an Chiralitätszentren, zum Beispiel bei entsprechender Substitution an dem die Reste R² und R³ tragenden chiralen Kohlenstoffatom und/oder an dem Zentrum W im Imidazolidin-Ring in der Formel I, eine einheitliche Konfiguration aufweisen.

Die Verbindungen der Formel I können beispielsweise hergestellt werden durch Fragmentkondensation einer Verbindung der Formel II 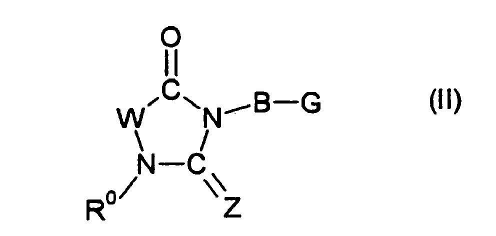 mit einer Verbindung der Formel III, 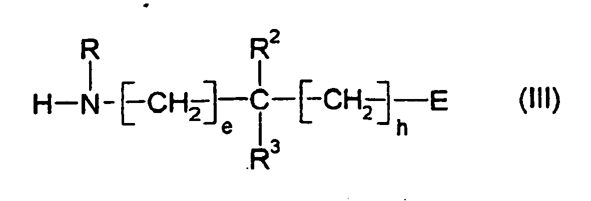 wobei in den Formeln II und III die Gruppen W, Z, B, E, R, R⁰, R² und R³ sowie e und h wie oben angegeben definiert sind oder auch in diesen Gruppen funktionelle Gruppen in geschützter Form oder in Form von Vorstufen enthalten sein können, und wobei G für Hydroxycarbonyl, (C₁-C₆)-Alkoxycarbonyl oder aktivierte Carbonsäurederivate wie Säurechloride oder Aktivester steht. In den Verbindungen der Formel III kann, wenn Verbindungen der Formel I hergestellt werden sollen, in denen zum Beispiel R³ in der Formel I für ein Carbonsäurederivat steht oder ein solches enthält, aber beispielsweise auch der Rest R³ zunächst für eine in geschützter Form vorliegende Hydroxycarbonylgruppe stehen oder eine solche enthalten, und dann erst nach der Kondensation der Verbindungen der Formeln II und III in einem oder mehreren weiteren Schritten die gewünschte endgültige Gruppe R³ aufgebaut werden.

Zur Kondensation der Verbindungen der Formel II mit denen der Formel III verwendet man vorteilhafterweise die dem Fachmann an sich wohlbekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Als Kondensationsmittel kommen zum Beispiel Carbonyldiimidazol, Carbodiimide wie Dicyclohexylcarbodiimid oder Diisopropylcarbodiimid, das O-((Cyan(ethoxycarbonyl)methylen)amino)-N,N,N',N'-tetramethyluronium-tetrafluoroborat (TOTU) oder Propylphosphonsäureanhydrid (PPA) in Betracht. Bei der Kondensation ist es in der Regel nötig, daß vorhandene, nicht reagierende Aminogruppen durch reversible Schutzgruppen geschützt werden. Gleiches gilt für nicht an der Reaktion beteiligte Carboxylgruppen, die während der Kondensation bevorzugt als (C₁-C₆)-Alkylester, zum Beispiel tert-Butylester, oder Benzylester vorliegen. Ein Aminogruppen-Schutz erübrigt sich, wenn die Aminogruppen noch in Form von Vorstufen, zum Beispiel als Nitrogruppen, vorliegen und erst nach der Kupplung, zum Beispiel durch Hydrierung, gebildet werden. Nach der Kupplung werden die vorhandenen Schutzgruppen in geeigneter Weise abgespalten. Beispielsweise können NO₂-Gruppen (Guanidinoschutz in Aminosäuren), Benzyloxycarbonylgruppen und Benzylgruppen in Benzylestern abhydriert werden. Die Schutzgruppen vom tert-Butyltyp werden sauer abgespalten, während der 9-Fluorenylmethyloxycarbonylrest durch sekundäre Amine entfernt wird. Die Herstellung der Verbindungen der Formel I kann beispielsweise auch erfolgen, indem man die Verbindungen nach üblichen Methoden schrittweise an einer Festphase aufbaut, wobei die einzelnen Bauelemente des Moleküls in unterschiedlicher Reihenfolge eingeführt werden können.

Verbindungen der Formel II, in der W für R¹-A-C(R¹³) steht und Z für Sauerstoff steht, können beispielsweise hergestellt werden, indem man zunächst Verbindungen der Formel IV 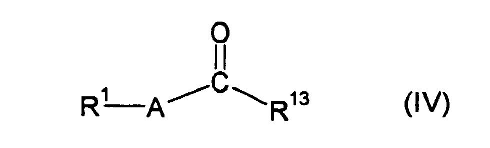 in einer Bucherer-Reaktion zu Verbindungen der Formel V, 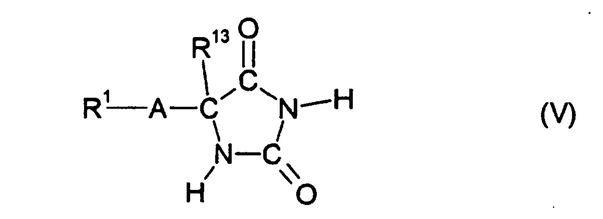 in der ebenso wie in der Formel IV R¹, R¹³ und A wie oben angegeben definiert sind, umsetzt (H. T. Bucherer, V. A. Lieb, J. Prakt. Chem. 141(1934), 5). Verbindungen der Formel VI, 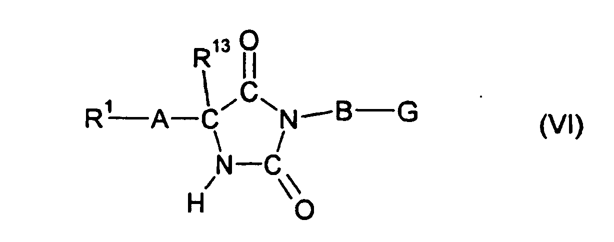 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R⁰-LG, in der R⁰ die oben angegebenen Bedeutungen hat und LG eine nucleophil substituierbare Abgangsgruppe, zum Beispiel Halogen, insbesondere Chlor oder Brom, (C₁-C₄)-Alkoxy, gegebenenfalls substituiertes Phenoxy oder eine heterocyclische Abgangsgruppe wie zum Beispiel Imidazolyl, darstellt, führt zu den entsprechenden Verbindungen der Formel II. Diese Umsetzungen können analog bekannten, dem Fachmann geläufigen Methoden durchgeführt werden. Je nach dem Einzelfall kann es hierbei, wie bei allen Schritten in der Synthese der Verbindungen der Formel I, angebracht sein, funktionelle Gruppen die zu Nebenreaktionen oder unerwünschten Reaktionen führen könnten, durch eine dem Syntheseproblem angepaßte Schutzgruppenstrategie temporär zu blockieren, was dem Fachmann bekannt ist. Hinsichtlich der Herstellung der Verbindungen der Formeln V und VI in racemischer Form und in enantiomerenreiner Form wird hier insbesondere Bezug genommen auf die entsprechenden Ausführungen in der WO-A-96/33976, die Bestandteil der vorliegenden Offenbarung sind.

Steht W für R¹-A-CH=C, so kann dieses Strukturelement beispielsweise eingeführt werden, indem analog bekannten Methoden ein Aldehyd mit einem Dioxoimidazolidin oder Thioxo-oxo-imidazolidin kondensiert wird, das eine unsubstituierte Methylengruppe in der der Gruppe W entsprechenden Position enthält.

Die Aminoverbindungen der Formel III können nach oder analog zu wohlbekannten Standardverfahren aus Ausgangsverbindungen aufgebaut werden, die käuflich sind oder nach oder analog zu Literaturvorschriften erhältlich sind.

Verbindungen der Formel I, in denen W für R¹-A-C(R¹³) steht, können auch wie folgt erhalten werden:

Durch Reaktion von nach Standardverfahren erhältlichen α-Aminosäuren oder N-substituierten α-Aminosäuren oder bevorzugt deren Estern, zum Beispiel der Methyl-, Ethyl-, tert-Butyl- oder Benzylester, beispielsweise einer Verbindung der Formel VII, 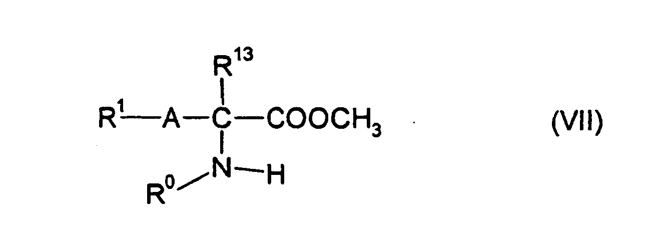 worin R⁰, R¹, R¹³ und A wie oben angegeben definiert sind, mit einem Isocyanat oder Isothiocyanat beispielsweise der Formel VIII, 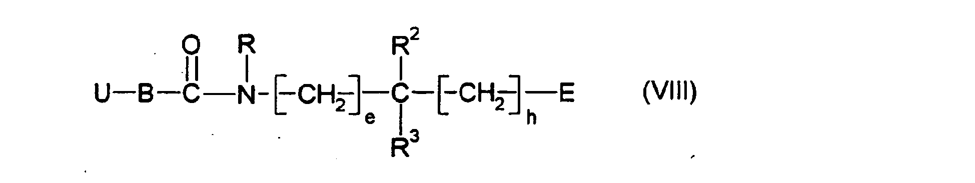 worin B, E, R, R², R³, e und h wie oben angegeben definiert sind und U für Isocyanato oder Isothiocyanato steht, erhält man Harnstoffderivate oder Thiohamstoffderivate der Formel IX, 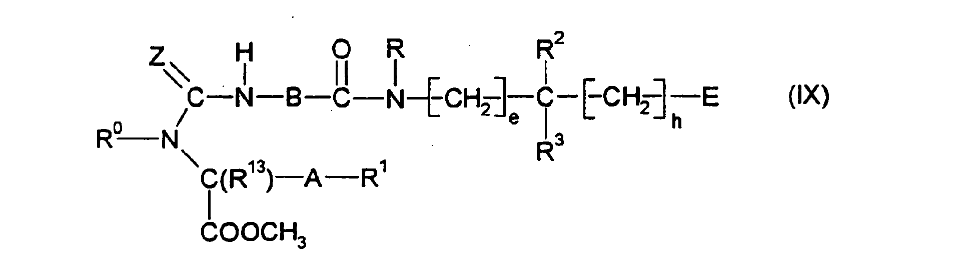 für die die oben angegebenen Definitionen gelten, und die durch Erhitzen mit Säure unter Verseifung der Esterfunktionen zu Verbindungen der Formel Ia 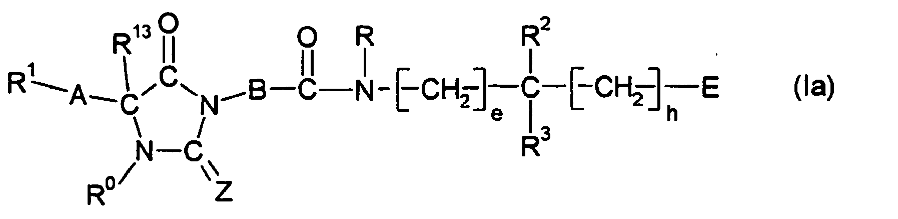 cyclisiert werden, für die die oben angegebenen Bedeutungen gelten. Die Cyclisierung der Verbindungen der Formel IX zu den Verbindungen der Formel la kann auch durch Behandlung mit Basen in inerten Lösungsmittel durchgeführt werden, zum Beispiel durch Behandlung mit Natriumhydrid in einem aprotischen Lösungsmittel wie Dimethylformamid. Während der Cyclisierung können wiederum funktionelle Gruppen in geschützter Form vorliegen.

Verbindungen der Formel I, in denen W für R¹-A-C(R¹³) steht, können auch erhalten werden, indem man eine Verbindung der Formel VII mit einem Isocyanat oder Isothiocyanat der Formel X 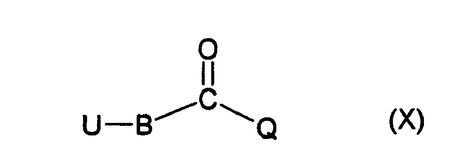 umsetzt, in der B und U wie oben für die Formel VIII angegeben definiert sind und Q eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XI 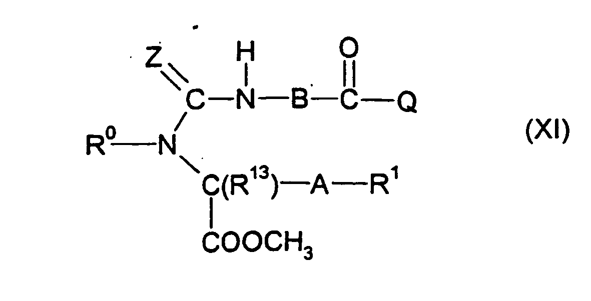 erhalten, in der Z, A, B, Q, R⁰, R¹ und R¹³ wie oben für die Formeln IX und X angegeben definiert sind, die dann unter dem Einfluß einer Säure oder einer Base, wie oben für die Cyclisierung der Verbindungen der Formel IX beschrieben, zu einer Verbindung der Formel XII, 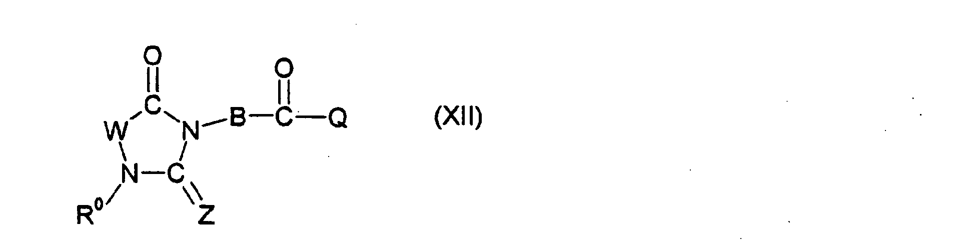 in der W für R¹-A-C(R¹³) steht und Z, B, Q, und R⁰ wie oben für die Formeln la und X angegeben definiert sind, cyclisiert wird. Aus der Verbindung der Formel XII wird dann durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel la erhalten. Auch hier können während der Cyclisierung funktionelle Gruppen in geschützter Form oder in Form von Vorstufen vorliegen.

Eine weitere Methode zur Herstellung von Verbindungen der Formel Ia ist beispielsweise die Umsetzung von Verbindungen der Formel XIII, 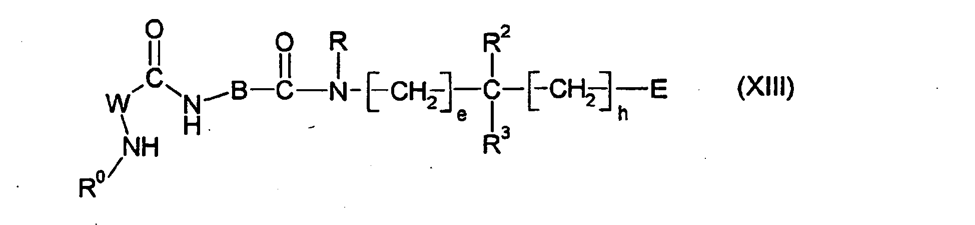 in der W für R¹-A-C(R¹³) steht und für die ansonsten die oben angegebenen Definitionen gelten, mit Phosgen oder Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Hinsichtlich der Herstellung der Verbindungen der Formel I wird weiterhin vollinhaltlich Bezug genommen auf die WO-A-95/14008, auf die EP-A-796 855 (europäische Patentanmeldung 97103712.2) und die ihr entsprechenden Anmeldungen, sowie auf die WO-A-96/33976.

Die Verbindungen der Formel I sind wertvolle Arzneimittelwirkstoffe, die sich beispielsweise für die Therapie und Prophylaxe von Entzündungserkrankungen, allergischen Erkrankungen oder Asthma eignen. Die Verbindungen der Formel I und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Präparaten verabreicht werden, die eine enterale oder parenterale Anwendung gestatten und die als aktiven Bestandteil eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten.

Gegenstand der vorliegenden Erfindung sind daher auch die Verbindungen der Formel I und/oder ihre physiologisch verträglichen Salze zur Verwendung als Arzneimittel, die Verwendung der Verbindungen der Formel I und/oder ihrer physiologisch verträglichen Salze zur Herstellung von Arzneimitteln für die Therapie und Prophylaxe der oben oder im folgenden erläuterten Krankheiten, zum Beispiel für die Therapie und Prophylaxe von Entzündungserkrankungen, sowie die Verwendung der Verbindungen der Formel I und/oder ihrer physiologisch verträglichen Salze bei der Therapie und Prophylaxe dieser Krankheiten. Weiterhin sind Gegenstand der vorliegenden Erfindung pharmazeutische Präparate, die eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten.

Die Arzneimittel können oral, zum Beispiel in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, zum Beispiel in Form von Suppositorien, oder parenteral, zum Beispiel in Form von Injektionslösungen oder Infusionslösungen, Mikrokapseln oder Rods, oder perkutan, zum Beispiel in Form von Salben, Lösungen oder Tinkturen, oder auf anderem Wege, zum Beispiel in Form von Nasalsprays oder Aerosolmischungen, erfolgen.

Die Herstellung der erfindungsgemäßen pharmazeutischen Präparate erfolgt in an sich bekannter Weise, wobei neben der oder den Verbindungen der Formel I und/oder ihren physiologisch verträglichen Salzen pharmazeutisch inerte anorganische oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure. Die pharmzeutischen Präparate enthalten normalerweise etwa 0,5 bis 90 Gew.-% der Verbindungen der Formel I und/oder ihrer physiologisch verträglichen Salze.

Die pharmazeutischen Präparate können neben den Wirkstoffen und Trägerstoffen noch Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, überzugsmittel oder Antioxidantien enthalten. Sie können auch zwei oder mehrere Verbindungen der Formel I und/oder deren physiologisch verträgliche Salze enthalten. Ferner können sie neben mindestens einer Verbindung der Formel I und/oder ihren physiologisch verträglichen Salzen noch einen oder mehrere andere therapeutisch oder prophylaktisch wirksame Stoffe enthalten, zum Beispiel Stoffe mit entzündungshemmender Wirkung. Die pharmazeutische Präparate enthalten normalerweise 0,2 bis 500 mg, vorzugsweise 1 bis 100 mg Wirkstoff der Formel I und/oder dessen physiologisch verträgliche Salze.

Die Verbindungen der Formel I haben die Fähigkeit, Zell-Zell- und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann beispielsweise in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird. Außer als Entzündungshemmstoffe eignen sich die Verbindungen der Formel I und ihre physiologisch verträglichen Salze daher generell zur Therapie und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Therapie und Prophylaxe von Krankheiten, die zumindest teilweise durch ein unerwünschtes Ausmaß an Leukozytenadhäsion und/oder Leukozytenmigration verursacht werden oder damit verbunden sind, oder zu deren Vorbeugung, Linderung oder Heilung die Adhäsion und/oder Migration von Leukozyten verringert werden soll.

Die Verbindungen der Formel I können bei entzündlichen Erscheinungen unterschiedlichster Ursache als Entzündungshemmer eingesetzt werden. Anwendung finden sie beispielsweise zur Therapie oder Prophylaxe der rheumatoiden Arthritis, der inflammatorischen bowel disease (ulcerativen Colitis), des systemischen Lupus erythematosus oder zur Therapie oder Prophylaxe von inflammatorischen Erkrankungen des zentralen Nervensystems wie zum Beispiel der multiplen Sklerose, zur Therapie oder Prophylaxe von Asthma oder von Allergien, zum Beispiel Allergien vom verzögerten Typ (Typ IV-Allergie). Weiterhin eignen sie sich zur Therapie oder Prophylaxe von cardiovaskulären Erkrankungen, der Arteriosklerose, von Restenosen, zur Therapie oder Prophylaxe von Diabetes, zur Verhinderung der Schädigung von Organtransplantaten, zur Hemmung von Tumorwachstum oder Tumormetastasierung bei verschiedenen Malignitäten, zur Therapie der Malaria sowie von weiteren Krankheiten, bei denen eine Blockierung des Integrins VLA-4 und/oder eine Beeinflussung der Leukozytenaktivität zur Vorbeugung, Linderung oder Heilung angebracht erscheint.

Die Dosis bei der Anwendung der Verbindungen der Formel I kann innerhalb weiter Grenzen variieren und ist wie üblich in jedem einzelnen Fall den individuellen Gegebenheiten anzupassen. Sie hängt beispielsweise von der eingesetzten Verbindung oder von der Art und Schwere der zu behandelnden Krankheit ab oder davon, ob ein akuter oder chronischer Krankheitszustand behandelt wird oder ob Prophylaxe betrieben wird. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg (jeweils pro kg Körpergewicht) bei einem ca. 75 kg schweren Erwachsenen zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3 oder 4, Teilverabreichungen aufgeteilt werden. Gegebenenfalls kann es, je nach individuellem Verhalten, erforderlich werden, von der angegebenen Tagesdosis nach oben oder nach unten abzuweichen.

Gegenstand der vorliegenden Erfindung sind daher auch die Verbindungen der Formel I zur Hemmung der Adhäsion und/oder Migration von Leukozyten oder zur Hemmung des VLA-4-Rezeptors und die Verwendung der Verbindungen der Formel I zur Herstellung von Arzneimitteln dafür, also von Arzneimitteln zur Therapie oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, sowie die Verwendung der Verbindungen der Formel I und/oder ihrer physiologisch verträglichen Salze bei der Therapie und Prophylaxe derartiger Krankheiten.

Die Verbindungen der Formel I und ihre Salze können weiterhin für diagnostische Zwecke, zum Beispiel in in-vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird. Weiterhin können sie als Zwischenprodukte für die Herstellung anderer Verbindungen dienen, insbesondere anderer Arzneimittelwirkstoffe, die aus den Verbindungen der Formel I beispielsweise durch Abwandlung oder Einführung von Resten oder funktionellen Gruppen erhältlich sind.

### Beispiele

Die Verbindungen wurden über Massenspektren (MS) und/oder NMR-Spektren identifiziert. Verbindungen, die durch Chromatographie unter Verwendung eines Laufmittels gereinigt wurden, das beispielsweise Essigsäure oder Trifluoressigsäure enthielt, und anschließend gefriergetrocknet wurden, enthielten zum Teil je nach Durchführung der Gefriertrocknung noch die aus dem Laufmittel stammende Säure, sind also teilweise oder vollständig in Form eines Salzes der verwendeten Säure, zum Beispiel in Form des Essigsäuresalzes oder Trifluoressigsäuresalzes, angefallen.

Es bedeuten:

| | |
|---|---|
| DMF | N,N-Dimethylformamid |
| THF | Tetrahydrofuran |
| DCC | N,N'-Dicyclohexylcarbodiimid |
| HOBt | 1-Hydroxybenzotriazol |
| TOTU | O-(Cyan(ethoxycarbonyl)methylenamino)-1,1,3,3-tetramethyluronium-tetrafluoroborat |

### Beispiel 1

### ((R,S)-2-((S)-4-Phenyl-3-benzyl-4-methyl-2,5-dioxo-imidazolidin-1-yl)-2-(2-methylpropyl)-acetyl)-L-aspartyl-L-phenylglycin

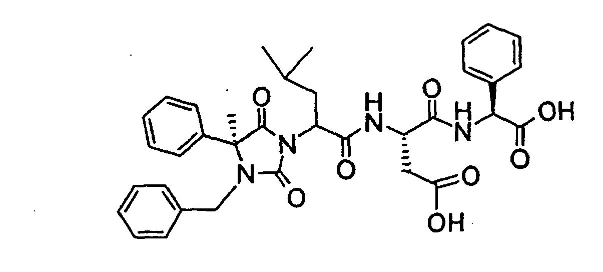

### 1a) (R,S)-2-Brom-4-methylpentansäure-tert-butylester (1.1)

Man gab zu einer Lösung von 2,5 g (12,8 mmol) (R,S)-2-Brom-4-methylpentansäure in 80 ml Chloroform und 80 ml tert-Butylacetat 1,96 ml konzentrierte Schwefelsäure und 0,515 ml Oleum (20 %ig) und ließ das Gemisch 3 h bei Raumtemperatur rühren. Dann stellte man durch Zugabe von 10 %iger NaHCO₃-Lösun einen pH-Wert von 4 ein. Die wäßrige Phase wurde abgetrennt und 2 x mit Dichlormethan extrahiert. Die vereinigten organischen Phasen wurden über Natriumsulfat getrocknet. Nach Filtration und Einengen des Filtrats im Vakuum erhielt man 2,62 g (82 %) 1.1.

### 1b) (R,S)-2-((S)-4-(4-Brom-phenyl)-4-methyl-2,5-dioxo-imidazolidin-1-yl)-4-methyl-pentansäure-tert-butylester (1.2)

Man gab zu einer Lösung von 2,08 g (7,72 mmol) an (S)-4-(4-Brom-phenyl)-4-methyl-2,5-dioxo-imidazolidin in 20 ml absolutem DMF unter Argon bei 0 °C 213 mg (8,87 mmol) Natriumhydrid, ließ 1 h bei Raumtemperatur rühren, gab 1,94 g (7,72 mmol) 1.1 zu, rührte 5 h bei Raumtemperatur und ließ das Gemisch über Nacht bei Raumtemperatur stehen. Das Lösungsmittel wurde im Vakuum entfernt, der Rückstand in Essigester aufgenommen und die Essigester-Lösung mit Wasser gewaschen. Die organische Phase wurde über Natriumsulfat getrocknet, das Trockenmittel abfiltriert und das Filtrat im Vakuum eingeengt. Der Rückstand wurde mit Heptan/Essigester (2:1) über Kieselgel chromatographiert. Nach Einengen der Produktfraktionen erhielt man 2,45 g (72 %) 1.2.

### 1c) (R,S)-2-((S)-4-(4-Brom-phenyl)-3-benzyl-4-methyl-2,5-dioxo-imidazolidin-1-yl)-4-methyl-pentansäure-tert-butylester (1.3)

Man gab zu einer Lösung von 1,92 g (4,37 mmol) 1.2 in 10 ml absolutem DMF unter Argon bei 0 °C 126 mg (5,24 mmol) Natriumhydrid, ließ 1 h bei Raumtemperatur rühren, gab 570 µl (4,8 mmol) Benzylbromid zu und ließ erneut 1 h bei Raumtemperatur rühren. Das Lösungsmittel wurde im Vakuum entfernt, der Rückstand zwischen Wasser und Essigester verteilt und nach Phasentrennung die Wasserphase mit Essigester extrahiert. Die vereinigten organischen Phasen wurden über Natriumsulfat getrocknet, das Trockenmittel wurde abfiltriert und das Filtrat im Vakuum eingeengt. Man erhielt 2,17 g (94 %) 1.3.

### 1d) (R,S)-2-((S)-4-Phenyl-3-benzyl-4-methyl-2,5-dioxo-imidazolidin-1-yl)-4-methyl-pentansäure (1.4)

Eine Lösung von 1 g (1,88 mmol) 1.3 in 100 ml Ethanol wurde über 40 mg 10 % Pd/C hydriert. Nach 2 h wurde der Katalysator abfiltriert, das Filtrat im Vakuum eingeengt, der Rückstand in Essigester gelöst und die Lösung mit 10 %iger NaHCO₃-Lösung und Wasser gewaschen und über Natriumsulfat getrocknet. Nach Filtration und Entfernen des Lösungsmittels im Vakuum wurde der Rückstand mit 10 ml 90 %iger Trifluoressigsäure versetzt. Nach 15 min bei Raumtemperatur wurde die Trifluoressigsäure im Vakuum entfernt und der Rückstand 2 x mit Toluol eingeengt. Man erhielt 740 mg (100 %) 1.4.

### 1e) ((R,S)-2-((S)-4-Phenyl-3-benzyl-4-methyl-2,5-dioxo-imidazolidin-1-yl)-2-(2-methyl-propyl)-acetyl)-L-aspartyl-L-phenylglycin (1.5)

Zu einer Lösung von 200 mg (0,507 mmol) 1.4 und 210 mg (0,507 mmol) H-Asp(O^{t}Bu)-Phg-O^{t}Bu-Hydrochlorid in 10 ml absolutem DMF wurden 166 mg (0,507 mmol) TOTU und 172 µl (1,014 mmol) Diisopropyl-ethylamin gegeben. Nach 2 h Rühren bei Raumtemperatur wurde das Reaktionsgemisch im Vakuum eingeengt, der Rückstand in Essigester aufgenommen und die organische Phase 2 x mit gesättigter NaHCO₃-Lösung und Wasser gewaschen. Nach Trocknen über Natriumsulfat, Filtration und Einengen des Filtrats im Vakuum érhielt man 393 mg Rohprodukt, das mit Heptan/Essigester (3:1) über Kieselgel chromatographiert wurde. Nach Einengen der Produktfraktionen wurde der Rückstand in 5 ml 90 %iger Trifluoressigsäure gelöst, nach 15 min bei Raumtemperatur die Trifluoressigsäure im Vakuum entfernt und der Rückstand in 20 %iger Essigsäure gelöst und gefriergetrocknet. Man erhielt 219 mg (67 %) 1.5.
ES(+)-MS: 643,3 (M+H)⁺

### Beispiel 2

### (S)-3-((R, S)-2-((S)-4-Phenyl-3-benzyl-4-methyl-2,5-dioxo-imidazolidin-1-yl)-2-(2-methyl-propyl)-acetylamino)-2-benzyloxycarbonylamino-propionsäure

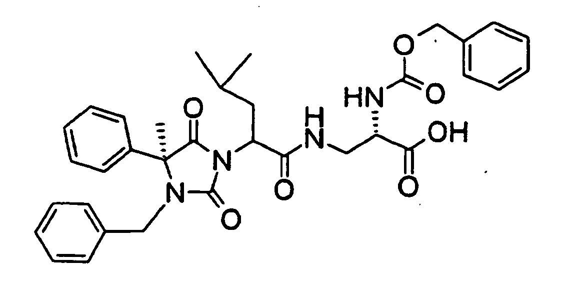

Die Verbindung wurde durch Reaktion von (R,S)-2-((S)-4-Phenyl-3-benzyl-4-methyl-2,5-dioxo-imidazolidin-1-yl)-4-methyl-pentansäure (1.4) und (S)-3-Amino-2-benzyloxycarbonylamino-propionsäure-tert-butylester analog der Herstellung von 1.4 hergestellt, wobei nach Spaltung des tert-Butylesters und Entfernen der Trifluoressigsäure im Vakuum der Rückstand mit Dichlormethan/Methanol/Essigsäure/Wasser (9:1:0,1:0,1) über Kieselgel chromatographiert wurde.
ES(+)-MS: 615,4 (M+H)⁺

Der (S)-3-Amino-2-benzyloxycarbonylamino-propionsäure-tert-butylester wurde wie folgt hergestellt. 10 g (42 mmol) (S)-3-Amino-2-benzyloxycarbonylamino-propionsäure wurden in einem Gemisch aus 100 ml Dioxan, 100 ml Isobutylen und 8 ml konz. H₂SO₄ 3 Tage bei 20 atm N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2 x mit je 100 ml Diethylether extrahiert. Die vereinigten organischen Phasen wurden mit 2 x 100 ml Wasser gewaschen und über Na₂SO₄ getrocknet. Nach Entfernen des Lösungsmittels im Vakuum erhielt man 9,58 g (78 %) (S)-3-Amino-2-benzyloxycarbonylamino-propionsäure-tert-butylester als blaßgelbes Öl.

### Beispiel 3

### (R,S)-3-((R,S)-2-((S)-4-Phenyl-3-benzyl-4-methyl-2,5-dioxo-imidazolidin-1-yl)-2-(2-methyl-propyl)-acetylamino)-3-(3,4-methylendioxy-phenyl)-propionsäure

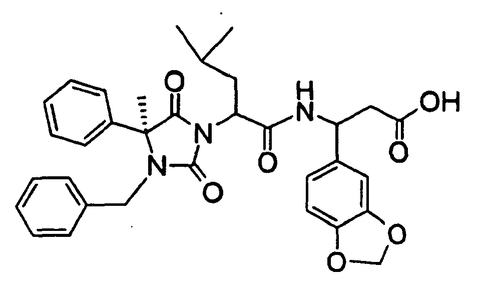

Die Verbindung wurde hergestellt durch Umsetzung von 1.4 mit (R,S)-3-Amino-3-(3,4-methylendioxy-phenyl)-propionsäure-tert-butylester-hydrochlorid und anschließende Spaltung des tert-Butylesters wie in Beispiel 1 beschrieben.
ES(+)-MS: 586,3 (M+H)⁺

Das (R,S)-3-Amino-3-(3,4-methylendioxy-phenyl)-propionsäure-tert-butylester-hydrochlorid wurde hergestellt, indem analog W. M. Radionow, E.A.Postovskaya, J. Am. Chem. Soc. 1929, 51, 841 (siehe auch Houben-Weyl, Methoden der Organischen Chemie, Band XI/2, Georg Thieme Verlag, Stuttgart, 1958, S. 497), zunächst die entsprechende β-Aminosäure hergestellt wurde. Diese wurde in das Benzyloxycarbonylamino-Derivat überführt, aus dem dann nach der folgenden Synthesevorschrift der tert-Butylester erhalten wurde: Zu 1 mmol der 3-Benzyloxycarbonylamino-carbonsäure in 13 ml absolutem Dichlormethan wurden 1,5 mmol Oxalylchlorid gegeben. Nach 4 h Rühren bei Raumtemperatur wurde das Reaktionsgemisch eingeengt und zum Rückstand 6,5 ml tert-Butanol gegeben. Es wurde 1 h bei Raumtemperatur gerührt und das Reaktionsgemisch im Vakuum eingeengt. Der Rückstand wurde in Essigester aufgenommen und 2 x mit gesättigter NaHCO₃-Lösung und Wasser extrahiert. Die organische Phase wurde über Natriumsulfat getrocknet und nach Filtration das Lösungsmittel im Vakuum entfernt. Zur Herstellung des ß-Aminosäure-tert-butylester-hydrochlorids wurde anschließend die Benzyloxycarbonylgruppe über 10 % Pd/C in Methanol/HCl abhydriert.

### Beispiel 4

### (S)-3-((R, S)-2-((R,S)-4-Phenyl-3-benzyl-4-methyl-2,5-dioxo-imidazolidin-1-yl)-2-isopropyl-acetylamino)-2-(1-adamantylmethyloxycarbonylamino)-propionsäure

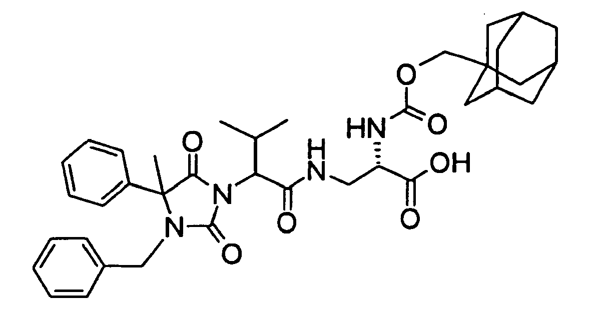

Die Verbindung wurde hergestellt durch Umsetzung von (R,S)-2-((R,S)-3-Benzyl-4-phenyl-4-methyl-2,5-dioxo-imidazolidin-1-yl)-2-isopropyl-essigsäure (hergestellt analog den Vorschriften in Beispiel 1 aus (R,S)-4-Methyl-4-phenyl-2,5-dioxo-imidazolidin) mit (S)-3-Amino-2-(1-adamantylmethyloxycarbonylamino)-propionsäure-tert-butylester und anschließende Spaltung des tert-Butylesters wie in Beispiel 1 beschrieben. Das Rohprodukt wurde mittels präparativer HPLC über RP-18 gereinigt.
ES(+)-MS: 659,4 (M+H)⁺

Der (S)-3-Amino-2-(1-adamantylmethyloxycarbonylamino)-propionsäure-tert-butylester wurde wie folgt hergestellt.

Man gab zu einer Lösung von 10 g (34 mmol) (S)-3-Amino-2-benzyloxycarbonylaminopropionsäure-tert-butylester (siehe Beispiel 2) in 600 ml THF/Wasser (2:1) bei 0 °C 8,9 g (40,8 mmol) Di-tert-butyl-dicarbonat und anschließend portionsweise 1 N NaOH, so daß der pH der Lösung zwischen 9 und 10 lag (Verbrauch an 1 N NaOH: 32 ml). Nach 3 h Rühren bei Raumtemperatur gab man 1 I Wasser zu und extrahierte 3 mal mit Diethylether. Nach Trocknen der organischen Phase über Natriumsulfat, Filtration und Entfernen des Lösungsmittels im Vakuum wurde der Rückstand mit Dichlormethan/Methanol (20:1) über Kieselgel chromatographiert. Man erhielt 13,19 g (98 %) (S)-2-Benzyloxycarbonylamino-3-tert-butoxycarbonylamino-propionsäure-tert-butylester.

13,1 g (S)-2-Benzyloxycarbonylamino-3-tert-butoxycarbonylamino-propionsäuretert-butylester wurden in Methanol/HCl über 10 % Pd/C hydriert. Nach 1,5 h wurde filtriert und das Filtrat im Vakuum eingeengt. Man erhielt 9,77 g (99 %) (S)-2-Amino-3-tert-butoxycarbonylamino-propionsäure-tert-butylester-hydrochlorid als farblosen Feststoff.

Eine Lösung von 10,9 g (65,4 mmol) 1-Hydroxymethyl-adamantan und 10,6 g (65,4 mmol) Carbonyldiimidazol in 60 ml THF wurde 1,5 h bei 50°C gerührt. Man gab 9,7 g (32,7 mmol) (S)-2-Amino-3-tert-butoxycarbonylamino-propionsäure-tert-butylesterhydrochlorid in 25 ml THF und 5,6 ml (32,7 mmol) Diisopropyl-ethylamin zu, rührte 4 h bei 60°C und ließ über Nacht bei Raumtemperatur stehen. Das Lösungsmittel wurde im Vakuum entfernt und der Rückstand mit Heptan/Essigester (7:3) über Kieselgel chromatographiert. Man erhielt 8,7 g (59 %) (S)-2-(1-Adamantylmethyloxycarbonylamino)-3-tert-butoxycarbonylamino-propionsäure-tert-butylester als farbloses Öl.

Eine Lösung von 8,7 g (19,22 mmol) an (S)-2-(1-Adamantylmethyloxycarbonylamino)-3-tert-butoxycarbonylamino-propionsäure-tert-butylester in 180 ml Trifluoressigsäure/Dichlormethan (1:1) wurde nach 1 min in 1,5 I eiskalte NaHCO₃-Lösun gegeben, das Gemisch dreimal mit Dichlormethan extrahiert und die Dichlormethan-Phasen anschließend über Natriumsulfat getrocknet. Nach Filtration und Entfernen des Lösungsmittels im Vakuum erhielt man 6,35 g (94 %) an (S)-3-Amino-2-(1-adamantylmethyloxycarbonylamino)-propionsäure-tert-butylester als farblosen Feststoff.

### Beispiel 5

### ((R,S)-4-(4-Pyridyl)-3-benzyl-4-methyl-2,5-dioxo-imidazolidin-1-yl)-acetyl-L-aspartyl-L-phenylglycin

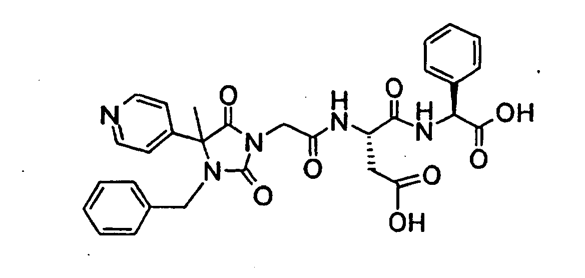

### 5a) (R,S)-4-(4-Pyridyl)-4-methyl-2,5-dioxo-imidazolidin (5.1)

36,34 g (300 mmol) 4-Acetylpyridin und 259,2 g (2,694 mol) Ammoniumcarbonat wurden in 400 ml 50 %igem Äthanol suspendiert. Dazu gab man 25,5 g (392 mmol) Kaliumcyanid. Man rührte 5 Stunden bei 50 - 60 °C, ließ das Gemisch auf Raumtemperatur abkühlen, stellte durch Zugabe von 6 N HCl den pH-Wert auf 6,3 ein und ließ über Nacht bei Raumtemperatur stehen. Man stellte erneut einen pH-Wert von 6,3 ein und entfernte das Lösungsmittel im Vakuum. Der Rückstand wurde mehrmals mit Dichlormethan aufgeschlämmt. Die unlöslichen Anteile wurden jeweils abfiltriert und die vereinigten Filtrate im Vakuum eingeengt. Der Rückstand wurde mit Dichlormethan/Methanol über Kieselgel chromatographiert. Nach Einengen der Produktfraktionen erhielt man 37,53 g (65 %) 5.1.

### 5b) ((R,S)-4-(4-Pyridyl)-3-benzyl-4-methyl-2,5-dioxo-imidazolidin-1-yl)-acetyl-L-aspartyl-L-phenylglycin (5.2)

Man gab zu einer Lösung von 50 mg (0,133 mmol) an ((R,S)-4-(4-Pyridyl)-3-benzyl-4-methyl-2,5-dioxo-imidazolidin-1-yl)-essigsäure-hydrochlorid (hergestellt durch Spaltung von ((R,S)-4-(4-Pyridyl)-3-benzyl-4-methyl-2,5-dioxo-imidazolidin-1-yl)-essigsäure-tert-butylester mit 90 %iger Trifluoressigsäure und anschließende Überführung in das Hydrochlorid, wobei der ((R,S)-4-(4-Pyridyl)-3-benzyl-4-methyl-2,5-dioxo-imidazolidin-1-yl)-essigsäure-tert-butylester durch Alkylierung von 5.1 erst mit Bromessigsäure-tert-butylester und anschließend mit Benzylbromid analog Beispiel 1 hergestellt wurde) und 55 mg (0,133 mmol) H-Asp(O^{t}Bu)-Phg-(O^{t}Bu) x HCl in 10 ml absolutem DMF 43,6 mg TOTU und 68 µl Diisopropyl-ethylamin. Nach 3 d bei Raumtemperatur wurde das Lösungsmittel im Vakuum entfernt, der Rückstand in Essigester aufgenommen, die Lösung mit gesättigter NaHCO₃-Lösung, Wasser und KHSO₄/K₂SO₄-lösung gewaschen und über Natriumsulfat getrocknet. Nach Filtration wurde das Lösungsmittel im Vakuum entfernt und der Rückstand mit 10 ml 90 %iger Trifluoressigsäure versetzt. Nach 1 h bei Raumtemperatur wurde die Trifluoressigsäure im Vakuum entfernt, der Rückstand zwischen Diethylether und Wasser verteilt, die wäßrige Phase gefriergetrocknet und der Rückstand durch zweimalige Chromatographie über Kieselgel gereinigt. Man erhielt 19,5 mg (25 %) 5.2.
ES(+)-MS: 588,3 (M+H)⁺

### Beispiel 6

### ((R,S)-2-((R,S)-4-(4-Pyridyl)-3-benzyl-4-methyl-2,5-dioxo-imidazolidin-1-yl)-2-(2-methyl-propyl)-acetyl)-L-aspartyl-L-phenylglycin

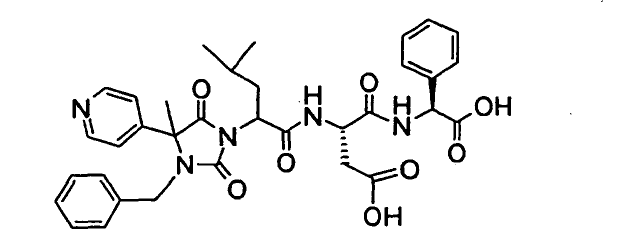

### 6a) (R,S)-2-((R,S)-4-(4-Pyridyl)-3-benzyl-4-methyl-2,5-dioxo-imidazolidin-1-yl)-2-(2-methyl-propyl)-essigsäure-tert-butylester (6.1)

Man gab zu einer Lösung von 4,1 g (21,44 mmol) (R,S)-4-(4-Pyridyl)-4-methyl-2,5-dioxo-imidazolidin (siehe Beispiel 5) in 30 ml absolutem DMF unter Eiskühlung 1,03 g (23,58 mmol) Natriumhydrid. Man ließ 15 min bei Raumtemperatur rühren und gab dann 4,23 g (21,44 mmol) (R,S)-2-Brom-4-methyl-pentansäure-tert-butylester zu. Nach 2 h Rühren und Stehen über Nacht bei Raumtemperatur wurde das Lösungsmittel im Vakuum entfernt und der Rückstand mit Dichlormethan/Methanol (95:5) über Kieselgel chromatographiert. Man erhielt 1,2 g (15 %) (R,S)-2-((R,S)-4-(4-Pyridyl)-4-methyl-2,5-dioxo-imidazolidin-1-yl)-2-(2-methyl-propyl)-essigsäure-tert-butylester, der analog Beispiel 1 durch Reaktion mit Benzylbromid zu 6.1 umgesetzt wurde.

### 6b) (R,S)-2-((R,S)-4-(4-Pyridyl)-3-benzyl-4-methyl-2,5-dioxo-imidazolidin-1-yl)-2-(2-methyl-propyl)-essigsäure-hydrochlorid (6.2)

1,4 g (3,1 mmol) 6.1 in 30 ml 90 %iger Trifluoressigsäure wurden 1 h bei Raumtemperatur gerührt. Die Trifluoressigsäure wurde im Vakuum entfernt und der Rückstand zwischen Diethylether und Wasser verteilt. Die Phasen wurden getrennt, die organische Phase eingeengt und der Rückstand mit Dichlormethan/Methanol/Essigsäure/Wasser (9,5:0,5:0,05:0,05) über Kieselgel gereinigt. Man erhielt 650 mg (47 %) 6.2.

### 6c) ((R,S)-2-((R,S)-4-(4-Pyridyl)-3-benzyl-4-methyl-2,5-dioxo-imidazolidin-1-yl)-2-(2-methyl-propyl)-acetyl)-L-aspartyl-L-phenylglycin

Die Verbindung wurde analog Beispiel 5 durch Reaktion von 6.2 mit H-Asp(O^{t}Bu)-Phg-(O^{t}Bu) x HCl und anschließende Spaltung der tert-Butylester hergestellt. ES(+)-MS: 644,3 (M+H)⁺

### Beispiel 7

### ((R,S)-4-Phenyl-3-benzyl-4-methyl-2,5-dioxo-imidazolidin-1-yl)-acetyl-L-aspartyl-L-phenylglycin

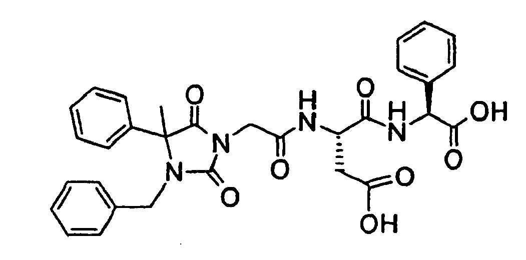

Die Verbindung wurde hergestellt durch Umsetzung von ((R,S)-4-(4-Phenyl)-3-benzyl-4-methyl-2,5-dioxo-imidazolidin-1-yl)-essigsäure (hergestellt analog Beispiel 1 aus (R,S)-4-Phenyl-4-methyl-2,5-dioxo-imidazolidin durch Alkylierung mit Chloressigsäure-methylester und anschließend mit Benzylbromid und nachfolgende Spaltung des Methylesters) mit H-Asp(O^{t}Bu)-Phg-(O^{t}Bu) x HCl analog Beispiel 1 und anschließende Spaltung der tert-Butylester.
ES(+)-MS: 587,1 (M+H)⁺

### Beispiel 8 (Vergleichsbeispiel)

### ((S)-4-(4-Hydroxymethyl-phenyl)-3-benzyl-4-methyl-2,5-dioxo-imidazolidin-1-yl)-acetyl-L-aspartyl-L-phenylglycin

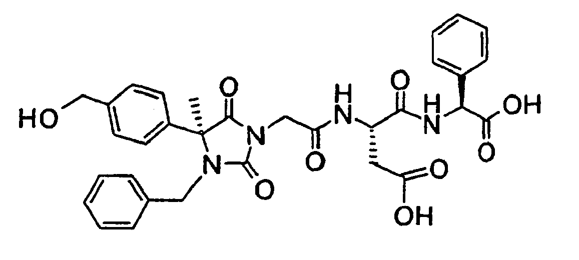

### 8a) ((S)-4-(4-Cyanphenyl)-3-benzyl-4-methyl-2,5-dioxo-imidazolidin-1-yl)-essigsäure-benzylester (8.1)

Man gab zu einer Lösung von 20 g (73,1 mmol) ((S)-4-(4-Cyanphenyl)-4-methyl-2,5-dioxo-imidazolidin-1-yl)-essigsäure in 120 ml absolutem DMF unter Eiskühlung 7,73 g (160,8 mmol) Natriumhydrid. Nach 30 min Rühren bei Raumtemperatur wurden 19 ml (160,8 mmol) Benzylbromid zugegeben. Das Reaktionsgemisch wurde 2 h bei Raumtemperatur gerührt, über Nacht stehen gelassen, das Lösungsmittel im Vakuum entfernt und der Rückstand mit Heptan/Essigester (2:1) über Kieselgel chromatographiert. Man erhielt 11,43 g (35 %) 8.1.

### 8b) ((S)-4-(4-Formyl-phenyl)-3-benzyl-4-methyl-2,5-dioxo-imidazolidin-1-yl)-essigsäure-benzylester (8.2)

Man gab zu einer Lösung von 6,08 g (13,42 mmol) 8.1 in 200 ml Pyridin/Essigsäure/Wasser (2:1:1) bei 0 °C 24,3 g Natriumhypophosphit x H₂O und 4,02 g Raney-Nickel und erhitzte das Reaktionsgemisch 8 h auf 60°C. Nach Abkühlen auf Raumtemperatur und Filtration wurde das Reaktionsgemisch im Vakuum eingeengt, der Rückstand in Essigester aufgenommen und die Essigesterphase 2 x mit Wasser, 2 x mit 10 %iger Zitronensäure-Lösung, 2 x mit gesättigter NaHCO₃-Lösung und mit gesättigter Kochsalz-Lösung extrahiert. Die organische Phase wurde über Magnesiumsulfat getrocknet und nach Filtration das Lösungsmittel im Vakuum entfernt. Man erhielt 4,82 g (79 %) 8.2.

### 8c) ((S)-4-(4-Hydroxymethyl-phenyl)-3-benzyl-4-methyl-2,5-dioxo-imidazolidin-1-yl)-essigsäure (8.3)

Man gab zu einer Lösung von 500 mg (1,1 mmol) 8.2 in 50 ml Ethanol 20 ml Wasser und anschließend bei 0 °C 22 mg (0,6 mmol) Natriumborhydrid. Nach 40 min Rühren bei 0 °C wurde das Reaktionsgemisch im Vakuum eingeengt, der Rückstand in 30 ml 6 N Salzsäure/THF (1:1) 12 h bei 50°C erhitzt und das Reaktionsgemisch über Nacht bei Raumtemperatur stehen lassen. Das Gemisch wurde mit Dichlormethan extrahiert und die organische Phase über Natriumsulfat getrocknet. Nach Filtration wurde das Lösungsmittel im Vakuum entfernt, der Rückstand mit Wasser versetzt und gefriergetrocknet. Man erhielt 440 mg rohes 8.3, das ohne weitere Reinigung im nächsten Syntheseschritt eingesetzt wurde.

### 8d) ((S)-4-(4-Hydroxymethyl-phenyl)-3-benzyl-4-methyl-2,5-dioxo-imidazolidin-1-yl)-acetyl-L-aspartyl-L-phenylglycin (8.4)

Eine Lösung von 200 mg (0,54 mmol) rohem 8.3, 225 mg (0,54 mmol) H-Asp(O^{t}Bu)-Phg-(O^{t}Bu) x HCl und 178 mg (0,54 mmol) TOTU wurde mit 185 µl (1,08 mmol) Diisopropyl-ethylamin versetzt. Nach 1 h bei Raumtemperatur wurde das Lösungsmittel im Vakuum entfernt, der Rückstand in Essigester gelöst und die Essigesterphase jeweils 2 x mit KHSO₄K₂SO₄-Lösung, gesättigter NaHCO₃-Lösung und gesättigter Kochsalz-Lösung extrahiert. Nach Phasentrennung wurde die organische Phase über Natriumsulfat getrocknet. Nach Filtration wurde das Lösungsmittel im Vakuum entfernt und der Rückstand durch Chromatographie mit Methyl-tert-butylether/Heptan (8:2) über Kieselgel gereinigt. Nach Einengen der Produktfraktionen wurde der Rückstand in 5 ml 90 %iger Trifluoressigsäure gelöst. Nach 1 h bei Raumtemperatur wurde die Trifluoressigsäure im Vakuum entfernt und der Rückstand mittels präparativer HPLC über RP-18 gereinigt. Man erhielt nach Gefriertrocknung 44 mg (13 %) 8.4.
ES(+)-MS: 617,2 (M+H)⁺

### Beispiel 9 (vergleichsbeipiel)

### (S)-3-(((S)-4-(4-Hydroxymethyl-phenyl)-3-benzyl-4-methyl-2,5-dioxo-imidazolidin-1-yl)-acetylamino)-2-(1-adamantylmethyloxycarbonylamino)-propionsäure

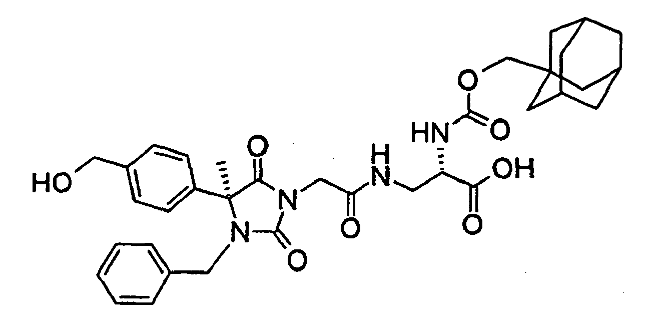

Die Herstellung erfolgte analog Beispiel 8 durch Kupplung von 8.3 mit (S)-2-(1-Adamantylmethyloxycarbonylamino)-3-amino-propionsäure-tert-butylester (siehe Beispiel 4) an Stelle von H-Asp(O^{t}Bu)-Phg-(O^{t}Bu) x HCl. Nach Spaltung des, tert-Butylesters mit 90 %iger Trifluoressigsäure wurde das Rohprodukt zwischen Wasser und Dichlormethan verteilt. Die organische Phase wurde abgetrennt, über Natriumsulfat getrocknet und nach Filtration das Lösungsmittel im Vakuum entfernt. Der Rückstand wurde durch präparative HPLC über RP-18 gereinigt.
ES(+)-MS: 647,3 (M+H)⁺

### Beispiel 10 (Vergleichsbeispiel)

### ((R,S)-4-(4-Hydroxyphenyl)-3-benzyl-4-methyl-2,5-dioxo-imidazolidin-1-yl)-acetyl-L-aspartyl-L-phenylglycin

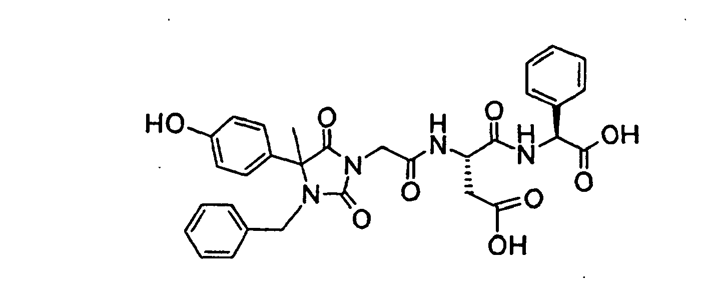

### 10a) 1-(4-(Tetrahydropyran-2-yloxy)-phenyl)-ethanon (10.1)

13,62 g (100 mmol) 4-Hydroxyacetophenon und 10,04 ml (110 mmol) 3,4-Dihydro-2H-pyran wurden in 100 ml wasserfreiem Methylenchlorid suspendiert. Bei 0 °C wurden unter Rühren 190 mg (1 mmol) p-Toluolsulfonsäure zugesetzt und 3 Stunden bei 0 °C gerührt. Es wurden nochmals 10.04 ml (110 mmol) 3,4-Dihydro-2H-pyran zugesetzt und weitere 3 Stunden bei Raumtemperatur gerührt. Der Ansatz wurde in 150 ml Wasser gegossen, die Phasen wurden getrennt und die organische Phase wurde mit gesättigter NaHCO₃-Lösung, gesättigter NaCl-Lösung sowie mit Wasser extrahiert. Die organische Phase wurde über Natriumsulfat getrocknet, eingeengt und zur Reinigung über Kieselgel (70-200 µm) mit Methylenchlorid als Laufmittel chromatographiert. Man erhielt 13,65 g (62 %) 10.1.

### 10b)(R,S)-4-Methyl-4-(4-(tetrahydropyran-2-yloxy)-phenyl)-2,5-dioxo-imidazolidin (10.2)

11,01 g (50 mmol) 10.1 und 42,3 g (440 mmol) Ammoniumcarbonat wurden in 200 ml 50 %igem Äthanol suspendiert. Dazu gab man 4,23 g (65 mmol) Kaliumcyanid. Man rührte 5 Stunden bei 50 bis 60 °C. Es entstand nach kurzer Zeit eine klare Lösung. Man ließ über Nacht bei Raumtemperatur stehen und rührte anschließend bei 60 °C 6 Stunden weiter. Mit 6 N HCl wurde der pH-Wert auf 6,3 eingestellt und das Gemisch 2 h unter Eiskühlung rühren lassen. Der Niederschlag wurde abgesaugt, mit Wasser gewaschen und über Phosphorpentoxid im Exikkator getrocknet. Man erhielt 9,5 g (65 %) 10.2.

### 10c) ((R,S)-4-Methyl-4-(4-(tetrahydropyran-2-yloxy)-phenyl)-2,5-dioxo-imidazolidin-1-yl)-essigsäure-methylester (10.3)

230 mg (10 mmol) Natrium wurden unter Argon in 25 ml wasserfreiem Methanol gelöst. Es wurden 2,9 g (10 mmol) 10.2 zugesetzt. Man erhitzte unter Rühren 2 Stunden zum Rückfluß. Anschließend gab man 1,66 g (10 mmol) Kaliumiodid zu und tropfte innerhalb von 15 Minuten eine Lösung von 0,975 ml (10 mmol) Chloressigsäuremethylester in 1,1 ml wasserfreiem Methanol zu. Es wurde 4 Stunden zum Rückfluß erhitzt und anschließend über Nacht bei Raumtemperatur stehen gelassen. Weitere 0,195 ml (2 mmol) Chloressigsäuremethylester in 0,22 ml wasserfreiem Methanol wurden zugesetzt und der Ansatz wurde 4 Stunden unter Rückfluß gerührt. Der Niederschlag wurde abgesaugt und das Filtrat eingeengt. Der Rückstand wurde in Methylenchlorid gelöst, vom Unlöslichen abfiltriert und mit Methylenchlorid/Essigester (9:1) über Kieselgel chromatographiert. Man erhielt 2,56 g (71 %) 10.3.

### 10d) ((R,S)-3-Benzyl-4-methyl-4-(4-(tetrahydropyran-2-yloxy)-phenyl)-2,5-dioxo-imidazolidin-1-yl)-essigsäure-methylester (10.4)

2,53 g (7 mmol) 10.3 wurden unter Argon in 8,5 ml wasserfreiem DMF gelöst. Bei 15 °C wurden 370 mg (7,7 mmol) Natriumhydrid (50 %ig in Öl) zugegeben. Man rührte 15 Minuten bei 15 °C und tropfte anschließend 0,91 ml (7,7 mmol) Benzylbromid zu. Man rührte 7,5 Stunden bei Raumtemperatur und ließ über Nacht bei Raumtemperatur stehen. Die klare Lösung wurde im Vakuum eingeengt und der Rückstand wurde zwischen Essigester und Wasser verteilt. Die organische Phase wurde abgetrennt und die wäßrige Phase nochmals mit Essigester gewaschen. Die organischen Phasen wurden vereinigt, mit Wasser gewaschen, über Natriumsulfat getrocknet und eingeengt. Der Rückstand wurde mit Methylenchlorid/Essigester (9,5:0,5) über Kieselgel chromatographiert. Man erhielt 1,59 g (50 %) 10.4.

### 10e) ((R,S)-4-(4-Hydroxy-phenyl)-3-benzyl-4-methy/-2,5-dioxo-imidazolidin-1-yl)-essigsäure (10.5)

1,53 g (3.5 mmol) 10.4 wurden mit 30 ml konzentrierter Salzsäure 3 h unter Rückfluß erhitzt. Nach Einengen der Lösung im Vakuum wurde der Rückstand mit Wasser verrieben, über Nacht gekühlt und anschließend abgesaugt. Man trocknete über Phosphorpentoxid im Exsikkator und erhielt 1,22 g (98 %) 10.5.

### 10f) ((R,S)-4-(4-Hydroxy-phenyl)-3-benzyl-4-methyl-2,5-dioxo-imidazolidin-1-yl)-acetyl-L-aspartyl-L-phenylglycin-di-tert-butylester (10.6)

354 mg (1 mmol) 10.5, 415 mg (1 mmol) H-Asp(O^{t}Bu)-Phg-O^{t}Bu x HCl und 135 mg (1 mmol) HOBt wurden in 10 ml DMF gelöst. Bei 0 °C gab man 0,13 ml (1 mmol) N-Ethylmorpholin und 220 mg (1 mmol) DCC zu. Man rührte 1 Stunde bei 0 °C und 3 Stunden bei Raumtemperatur und ließ über Nacht bei Raumtemperatur stehen. Es wurde abgesaugt und das Filtrat im Vakuum eingeengt. Der Rückstand wurde in Essigester gelöst und mit NaHCO₃-Lösung, K₂SO₄KHSO₄-Lösung und gesättigter Kochsalzlösung gewaschen. Nach Trocknen über Natriumsulfat wurde das Trockenmittel abfiltriert und das Filtrat im Vakuum eingeengt. Der ölige Rückstand wurde mit Diethylether verrieben und die organische Phase eingeengt. Man erhielt 730 mg (100 %) 10.6.

### 10g)((R,S)-4-(4-Hydroxy-phenyl)-3-benzyl-4-methyl-2,5-dioxo-imidazolidin-1-yl)-acetyl-L-aspartyl-L-phenylglycin (10.7)

370 mg (0,52 mmol) 10.6 wurden in 4 ml 90 %iger Trifluoressigsäure gelöst und 1 Stunde bei Raumtemperatur stehen gelassen. Anschließend engte man ein. Der Rückstand wurde mit Diethylether verrieben und abgesaugt. Man erhielt 202 mg (64 %) 10.7.

Die Aspartyl-Phenylglycin-Derivate der Beispiele 12 bis 126 wurden durch Festphasensynthese nach der in Beispiel 11 angegebenen allgemeinen Vorschrift hergestellt.

### Beispiel 11

### Allgemeine Vorschrift zur Herstellung von Aspartyl-Phenylglycin-Derivaten durch Festphasensynthese

### Allgemeines

Die Synthesen am polymeren Träger wurden nach der Synthesesequenz durchgeführt, die im Schema 1 dargestellt ist. Die Reste R⁵⁰ bis R⁵⁵ im Schema 1 haben die Bedeutung der Reste, die sich in der Formel I in der betreffenden Position im Molekül befinden, oder sie können funktionelle Gruppen in geschützter Form oder in Form von Vorstufen enthalten. R⁵⁰ entspricht dem Rest R. R⁵¹ entspricht den Resten R⁴ bzw. R¹⁵, wobei in diesen Resten vorhandene funktionelle Gruppen in geschützter Form oder in Form von Vorstufen vorliegen können (der Rest -NHR⁵¹ kann also beispielsweise für den Rest einer Aminosäure stehen, der formal durch Entfernen eines Wasserstoffatoms von der Aminogruppe erhalten wird). R⁵² entspricht zusammen mit der CH-Gruppe, an die dieser Rest gebunden ist, der Gruppe B (R⁵² entspricht also einem Substituenten an einer für B stehenden Methylengruppe). R⁵³ entspricht R¹³, R⁵⁴ entspricht der Gruppe R¹-A, wobei darin 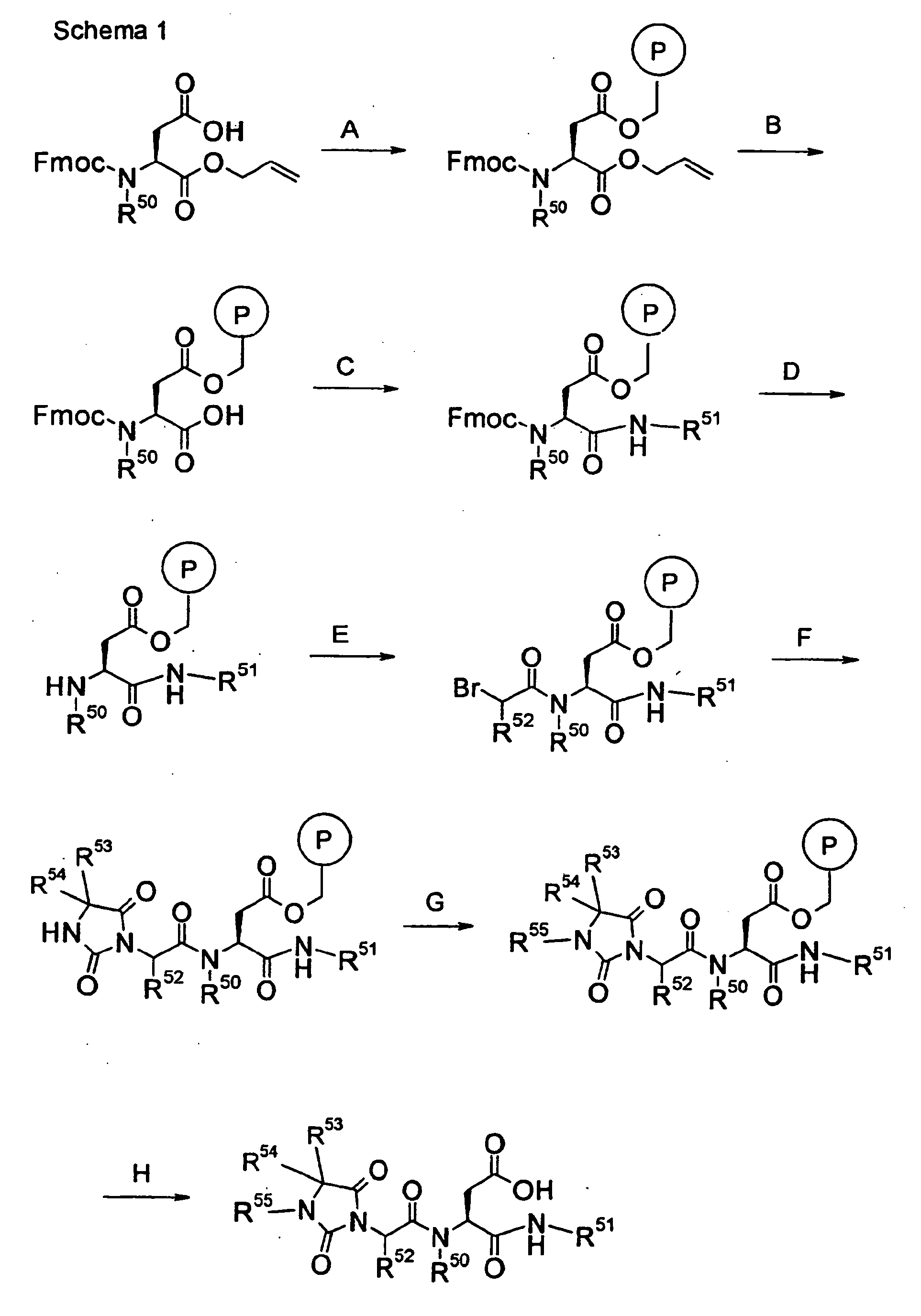 vorhandene funktionelle Gruppen in geschützter Form oder in Form von Vorstufen vorliegen können. R⁵⁵ entspricht der Gruppe R⁰.

Die Synthese von Zwischenstufen in größerem Maßstab wurde in speziellen Reaktionsgefäßen mit eingelassenen Fritten am Boden des Reaktionsgefäßes durchgeführt, die Synthese der Verbindungen der Formel I wurde in Spritzen oder Reaktionsblöcken durchgeführt (Act 496, MultiSynTech). Die Synthesen am Harz wurden durch on bead-Analytik (FT-IR mit ATR-Einheit und MAS-NMR) und Abspaltung einer analytischen Probe vom Harz (HPLC, MS, NMR) verfolgt.

Herstellung des Asparaginsäurebausteines FmocAsp(OH)OAllyl FmocAsp(OtBu)OAllyl (40 g, 88,7 mmol) wurde mit 25 ml Trifluoressigsäure versetzt und 30 min bei Raumtemperatur gerührt. Das Lösungsmittel wurde am Rotationsverdampfer abgezogen. Der Rückstand wurde im Vakuum getrocknet. Man erhielt FmocAsp(OH)OAllyl als gelbes Öl (33,9 g, 97 %).
ES(+)-MS: 395,2 (M+H)⁺

### Anknüpfung an den polymeren Träger (Schritt A in Schema 1)

40 g Wang-Polystyrolharz (1,1 mmol/g; Bachem) wurden 5 min mit 20 ml DMF bei Raumtemperatur vorgequollen. Nach Zugabe einer Lösung von 26,0 g (1,5 Äquivalente) FmocAsp(OH)OAllyl und 34,3 g (1,5 Äquivalente) 1-Benzotriazolyloxytripyrrolidinophosphonium-hexafluorophosphat (PyBOP) und 9,3 ml (1,5 Äquivalente) Düsopropyl-ethylamin in 120 ml DMF wurde das Gemisch 10 h bei 40 °C geschüttelt. Nach beendeter Reaktion wurde die Lösung abgesaugt und das Harz mit DMF gewaschen (5 x 20 ml). Nach Zugabe einer Lösung von Acetanhydrid (10 ml) und Diisopropyl-ethylamin (9,3 ml, 1,5 Äquivalente) in 40 ml DMF wurde das Gemisch erneut für 30 min bei Raumtemperatur geschüttelt. Die Lösung wurde abgesaugt und das Harz nacheinander jeweils dreimal mit 40 ml DMF, Methanol und Dichlormethan gewaschen. Das Harz wurde anschließend im Vakuum getrocknet. Die Bestimmung der Beladung nach der Fmoc-Methode ergab eine Beladung von 0,6 mmol/g.

### Abspaltung der Allylgruppe am polymeren Träger (Schritt B)

Das Harz wurde unter Argon 5 min in DMF bei Raumtemperatur vorgequollen. Nach Zugabe von Tetrakis(triphenylphosphin)palladium und N-Methylpyrrolidin (10 Äquivalente) wurde das Gemisch unter Argon 6 h bei 40 °C geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### Kupplung mit Aminoverbindungen am polymeren Träger (Schritt C)

Das beladene Harz mit freier Carboxylfunktion wurde 5 min in DMF bei Raumtemperatur vorgequollen. Nach Zugabe einer Lösung von HOBt (1,2 Äquivalente), TOTU (1,2 Äquivalente) und Düsopropyl-ethylamin (1,2 Äquivalente) in DMF wurde das Gemisch 30 min bei Raumtemperatur geschüttelt. Die Aminoverbindung (1,2 Äquivalente) wurde gelöst in DMF zugegeben. Die Suspension wurde bei Raumtemperatur bis zur vollständigen Umsetzung geschüttelt (HPLC-Kontrolle). Nach beendeter Reaktion wurde die Lösung abgesaugt und das Harz nacheinander jeweils dreimal mit DMF, Methanol, Toluol und Dichlormethan gewaschen und anschließend getrocknet.

### Abspaltung der Fmoc-Schutzgruppe (Schritt D)

Zur Abspaltung der Fmoc-Schutzgruppe wurde das Harz 5 min in DMF bei Raumtemperatur vorgequollen. Nach Zugabe einer Lösung von DMF/Piperidin (1:1) wurde 20 min bei Raumtemperatur geschüttelt. Die Lösung wurde abgesaugt und der Vorgang wiederholt. Die Abspaltung einer analytischen Probe ergab vollständige Umsetzung nach HPLC/MS-Untersuchung. Nach vollständiger Umsetzung wurde das Harz dreimal mit Dichlormethan gewaschen und direkt in die Kupplung eingesetzt.

### Kupplung mit α-Halogencarbonsäuren (Schritt E)

### a) Kupplung mit DIC

Aus α-Halogencarbonsäuren (5 Äquivalente) wurden durch 30-minütige Reaktion mit Diisopropylcarbodiimid (2,4 Äquivalente) in Dichlormethan die symmetrischen Anhydride gebildet. Nach dieser Zeit wurden 2 Äquivalente Diisopropyl-ethylamin zugegeben. Das Gemisch wurde zu dem Harz gegeben und 12 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend sofort weiter umgesetzt.

### b) Kupplung mit Säurehalogeniden

Das Harz wurde 5 min mit Dichlormethan bei Raumtemperatur vorgequollen. Die α-Halogencarbonsäurehalogenide (1,5 Äquivalente) wurden gelöst in Dichlormethan zugegeben. Nach Zugabe einer katalytischen Menge 4-Dimethylaminopyridin und Diisopropyl-ethylamin (1 Äquivalent) wurde das Gemisch für 8 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend sofort weiter umgesetzt.

### Kupplung der α-Halogenacylverbindungen mit Hydantoinen (Schritt F)

Die 4,4-disubstituierten Hydantoine (2 Äquivalente) wurden in DMF mit Diazabicycloundecen (DBU) (2 Äquivalente) bei Raumtemperatur aktiviert. Die aktivierte Lösung wurde nach 15 min zu dem in DMF für 5 min vorgequollenen Harz gegeben. Das Gemisch wurde 8 h bei Raumtemperatur geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### N-Alkylierung des Hydantoins am polymeren Träger (Schritt G)

### a) Alkylierung mit Caesiumcarbonat

Das Harz wurde 5 min in DMF bei Raumtemperatur vorgequollen. Nach Zugabe von Caesiumcarbonat (3 Äquivalente) wurde 30 min bei Raumtemperatur geschüttelt. Nach Zugabe des Alkylierungsmittels (Bromid oder lodid) wurde 6 h bei 50 °C geschüttelt. Nach beendeter Reaktion wurde die Lösung abgesaugt und das Harz nacheinander jeweils dreimal mit DMF, Methanol/Wasser/DMF(1,5:1,5:7), DMF, Toluol und Dichlormethan gewaschen und anschließend getrocknet.

### b) Alkylierung mit Phosphazenen

Das Harz wurde 5 min in DMF bei Raumtemperatur vorgequollen. Nach Zugabe von N"'-tert-Butyl-N,N,N',N',N",N"-hexamethylphosphorimidsäuretriamid (Phosphazen-Base P1-t-Bu) (3 Äquivalente) wurde 30 min bei Raumtemperatur geschüttelt. Nach Zugabe des Alkylierungsmittels (Bromid oder Iodid) wurde 4 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend getrocknet.

### Abspaltung vom Harz (Schritt H)

Zur Abspaltung der Verbindung vom Harz wurde ein Gemisch von Trifluoressigsäure/Dichlormethan (1:1) zum Harz zugegeben. Die Suspension wurde 1 h geschüttelt. Das Harz wurde abfiltriert. Die verbleibende Lösung wurde im Vakuum eingeengt. Der Rückstand wurde durch Kieselgelchromatographie gereinigt (Dichlormethan und Essigester).

Nach der im Beispiel 11 beschriebenen allgemeinen Methode wurden die Verbindungen der Beispiele 12 bis 126 hergestellt, die die in der Formel Ib angegebene Struktur aufweisen. Die Bedeutungen der Reste in den einzelnen Verbindungen sind in den Tabellen 1 und 2 angegeben.

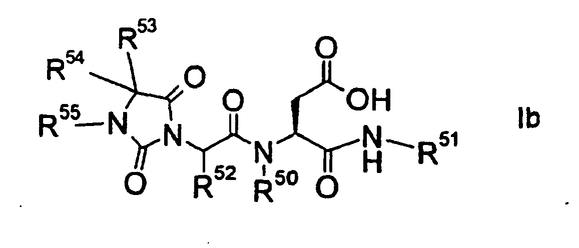

In den Tabellen 1 und 2 bedeuten:

| | | | |
|---|---|---|---|
| Bn = | Benzyl | 3-BrBn = | 3-Brombenzyl |
| 4-BrBn = | 4-Brombenzyl | 4-CIBn = | 4-Chlorbenzyl |
| 4-Bip = | 4-Biphenylylmethyl | 2-Py = | 2-Pyridylmethyl |
| 3-Py = | 3-Pyridylmethyl | 4-Py = | 4-Pyridylmethyl |
| H = | Wasserstoff | Me = | Methyl |
| Et = | Ethyl | nPr = | n-Propyl |
| iPr = | Isopropyl | nBu = | n-Butyl |
| iBu = | Isobutyl | nPe = | n-Pentyl |
| nHe = | n-Hexyl | All = | Allyl |
| Ph = | Phenyl | | |

Die folgenden Abkürzungen stehen für Reste , die für die Gruppe -NH-R⁵¹ in der Formel Ib stehen. Es sind Reste von Aminosäuren oder Derivaten davon, die formal durch Abstraktion eines Wasserstoffatoms von der Aminogruppe der Aminosäure erhalten werden.

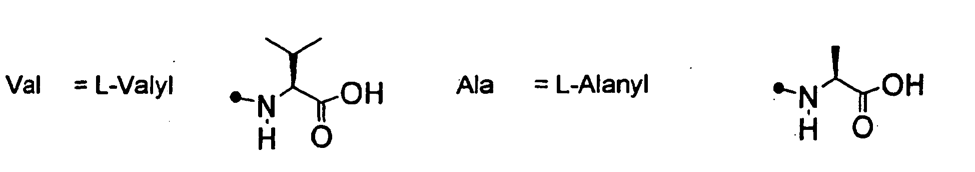

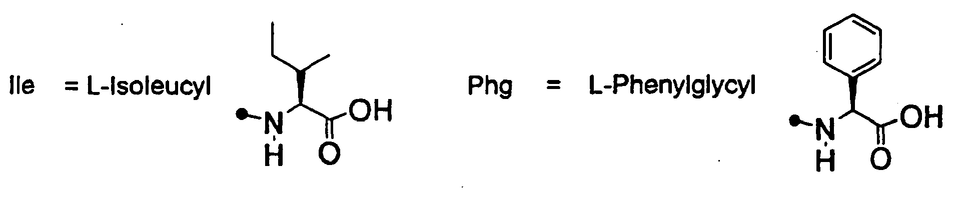

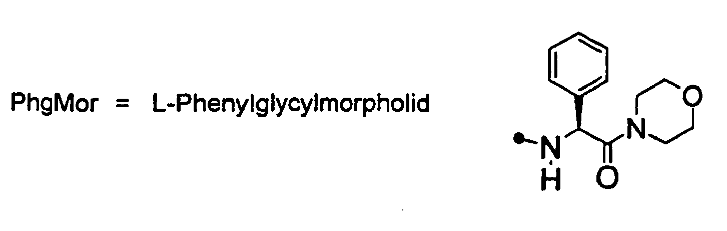

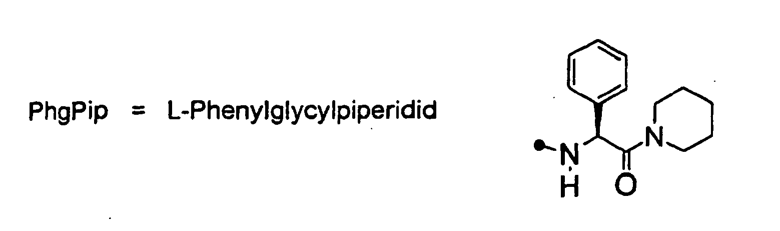

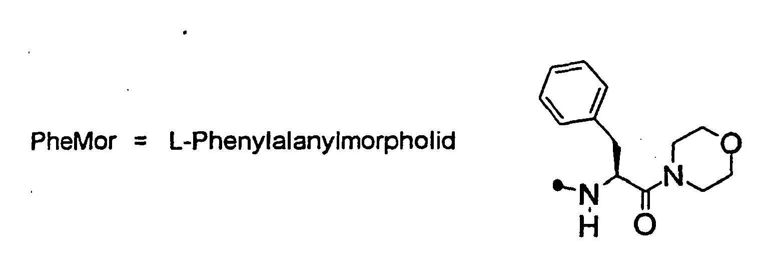

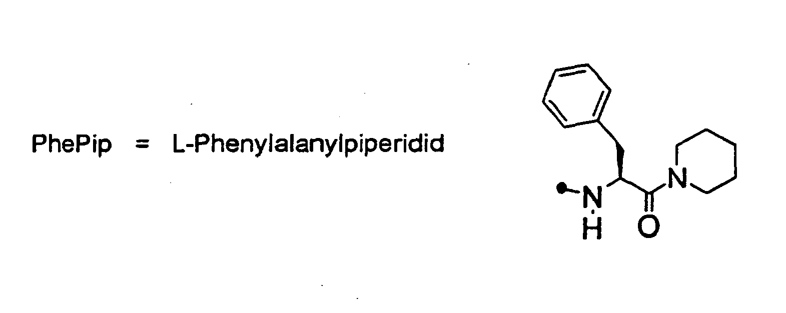

**Tabelle 1**

| Beispiel | R⁵⁰ | -NH-R⁵¹ | R⁵² | R⁵³ | R⁵⁴ | R⁵⁵ | ES(+)-MS |
|---|---|---|---|---|---|---|---|
| 12 | Me | Val | Bn | Me | Ph | Bn | 659 |
| 13 | Me | Val | iPr | Me | Ph | 4-Bip | 686 |
| 14 | Me | Val | H | Me | Ph | Bn | 568 |
| 15 | H | Phg | H | Me | Ph | 2-Py | 589 |
| 16 | H | Phg | H | Me | Ph | 3-Py | 589 |
| 17 | H | Phg | H | Me | Ph | 4-Py | 589 |
| 18 | H | Phg | Et | Me | Ph | Bn | 617 |
| 19 | H | Phg | H | Ph | Ph | Bn | 651 |
| 20 | H | Phg | nBu | Me | Ph | Bn | 644 |
| 21 | H | Phg | iBu | Me | Ph | Bn | 644 |
| 22 | H | Phg | nBu | Me | Ph | 2-Py | 645 |
| 23 | H | Phg | nBu | Me | Ph | 3-Py | 645 |
| 24 | H | Phg | nBu | Me | Ph | 4-Py | 645 |
| 25 | H | Phg | iBu | Me | Ph | 2-Py | 645 |
| 26 | H | Phg | iBu | Me | Ph | 3-Py | 645 |
| 27 | H | Phg | iBu | Me | Ph | 4-Py | 645 |
| 28 | H | Ile | H | Me | Ph | 4-BrBn | 647 |
| 29 | H | Ile | Bn | Me | Ph | Bn | 659 |
| 30 | H | Ile | iPr | Me | Ph | Bn | 610 |
| 31 | H | Ile | iPr | Me | Ph | 4-Bip | 686 |
| 32 | H | Ile | H | Me | Ph | Bn | 568 |
| 33 | H | Ile | nPe | Me | Ph | Bn | 639 |
| 34 | H | Ile | nPe | Me | Ph | 4-Bip | 715 |
| 35 | H | Ala | Bn | Me | Ph | Bn | 616 |
| 36 | H | Ala | iPr | Me | Ph | Bn | 568 |
| 37 | H | Ala | iPr | Me | Ph | 4-Bip | 644 |
| 38 | H | Ala | H | Me | Ph | Bn | 525 |
| 39 | H | Ala | nPe | Me | Ph | Bn | 596 |
| 40 | H | Ala | nPe | Me | Ph | 4-Bip | 672 |
| 41 | H | Phg | Bn | Me | Ph | Bn | 679 |
| 42 | H | Phg | iPr | Me | Ph | Bn | 630 |
| 43 | H | Phg | iPr | Me | Ph | 4-Bip | 707 |
| 44 H | | Phg | H | Me | Ph | Bn | 588 |
| 45 | H | Phg | nPe | Me | Ph | Bn | 658 |
| 46 | H | Phg | nPe | Me | Ph | 4-Bip | 735 |
| 47 | H | Phg | Et | Me | Ph | 2-Py | 618 |
| 48 | H | Phg | Et | Me | Ph | 3-Py | 618 |
| 49 | H | Phg | Et | Me | Ph | 4-Py | 618 |
| 50 | H | Phg | H | Ph | Ph | 2-Py | 651 |
| 51 | H | Phg | H | Ph | Ph | 3-Py | 651 |
| 52 | H | Phg | H | Ph | Ph | 4-Py | 651 |
| 53 | Me | Val | nPe | Me | Ph | Bn | 638 |
| 54 | Me | Val | nPe | Me | Ph | 4-Bip | 715 |
| 55 | H | Val | H | Me | Ph | Bn | 554 |
| 56 | H | Val | Bn | Me | Ph | Bn | 644 |
| 57 | H | Val | iPr | Me | Ph | 4-Bip | 672 |
| 58 | H | Val | iPr | Me | Ph | Bn | 596 |
| 59 | H | Val | nPe | Me | Ph | Bn | 624 |
| 60 | H | Val | nPe | Me | Ph | 4-Bip | 701 |
| 61 | H | PheMor | H | Me | Ph | Bn | 671 |
| 62 | H | PheMor | Bn | Me | Ph | Bn | 762 |
| 63 | H | PheMor | iPr | Me | Ph | 4-Bip | 790 |
| 64 | H | PheMor | iPr | Me | Ph | Bn | 714 |
| 65 | H | PheMor | nPe | Me | Ph | Bn | 742 |
| 66 | H | PheMor | nPe | Me | Ph | 4-Bip | 818 |
| 67 | H | PhePip | H | Me | Ph | Bn | 670 |
| 68 | H | PhePip | Bn | Me | Ph | Bn | 760 |
| 69 | H | PhePip | iPr | Me | Ph | 4-Bip | 788 |
| 70 | H | PhePip | nBu | Me | Ph | Bn | 712 |
| 71 | H | PhePip | nPe | Me | Ph | Bn | 726 |
| 72 | H | PhePip | nBu | Me | Ph | 4-Bip | 802 |
| 73 | H | PhgMor | H | Me | Ph | Bn | 658 |
| 74 | H | PhgMor | Bn | Me | Ph | Bn | 748 |
| 75 | H | PhgMor | iPr | Me | Ph | Bn | 700 |
| 76 | H | PhgMor | nPe | Me | Ph | Bn | 728 |
| 77 | H | PhgMor | nPe | Me | Ph | 4-Bip | 804 |
| 78 | H | PhgPip | H | Me | Ph | Bn | 656 |
| 79 | H | PhgPip | Bn | Me | Ph | Bn | 746 |
| 80 | H | PhgPip | iPr | Me | Ph | 4-Bip | 774 |
| 81 | H | PhgPip | iPr | Me | Ph | Bn | 698 |
| 82 | H | PhgPip | nPe | Me | Ph | Bn | 726 |
| 83 | H | PhgPip | nPe | Me | Ph | 4-Bip | 802 |
| 84 | H | Phg | 4-CIBn | Me | Ph | Bn | 713 |
| 85 | H | Phg | All | Me | Ph | Bn | 629 |
| 86 | H | Phg | H | Me | Ph | 4-BrBn | 667 |
| 87 | H | Phg | H | Me | Ph | 3-BrBn | 667 |
| 88 | H | Ph(CH₂)₃NH- | nBu | Me | Ph | Bn | 628 |
| 89 | H | Phg | nBu | Me | Ph | nPr | 595 |
| 90 | H | Phg | nBu | Me | Ph | iBu | 610 |
| 91 | H | Phg | nBu | Me | Ph | nHe | 638 |
| 92 | H | Phg | nPr | Me | Ph | Bn | 630 |
| 93 | H | Phg | nHe | Me | Ph | Bn | 672 |
| 94 | H | Phg | H | Me | Ph | nPr | 539 |
| 95 | H | PheMor | H | Me | Ph | nPr | 622 |
| 96 | H | PheMor | iBu | Me | Ph | Bn | 727 |
| 97 | H | Phg | H | Me | Ph | Et | 525 |
| 98 | H | Phg | H | Me | Ph | iBu | 553 |
| 99 | H | Phg | H | Me | Ph | iPr | 539 |
| 100 | H | Phg | nBu | Me | Ph | Bn | 644 |
| 101 | H | CH₃(CH₂)₇NH- | nBu | Me | Ph | Bn | 621 |
| 102 | H | Phg | Et | Me | Ph | iPr | 567 |
| 103 | H | Phg | nPr | Me | Ph | Bn | 630 |
| 104 | H | Phg | nPr | Me | Ph | iBu | 595 |
| 105 | H | Phg | nPr | Me | Ph | iPr | 581 |

**Tabelle 2**

| In allen Verbindungen der Tabelle 2 steht in der Formel Ib der Rest R⁵⁰ für Wasserstoff, der Rest -NH-R⁵¹ für Phg (= L-Phenylglycyl) und der Rest R⁵² für n-Butyl. | | | | |
|---|---|---|---|---|
| Beispiel | R⁵³ | R⁵⁴ | R⁵⁵ | ES(+)-MS |
| 106 | Me | 2-Fluorphenyl | Bn | 661 |
| 107 | Me | 3-Fluorphenyl | Bn | 661 |
| 108 | Me | 4-Fluorphenyl | Bn | |
| 109 | Me | 4-Fluorbenzyl | Bn | |
| 110 | Me | 3-Trifluormethylphenyl | Bn | |
| 111 | Me | 3-Chlorphenyl | Bn | |
| 112 | Bn | Bn | Bn | |
| 113 | Me | 4-Methoxybenzyl | Bn | |
| 114 | Me | Cyclohexyl | Bn | |
| 115 | Me | Bn | Bn | |
| 116 | Me | 2-Thienyl | Bn | |
| 117 | Me | 3-Trifluormethylbenzyl | Bn | |
| 118 | Cyclopropyl | Ph | Bn | |
| 119 | Cyclobutyl | Ph | Bn | |
| 120 | Me | 3,4,5-Trimethoxyphenyl | Bn | |
| 121 | Me | 4-Fluorphenyl | H | |
| 122 | Bn | Bn | H | |
| 123 | Me | 4-Methoxybenzyl | H | |
| 124 | Me | 3-Trifluormethylbenzyl | H | |
| 125 | Cyclobutyl | Ph | H | |
| 126 | Me | 3,4,5-Trimethoxybenzyl | H | |

### Beispiel 127

### (2-((R,S)-4-Phenyl-3-benzyl-4-methyl-2,5-dioxo-imidazolidin-1-yl)-2,2-dimethyl-acetyl)-L-aspartyl-L-phenylglycin

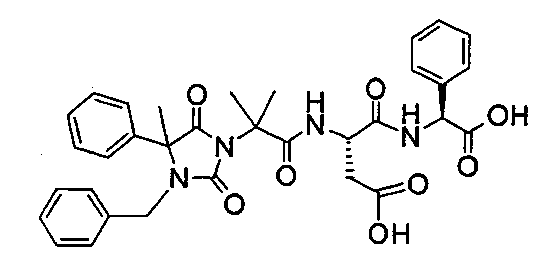

Die Verbindung wurde analog der im Beispiel 11 beschriebenen allgemeinen Vorschrift durch Festphasensynthese hergestellt.
ES(+)-MS: 616

Die 2,3-Diaminopropionsäure-Derivate der Beispiele 129 bis 168 wurden durch Festphasensynthese nach der in Beispiel 128 angegebenen allgemeinen Vorschrift hergestellt.

### Beispiel 128

### Allgemeine Vorschrift zur Herstellung von Diaminopropionsäure-Derivaten durch Festphasensynthese

### Allgemeines

Die Synthesen am polymeren Träger wurden nach der Synthesesequenz durchgeführt, die im Schema 2 dargestellt ist. Die obigen allgemeinen Erläuterungen zur Herstellung von Aspartyl-Phenylglycin-Derivaten durch Festphasensynthese gelten hier entsprechend.

### Kupplung der α-Fmoc-β-Alloc-2,3-Diaminopropionsäure an den polymeren Träger (Schritt J in Schema 2)

Man gab zu 1 g Wang-Polystyrolharz eine Lösung von 0,243 g (1,8 mmol) HOBt, 0,590 g (1,8 mmol) TOTU, 0,25 ml (1,8 mmol) Düsopropyl-ethylamin und 0,738 g (1,8 mmol) (S)-α-Fmoc-β-Alloc-2,3-Diaminopropionsäure in 5 ml DMF und ließ das Gemisch 12 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen. Die Bestimmung der Beladung nach der Fmoc-Methode ergab eine Beladung von 0,9 mmol/g.

### Abspaltung der Allyloxycarbonylgruppe am polymeren Träger (Schritt K)

Das Harz wurde unter Argon 5 min in DMF bei Raumtemperatur vorgequollen. Nach Zugabe von Tetrakis(triphenylphosphin)palladium und N-Methylpyrrolidin (10 Äquivalente) wurde das Gemisch unter Argon 6 h bei 40 °C geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

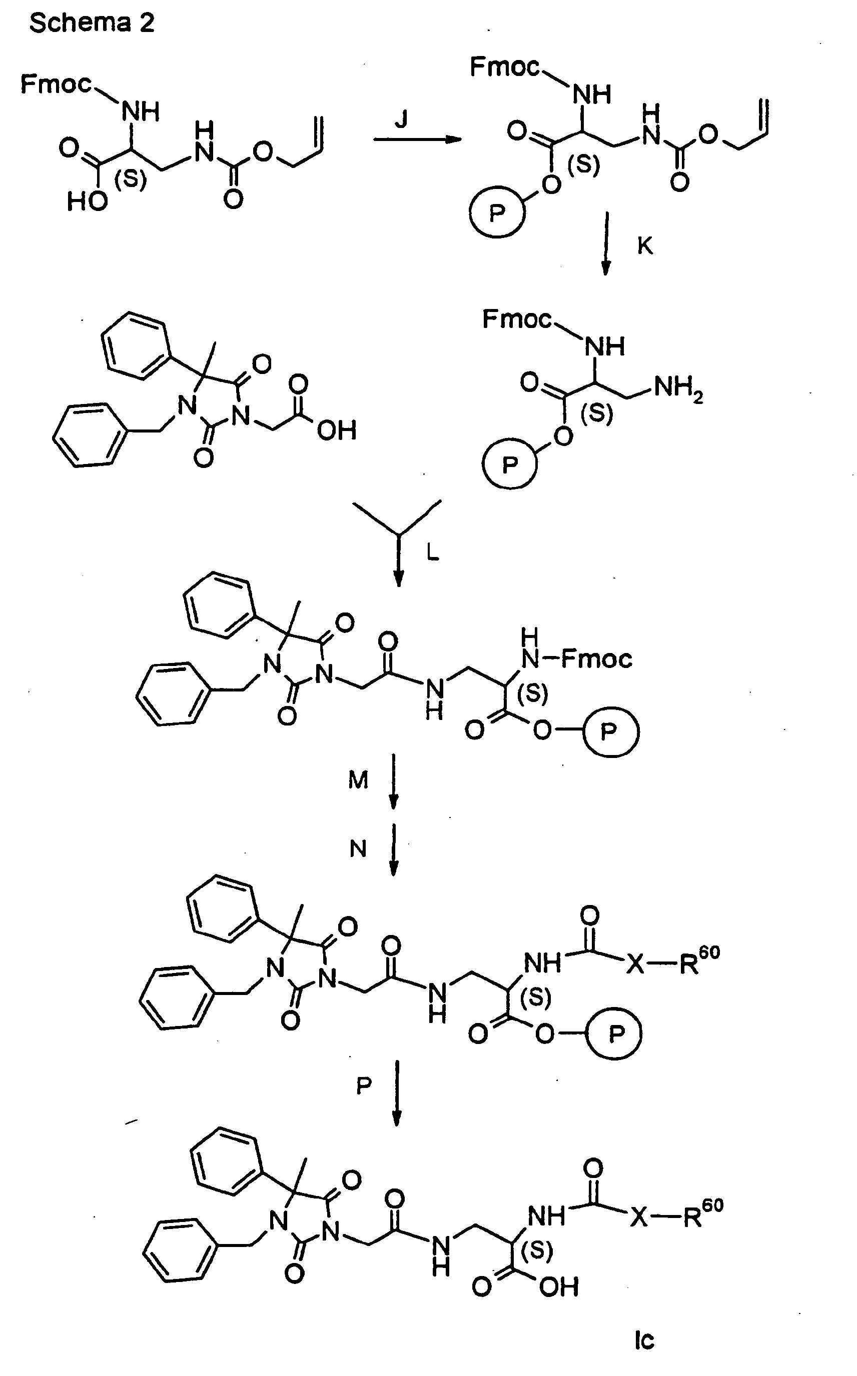

### Kupplung der α-Fmoc-2,3-Diaminopropionsäure mit Hydantoincarbonsäuren (Schritt L)

Man gab zu 100 mg Harz, das mit der α-Fmoc-2,3-Diaminopropionsäure (0,9 mmollg) beladen war, eine Lösung von 36 mg (0,27 mmol) HOBt, 88 mg (0,27 mmol) TOTU, 37 µl (0,27 mmol) Diisopropyl-ethylamin und 0,27 mmol (R,S)-3-Benzyl-4-phenyl-4-methyl-2,5-dioxo-imidazolidin-1-yl-essigsäure in 5 ml DMF und ließ das Gemisch 12 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen.

### Abspaltung der Fmoc-Schutzgruppe (Schritt M)

Zur Abspaltung der Fmoc-Schutzgruppe wurde das Harz 5 min in DMF bei Raumtemperatur vorgequollen. Nach Zugabe einer Lösung von DMF/Piperidin (1:1) wurde 20 min bei Raumtemperatur geschüttelt. Die Lösung wurde abgesaugt und der Vorgang wiederholt. Die Abspaltung einer analytischen Probe ergab vollständige Umsetzung nach HPLC/MS Untersuchung. Nach der vollständigen Umsetzung wurde das Harz dreimal mit Dichlormethan gewaschen und direkt in den nächsten Schritt eingesetzt.

### Acylierung der α-Aminogruppe der 2,3-Diaminopropionsäure (Schritt N)

### a) Herstellung von Carbonsäureamiden (Acylierung mit Carbonsäuren)

Man gab zu 100 mg Harz, das mit dem 2,3-Diaminopropionsäurebaustein beladen war, eine Lösung von 36 mg (0,27 mmol) HOBt, 88 mg (0,27 mmol) TOTU, 37 µl (0,27 mmol) Diisopropyl-ethylamin und 0,27 mmol der entsprechenden Carbonsäure der Formel R⁶⁰-COOH in 5 ml DMF und ließ das Gemisch 12 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen.

### b) Herstellung von Harnstoffen (Acylierung mit Isocyanaten)

Man gab zu 100 mg Harz, das mit dem 2,3-Diaminopropionsäurebaustein beladen war, eine Lösung von 0,27 mmol des entsprechenden Isocyanats der Formel R⁶⁰-N=C=O und einer katalytischen Menge (1 mg) 4-Dimethylaminopyridin in 5 ml DMF und ließ das Gemisch 8 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen.

### c) Herstellung von Carbamaten (Acylierung mit Kohlensäurederivaten)

Der entsprechende Alkohol (0,27 mmol) der Formel R⁶⁰-OH wurde mit jeweils äquivalenten Mengen Di-(N-succinimidyl)-carbonat und Diisopropyl-ethylamin 5 h bei 40 °C geschüttelt. Die Lösung wurde zu 100 mg Harz, das mit dem 2,3-Diaminopropionsäurebaustein beladen war, gegeben und das Gemisch 8 h bei Raumtemperatur geschüttelt. Das Harz wurde abfiltriert und 3 mal mit je 10 ml DMF, einmal mit 10 ml Toluol, einmal mit 10 ml Methanol und 3 mal mit 10 ml Dichlormethan gewaschen.

### Abspaltung vom Harz (Schritt P)

Zur Abspaltung der Verbindung vom Harz wurde ein Gemisch von Trifluoressigsäure und Dichlormethan (1:1) zum Harz zugegeben. Die Suspension wurde 1 h geschüttelt und dann das Harz abfiltriert. Die verbleibende Lösung wurde im Vakuum eingeengt. Der Rückstand wurde durch Chromatographie an Kieselgel gereinigt (Dichlormethan und Essigester).

Die im Schritt L eingesetzte 3-Benzyl-4-phenyl-4-methyl-2,5-dioxo-imidazolidin-1-yl-essigsäure wurde nach der folgenden allgemeinen Arbeitsvorschrift zur Herstellung von 4,4-disubstituierten Hydantoincarbonsäuren erhalten.

Zu 3,0 mmol Acetophenon und 3,0 g Ammoniumcarbonat in 3,8 ml Ethanol wurde eine Lösung von 288 mg Kaliumcyanid in 3,8 ml Wasser pipettiert. Das Gemisch wurde 5 h bei 55 °C gerührt. Anschließend wurden 8 ml 6 N Salzsäure langsam zudosiert und das Gemisch weitere 2 h bei 55 °C gerührt. Nach Zugabe von 6,0 ml Wasser wurde das Gemisch über 2 h auf Raumtemperatur abgekühlt. Das Produkt wurde abgesaugt, mit Wasser gewaschen und an der Luft getrocknet.

Das (R,S)-4-Methyl-4-phenyl-hydantoin wurde mit einem Äquivalent Caesiumcarbonat in DMF (20 ml/g Hydantoinderivat) suspendiert und für 20 min bei Raumtemperatur gerührt. Nach Zugabe von einem Äquivalent Bromessigsäure-tert-butylester wurde das Gemisch für 1 h bei Raumtemperatur gerührt. Anschließend wurde mit Wasser versetzt und mit Essigester extrahiert. Die vereinigten organischen Phasen wurden über Magnesiumsulfat getrocknet, filtriert und eingeengt. Man erhielt den Hydantoinessigsäureester als Öl.

Der Hydantoinessigsäureester wurde mit einem Äquivalent Caesiumcarbonat und einem Äquivalent Benzylbromid in DMF (20 ml/g Hydantoinderivat) suspendiert. Das Gemisch wurde für 1 h bei Raumtemperatur gerührt. Anschließend wurde mit Wasser versetzt und mit Essigester extrahiert. Die vereinigten organischen Phasen wurden über Magnesiumsulfat getrocknet, filtriert und eingeengt. Der Rückstand wurde durch Chromatographie an Kieselgel gereinigt (Hexan/Essigester). Man erhielt den 3-Benzyl-hydantoinessigsäureester als Öl. Die tert-Butylestergruppe wurde anschließend unter Standardbedingungen mit Trifluoressigsäure zur Carbonsäure gespalten.

Nach der im Beispiel 128 beschriebenen allgemeinen Vorschrift wurden die Verbindungen der Beispiele 129 bis 168 hergestellt, die die in der Formel Ic angegebene Struktur aufweisen. Die Bedeutung der Gruppen X und R⁶⁰ in den einzelnen Verbindungen der Formel Ic sind in der Tabelle 3 angegeben. Steht X für eine direkte Bindung, so heißt das, daß die Gruppe R⁶⁰ direkt an die Carbonylgruppe gebunden ist und somit eine Gruppe R⁶⁰CO vorliegt.

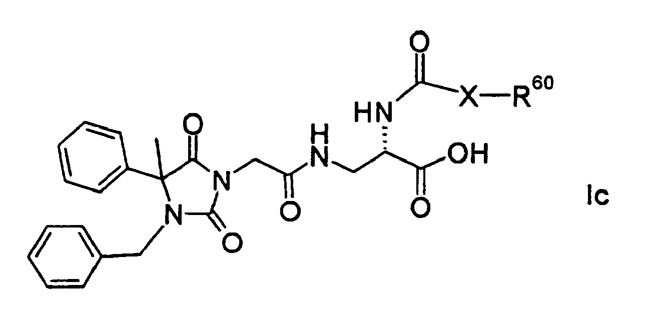

**Tabelle 3**

| Beispiel | -X- | R⁶⁰ | ES-(+)-MS |
|---|---|---|---|
| 129 | direkte Bindung | 3-Methylphenyl | 543 |
| 130 | direkte Bindung | 2-Methylphenyl | 543 |
| 131 | direkte Bindung | 2,4-Dimethoxyphenyl | 589 |
| 132 | direkte Bindung | 3,5-Dinitrophenyl | 619 |
| 133 | direkte Bindung | 4-tert-Butylphenyl | 585 |
| 134 | direkte Bindung | 2,4,5-Trimethylphenyl | 571 |
| 135 | -NH- | 4-Chlorphenyl | 579 |
| 136 | -NH- | 4-Isopropylphenyl | 586 |
| 137 | -NH- | 2-Nitrophenyl | 589 |
| 138 | direkte Bindung | 4-Chlorphenyl | 564 |
| 139 | direkte Bindung | 4-Methylphenyl | 543 |
| 140 | direkte Bindung | 4-Methoxyphenyl | 559 |
| 141 | direkte Bindung | 4-Nitrophenyl | 574 |
| 142 | -NH- | 4-(Trifluormethoxy)phenyl | 628 |
| 143 | -NH- | 2-Methoxyphenyl | 574 |
| 144 | -NH- | 3,5-Bis(trifluormethyl)phenyl | 680 |
| 145 | -NH- | Benzyl | 558 |
| 146 | -O- | 2-Methoxyethyl | 527 |
| 147 | -O- | Prop-2-inyl | 507 |
| 148 | -O- | 2,2,2-Trifluorethyl | 551 |
| 149 | -O- | Cyclopentyl | 537 |
| 150 | -O- | 2-Cyclohexylethyl | 580 |
| 151 | -O- | Prop-2-enyl | 510 |
| 152 | -O- | 2-(4-Fluorphenyl)ethyl | 591 |
| 153 | -O- | 2-(4-Nitrophenyl)ethyl | 618 |
| 154 | -O- | 2-(3-Methoxyphenyl)ethyl | 604 |
| 155 | -O- | Cyclopropylmethy) | 523 |
| 156 | -O- | Isobutyl | 525 |
| 157 | -O- | 2,2-Dimethylpropyl | 539 |
| 158 | -O- | Cyclobutylmethyl | 537 |
| 159 | -O- | 2-Ethylbutyl | 553 |
| 160 | -O- | Cyclopentylmethyl | 551 |
| 161 | -O- | 2-(4-Methylphenyl)ethyl | 589 |
| 162 | -O- | 4-Benzylbenzyl | 650 |
| 163 | -O- | 4-Nitrobenzyl | 604 |
| 164 | -O- | 2-Phenylethyl | 573 |
| 165 | -O- | 2-(4-Methoxyphenyl)ethyl | 604 |
| 166 | -O- | 2-(1-Naphthyl)ethyl | 624 |
| 167 | -O- | 2-(2-Naphthyl)ethyl | 624 |
| 168 | -O- | 2-(4-tert-Butylphenyl)ethyl | 630 |

### Beispiel 169

### (S)-3-((S)-2-((S)-4-Phenyl-3-benzyl-4-methyl-2,5-dioxo-imidazolidin-1-yl)-2-(2-methyl-propyl)-acetylamino)-3-(3,4-methylendioxy-phenyl)-propionsäure

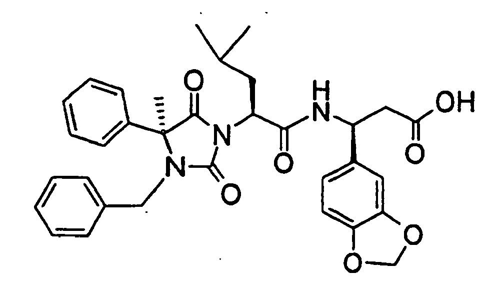

### 169a) (S)-2-Amino-2-(4-Brom-phenyl)-propionsäuremethylester (169.1)

15 g (55,7 mmol) (S)-4-(4-Bromphenyl)-4 methyl-2,5-dioxo-imidazolidin wurden in 107 ml 3 N Natronlauge suspendiert und die Suspension 2 h bei 145°C im Autoklaven erhitzt. Man ließ auf Raumtemperatur abkühlen, filtrierte den Niederschlag ab, löste ihn in Wasser und stellte mit 1 N Salzsäure pH 1 ein. Nach Gefriertrocknen wurde der Feststoff in 150 ml absolutem Methanol suspendiert. Die Suspension wurde auf -15°C abgekühlt und mit 8,8 ml Thionylchlorid versetzt. Nach 6 h Rühren bei Raumtemperatur und Stehenlassen über Nacht wurden weitere 100 ml absolutes Methanol und 8,8 ml Thionylchlorid zugegeben. Das Gemisch wurde 8 h bei Raumtemperatur gerührt und erneut über Nacht stehen lassen. Nach Entfernen flüchtiger Anteile im Vakuum wurde der Rückstand mit Natriumhydrogencarbonat-Lösung und Natriumcarbonat-Lösung auf pH 9,3 gestellt und anschließend die wäßrige Phase 2 x mit Essigester extrahiert. Nach Trocknen über Natriumsulfat, Filtration und Entfernen des Lösungsmittels im Vakuum erhielt man 11,4 g (79 %) 169.1.

### 169b) (S)-2-((S)-4-(4-Brom-phenyl)-4-methyl-2,5-dioxo-imidazolidin-1-yl)-2-(2-methyl-propyl)-essigsäure-tert-butylester (169.2)

Man gab zu einer Lösung von 5,8 g (22,5 mmol) 169.1 in 50 ml DMF 4,8 g L-Leucin-tert-butylester-isocyanat (hergestellt aus L-Leucin-tert-butylester analog J. S. Nowick et al., J. Org. Chem. 1996, 61, 3929). Nach 4 h Rühren bei Raumtemperatur wurde das Lösungsmittel entfernt und der Rückstand mit Heptan/tert-Butyl-methylether = 6/4 über Kieselgel chromatographiert. Die das Zwischenprodukt enthaltenden Fraktionen wurden vereinigt, das Lösungsmittel wurde im Vakuum entfernt, der Rückstand erneut in 90 ml absolutem DMF gelöst und die Lösung bei 0 °C mit 775 mg einer 55 - 65 %igen Natriumhydrid-Dispersion in Öl versetzt. Nach 3 h Rühren bei Raumtemperatur wurde das Lösungsmittel im Vakuum entfernt und der Rückstand mit Heptan/tert-Butyl-methylether =1/1 über Kieselgel chromatographiert. Nach Einengen der Produktfraktionen erhielt man 7,8 g (79 %) an 169.2 als farblosen Feststoff.

### 169c) (S)-2-((S)-4-(4-Brom-phenyl)-3-benzyl-4-methyl-2,5-dioxo-imidazolidin-1-yl)-2-(2-methyl-propyl)-essigsäure-tert-butylester (169.3)

Man gab zu einer Lösung von 1,75 g (4 mmol) 169.2 in 20 ml absolutem DMF 540 µl (4,4 mmol) Benzylbromid und anschließend bei 0 °C 140 mg einer 55 - 65 %igen Natriumhydrid-Dispersion in Öl und ließ 15 min bei 0 °C und 3 h bei Raumtemperatur rühren. Nach Stehenlassen über Nacht wurde das Lösungsmittel im Vakuum entfernt und der Rückstand mit Heptan/Essigester = 8/2 über Kieselgel chromatographiert. Die Produktfraktionen wurden vereinigt und das Lösungsmittel im Vakuum entfernt. Man erhielt 1,97 g (93 %) 169.3.

### 169d) (S)-2-((S)-4-Phenyl-3-benzyl-4-methyl-2,5-dioxo-imidazolidin-1-yl)-2-(2-methyl-propyl)-essigsäure-tert-butylester (169.4)

1,9 g (3,59 mmol) 169.3 in 190 ml Ethanol wurden 2 h über 76 mg 10 % Palladium/Kohlenstoff hydriert. Der Katalysator wurde abfiltriert, das Lösungsmittel im Vakuum entfernt, der Rückstand in Essigester gelöst und die Lösung mit einer 10 %igen Natriumhydrogencarbonat-Lösung gewaschen. Die Phasen wurden getrennt und die organische Phase über Natriumsulfat getrocknet. Nach Filtration erhielt man 1,3g(80%) 169.4.

### 169e) (S)-2-((S)-4-Phenyl-3-benzyl-4-methyl-2,5-dioxo-imidazolidin-1-yl)-2-(2-methyl-propyl)-essigsäure (169.5)

1,3 g (2,89 mmol) 169.4 in einem Gemisch aus 10 ml 6 N Salzsäure und 2 ml Tetrahydrofuran wurden 4 h unter Rückfluß erhitzt. Nach Entfernen des Lösungsmittels im Vakuum und Chromatographie des Rückstandes mit Heptan/Essigester = 3/2 erhielt man 510 mg (45 %) 169.5.

### 169f) (S)-3-((S)-2-((S)-4-Phenyl-3-benzyl-4-methyl-2,5-dioxo-imidazolidin-1-yl)-2-(2-methyl-propyl)-acetylamino)-3-(3,4-methylendioxy-phenyl)-propionsäure

Die Verbindung wurde hergestellt analog Beispiel 1 durch Umsetzung von 169.5 mit (S)-3-Amino-3-(3,4-methylendioxy-phenyl)-propionsäure-tert-butylester (hergestellt analog S. G. Davis et al., Tetrahedron Asymmetry 1991, 2, 183), Spaltung des tert-Butylesters mit Trifluoressigsäure wie in Beispiel 1 beschrieben, und anschließende Reinigung des Rohproduktes mittels präparativer HPLC (RP18, Eluens: Acetonitril/Wasser = 501120).
ES(+)-MS: 586.4 (M+H)⁺

Analog Beispiel 169 können auch die folgenden beiden Verbindungen hergestellt werden:

### (S)-3-((S)-2-((S)-4-Phenyl-3-benzyl-4-methyl-2,5-dioxo-imidazolidin-1-yl)-2-(2-methyl-propyl)-acetylamino)-3-(2,4-dimethoxy-phenyl)-propionsäure

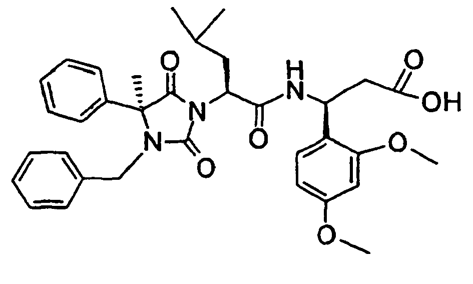

(durch Umsetzung von 169.5 mit (S)-3-Amino-3-(2,4-dimethoxy-phenyl)-propionsäure-tert-butylester und anschließende Spaltung des tert-Butylesters mit Trifluoressigsäure)

### (S)-3-((S)-2-((S)-4-Phenyl-3-((4-biphenylyl)-methyl)-4-methyl-2,5-dioxo-imidazolidin-1-yl)-2-(2-methyl-propyl)-acetylamino)-3-(3,4-methylendioxy-phenyl)-propionsäure

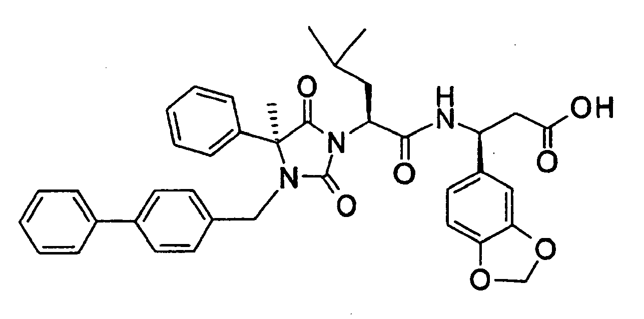

(durch Umsetzung von (S)-2-((S)-4-Phenyl-3-((4-biphenylyl)-methyl)-4-methyl-2,5-dioxo-imidazotidin-1-yl)-2-(2-methyl-propyl)-essigsäure (erhältlich durch Umsetzung von 169.2 mit 4-Phenyl-benzylbromid analog der Synthese von 169.3 und anschließende Folgereaktionen analog der Herstellung von 169.5) mit (S)-3-Amino-3-(3,4-methylendioxy-phenyl)-propionsäure-tert-butylester und anschließende Spaltung des tert-Butylesters mit Trifluoressigsäure)

### Beispiel 170

### (S)-3-((R,S)-2-((R,S)-4-(4-Pyridyl)-3-benzyl-4-methyl-2,5-dioxo-imidazolidin-1-yl)-2-(2-methyl-propyl)-acetylamino)-2-(1-adamantylmethyloxycarbonylamino)-propionsäure

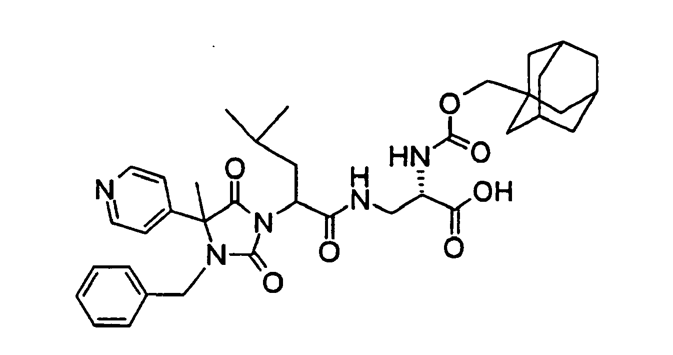

Die Verbindung wurde analog Beispiel 5 hergestellt durch Umsetzung von 6.2 (siehe Beispiel 6) mit (S)-2-(1-Adamantylmethyloxycarbonylamino)-3-amino-propionsäure-tert-butylester (Herstellung siehe Beispiel 4) und anschließende Spaltung des tert-Butylesters mit Trifluoressigsäure.
ES(+)-MS: 674.5 (M+H)⁺

### Beispiel 171

### Allgemeine Vorschrift zur Herstellung von 2-(N-((2,5-Dioxo-imidazolidin-1-yl)-acetyl)-N-alkyl-amino)-propionsäuren

### 171 a) Allgemeine Arbeitsvorschrift zur Herstellung von N-alkylierten β-Alanin-tert-butylestern

Das primäre Alkylamin (50 mmol) wurde in 80 ml Methanol gelöst (wenn das Alkylamin in Form des Hydrochlorids eingesetzt wurde, wurde zunächst durch die Zugabe von Kalium-tert-butylat (45 mmol) das Amin freigesetzt). 7.25 ml Acrylsäuretert-butylester (50 mmol) wurden zugegeben und nach Durchmischen wurde 2 Tage bei Raumtemperatur stehen gelassen. Dann wurde von eventuell vorhandenen Feststoffen abfiltriert, am Rotationsverdampfer bei 60 °C eingeengt und zweimal mit Toluol koevaporiert. Der Rückstand wurde in 100 ml absolutem Diethylether aufgenommen, filtriert und das Filtrat scharf eingeengt. Das so erhaltene Produkt fiel als Öl oder Feststoff an und wurde ohne weitere Reinigung im nächsten Reaktionsschritt eingesetzt.

### 171 b) Allgemeine Arbeitsvorschrift zur Acylierung von N-alkylierten β-Alanin-tert-butylestem mit Hydantoincarbonsäuren und Spaltung der β-Alanin-tert-butylester

Die Hydantoincarbonsäure (0.5 mmol) (siehe Beispiel 128), 114 mg N-Ethyl-N'-(3-dimethylaminopropyl)carbodümid-Hydrochlorid (0.6 mmol), 70 mg 1-Hydroxybenzotriazol (0.6 mmol) und der N-alkylierte β-Alanin-tert-butylester (1.0 mmol) wurden in 2 ml absolutem DMF gelöst und 8 h bei Raumtemperatur gerührt. Die Reaktionsmischung wurde in 100 ml Ethylacetat aufgenommen und je dreimal mit KHSO₄-Lösung (10%), KHCO₃-Lösung und Wasser gewaschen. Die Ethylacetatphase wurde mit MgSO₄ getrocknet und zur Trockene eingeengt. Der Rückstand wurde mit 3 ml Trifluoressigsäure versetzt und 1 h bei Raumtemperatur stehen gelassen. Die Trifluoressigsäure wurde im Vakuum entfernt und der Rückstand mit Toluol und Diethylether koevaporiert.

### Beispiel 172

### 2-(N-(((R,S)-4-Phenyl-3-benzyl-4-methyl-2,5-dioxo-imidazolidin-1-yl)-acetyl)-N-benzyl-amino)-propionsäure

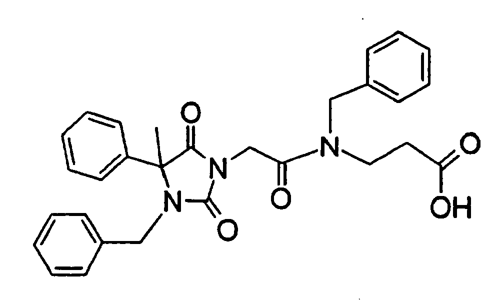

Die Verbindung wurde ausgehend von Benzylamin nach der Vorschrift in Beispiel 171 hergestellt. Ausbeute: 183 mg (73 %) farbloses Pulver.

### Beispiel 173

### 2-(N-(((R,S)-4-Phenyl-3-benzyl-4-methyl-2,5-dioxo-imidazolidin-1-yl)-acetyl)-N-octyl-amino)-propionsäure

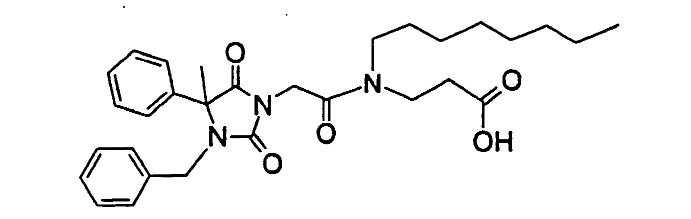

Die Verbindung wurde ausgehend von n-Octylamin nach der Vorschrift in Beispiel 171 hergestellt. Ausbeute: 293 mg (99 %) farbloses Öl.

### Untersuchung der biologischen Aktivität

Als Testmethode für die Wirksamkeit der Verbindungen der Formel I auf die Interaktion zwischen VCAM-1 und VLA-4 wird ein Assay verwendet, der für diese Interaktion spezifisch ist. Die zellulären Bindungspartner, das heißt die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode

### Assay zur Messung der Adhäsion von U937-Zellen (ATCC CRL 1593) an hVCAM-1 (1-3)-IgG

### 1. Herstellung von humanem VCAM-1(1-3)-IgG und humanem CD4-IgG

Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen) (von Dr. Brian Seed, Massachusetts General Hospital, Boston, USA; vgl. Damle und Aruffo, Proc. Natl. Acad. Sci. USA 1991, 88, 6403-6407). Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextranvermittelter DNA-Transfektion in COS-Zellen (ATCC CRL1651) gemäß Standardprozeduren exprimiert (Ausubel et al., Current Protocols in Molecular Biology, John Wiley & Sons, Inc., 1994).

### 2. Assay zur Messung der Adhäsion von U937-Zellen an hVCAM-1 (1-3)-IgG

2.1 96 well-Mikrotitertestplatten (Nunc Maxisorb) wurden mit 100 µl/well einer Ziege-anti-human-IgG-Antikörperlösung (10 µg/ml in 50 mM Tris, pH 9,5) 1 Stunde bei Raumtemperatur inkubiert. Nach Entfernen der Antikörperlösung wurde einmal mit PBS gewaschen.
2.2 150 µl/well eines Blockierungspuffers (1 % BSA in PBS) wurde 0,5 Stunden bei Raumtemperatur auf den Platten inkubiert. Nach Entfernen des Biockierungspuffers wurde einmal mit PBS gewaschen.
2.3 100 µl pro well eines Zellkulturüberstandes von transfektierten COS-Zellen wurden für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem IgG₁ (hVCAM-1(1-3)-IgG), codiert. Der Gehalt an hVCAM-1(1-3)-IgG betrug ca. 0,5 - 1 µg/ml. Nach Entfernen des Kulturüberstandes wurde einmal mit PBS gewaschen.
2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
2.5 20 µl Bindungspuffer (100 mM NaCl, 100 µM M₉Cl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
2.6 U937-Zellen wurden 20 Minuten in Fc-Rezeptor-Blockpuffer inkubiert und anschließend in einer Konzentration von 1 x 10⁶/ml und in einer Menge von 100 µl pro well zupipettiert (Endvolumen 125µl/well).
2.7 Die Platten wurden in einem 45°-Winkel in Stoppuffer (100 mM NaCl, 100 µM M₉Cl₂, 100 µM MnCl₂, 100 µM CaCl₂ in 25 mM Tris, pH 7,5) langsam eingetaucht und ausgeschlagen. Der Vorgang wurde wiederholt.
2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
2.9 Die Platten wurden ausgeschlagen, in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurde mit der Flüssigkeit in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50 % führt.

Es wurden die folgenden Testergebnisse erhalten:

| Beispiel | U937NCAM-1 Zelladhäsionstest IC₅₀ (µM) |
|---|---|
| 4 | 45 |
| 7 | 8 |
| 8 | 4,5 |
| 9 | 4 |
| 10 | 9,5 |

## Patentansprüche

1. Verbindung der Formel I, 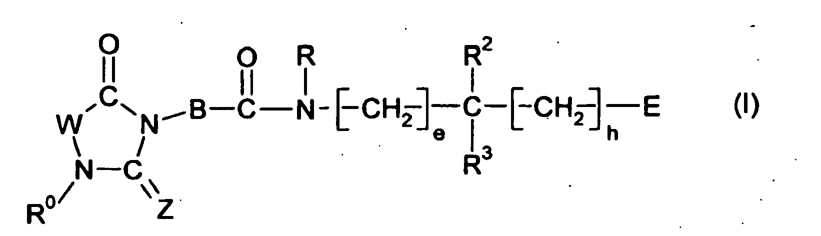 worin
W für R¹-A-C(R¹³) steht;
Z für Sauerstoff steht;
A für eine direkte Bindung steht;
B für einen unsubstituierten Methylenrest oder für einen Methylenrest, der durch einen (C₁-C₈)-Alkylrest substituiert ist, steht;
E R¹⁰CO bedeutet;
R Wasserstoff oder (C₁-C₄)-Alkyl bedeutet;
R⁰ für im Arylrest gegebenenfalls substituiertes (C₆-C₁₄)-Aryl-(C₁-C₄)-alkyl steht;
R¹ für einen unsubstituierten Rest aus der Reihe Phenyl, 2-Thienyl, 3-Thienyl, 3- Pyridyl und 4-Pyridyl steht;
R² Wasserstoff oder (C₁-C₄)-Alkyl bedeutet;
R³ für einen unsubstituierten Phenylrest oder einen durch einen, zwei oder drei gleiche oder verschiedene Reste aus der Reihe (C₁-C₄)-Alkyl, (C₁-C₄)-Alkoxy, Hydroxy, Halogen. Trifluormethyl, Nitro, Methylendioxy, Ethylendioxy und Cyan substituierten Phenylrest steht, oder R³ für R¹¹NH, CON(CH₃)R⁴ oder CONHR⁴ steht;
R⁴ (C₁-C₆)-Alkyl bedeutet, das durch einen oder zwei gleiche oder verschiedene Reste aus der Reihe Hydroxy, (C₁-C₈)-Alkoxy, Phenyl, Benzyl, Hydroxycarbonyl, Aminocarbonyl und (C₁-C₆)-Alkoxycarbonyl substituiert ist;
R¹⁰ Hydroxy, (C₁-C₈)-Alkoxy, (C₆-C₁₀)-Aryl-(C₁-C₄)-alkoxy, das im Arylrest auch substituiert sein kann, gegebenenfalls substituiertes (C₆-C₁₀)-Aryloxy, (C₁-C₈)- Alkylcarbonyloxy-(C₁-C₄)-alkoxy, (C₆-C₁₀)-Arylcarbonyloxy-(C₁-C₄)-alkoxy, Amino oder Mono- oder Di-((C₁-C₈)-alkyl)-amino bedeutet;
R¹¹ für R^{12a}-CO, R^{12a}-O-CO oder R^{12a}-NH-CO steht;
R^{12a} (C₁-C₁₀)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁**-**C₈)-alkyl oder den Rest R¹⁵ bedeutet;
R¹³ Wasserstoff oder (C₁-C₄)-Alkyl bedeutet;
R¹⁵ für R¹⁶-(C₁-C₃)-alkyl oder für R¹⁶ steht;
R¹⁶ für-einen 2-Norbornylrest, 2-Bicyclo[3.2.1]octylrest, Adamantylrest, Homoadamantylrest oder Noradamantylrest steht;
e und h unabhängig voneinander für 0 oder 1 stehen;
in allen ihren stereoisomeren Formen und Mischungen davon in allen Verhältnissen, und ihre physiologisch verträglichen Salze.

2. Verbindung der Formel I gemäß Anspruch 1, worin R¹ für einen unsubstituierten Rest aus der Reihe Phenyl, 2-Thienyl, 3-Thienyl und 4-Pyridyl steht, in allen ihren stereoisomeren Formen und Mischungen davon in allen Verhältnissen, und ihre physiologisch verträglichen Salze.

3. Verbindung der Formel **I** gemäß Anspruch 1 und/oder 2, worin R¹³ für (C₁-C₄)-Alkyl steht, in allen ihren stereoisomeren Formen und Mischungen davon in allen Verhältnissen, und ihre physiologisch verträglichen Salze.

4. Verfahren zur Herstellung einer Verbindung der Formel **I** gemäß einem oder mehreren der Ansprüche 1 bis 3, **dadurch gekennzeichnet, daß** man eine Fragmentkondensation einer Verbindung der Formel II 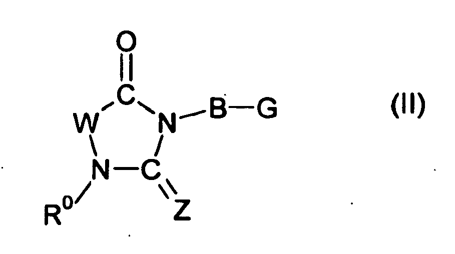 mit einer Verbindung der Formel III, 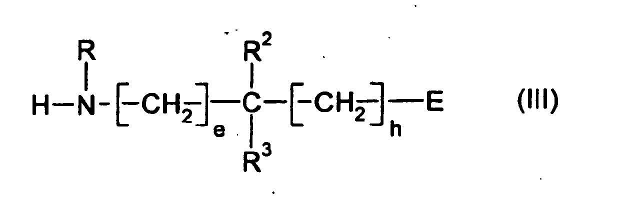 durchführt, wobei in den Formeln II und III die Gruppen W, Z, B, E, R, R⁰, R² und R³ sowie e und h wie in den Ansprüchen 1 bis 3 definiert sind oder auch funktionelle Gruppen in geschützter Form oder in Form von Vorstufen enthalten sein können, und wobei G für Hydroxycarbonyl, (C₁-C₆)-Alkoxycarbonyl oder ein aktiviertes Carbonsäurederivat steht.

5. Verbindung der Formel I gemäß einem oder mehreren der Ansprüche 1 bis 3 und/oder ein physiologisch verträgliches Salz davon zur Verwendung als Arzneimittel.

6. Pharmazeutisches Präparat, **dadurch gekennzeichnet, daß** es eine oder mehrere Verbindungen der Formel I gemäß einem oder mehreren der Ansprüche 1 bis 3 und/oder ihre physiologisch verträgliche Salze neben pharmazeutisch einwandfreien Trägerstoffen und/oder Zusatzstoffen enthält.

7. Verbindung der Formel I gemäß einem oder mehreren der Ansprüche 1 bis 3 und/oder ein physiologisch verträgliches Salz davon zur Verwendung als Entzündungshemmstoff.

8. Verbindung der Formel I gemäß einem oder mehreren der Ansprüche 1 bis 3 und/oder ein physiologisch verträgliches Salz davon zur Verwendung in der Therapie oder Prophylaxe der rheumatoiden Arthritis, der inflammatorischen bowel disease, des systemischen Lupus erythematosus oder von inflammatorischen Erkrankungen des zentralen Nervensystems.

9. Verbindung der Formel I gemäß einem oder mehreren der Ansprüche 1 bis 3 und/oder ein physiologisch verträgliches Salz davon zur Verwendung in der Therapie oder Prophylaxe von Asthma oder Allergien.

10. Verbindung der Formel I gemäß einem oder mehreren der Ansprüche 1 bis 3 und/oder ein physiologisch verträgliches Salz davon zur Verwendung in der Therapie oder Prophylaxe von cardiovaskulären Erkrankungen, der Arteriosklerose, von Restenosen oder von Diabetes, zur Verhinderung der Schädigung von Organtransplantaten, zur Hemmung von Tumorwachstum oder Tumormetastasierung oder zur Therapie der Malaria.

11. Verbindung der Formel I gemäß einem oder mehreren der Ansprüche 1 bis 3 und/oder ein physiologisch verträgliches Salz davon zur Verwendung als Hemmstoff der Adhäsion und/oder der Migration von Leukozyten oder als Hemmstoff des VLA-4-Rezeptors.

## Claims

1. A compound of the formula I, 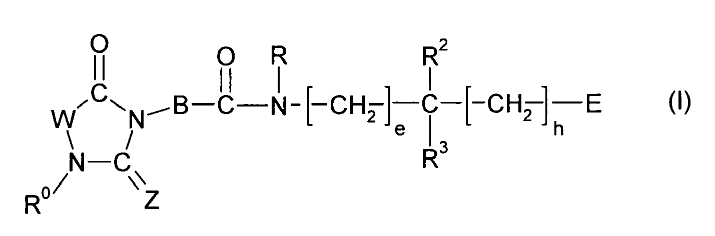 in which
W is R¹-A-C(R¹³);
Z is oxygen;
A is a direct bond;
B is an unsubstituted methylene radical or a methylene radical which is substituted by a (C₁-C₈)-alkyl radical;
E is R¹⁰CO;
R is hydrogen or (C₁-C₄)-alkyl;
R⁰ is (C₆-C₁₄)-aryl-(C₁-C₄)-alkyl optionally substituted in the aryl radical;
R¹ is an unsubstituted radical from the group consisting of phenyl, 2-thienyl, 3- thienyl, 3-pyridyl and 4-pyridyl;
R² is hydrogen or (C₁-C₄)-alkyl;
R³ is an unsubstituted phenyl radical or a phenyl radical which is substituted by one, two or three identical or different radicals from the group consisting of (C₁-C₄)-alkyl, (C₁-C₄)-alkoxy, hydroxy, halogen, trifluoromethyl, nitro, methylenedioxy, ethylenedioxy and cyano, or R³ is R¹¹NH, CON(CH₃)R⁴ or CONHR⁴;
R⁴ is (C₁-C₆)-alkyl which is substituted by one or two identical or different radicals from the group consisting of hydroxy, (C₁-C₈)-alkoxy, phenyl, benzyl, hydroxycarbonyl, aminocarbonyl and (C₁-C₆)-alkoxycarbonyl;
R¹⁰ is hydroxy, (C₁-C₈)-alkoxy, (C₆-C₁₀)-aryl-(C₁-C₄)-alkoxy which can also be substituted in the aryl radical, optionally substituted (C₆-C₁₀)-aryloxy, (C₁-C₈)- alkylcarbonyloxy-(C₁-C₄)-alkoxy, (C₆-C₁₀)-arylcarbonyloxy-(C₁-C₄)-alkoxy, amino or mono- or di-((C₁-C₈)-alkyl)-amino;
R¹¹ is R^{12a}-CO, R^{12a}-O-CO or R^{12a}-NH-CO;
R^{12a} is (C₁-C₁₀)-alkyl, (C₃-C₁₂)-cycloalkyl, (C₃-C₁₂)-cydoalkyl-(C₁-C₈)-alkyl, optionally substituted (C₆-C₁₄)-aryl, (C₆-C₁₄)-aryl-(C₁-C₈)-alkyl optionally substituted in the aryl radical, or the radical R¹⁵;
R¹³ is hydrogen or (C₁-C₄)-alkyl;
R¹⁵ is R¹⁶-(C₁-C₃)-alkyl or R¹⁶;
R¹⁶ is a 2-norbornyl, 2-bicyclo[3.2.1]octyl, adamantyl, homoadamantyl or noradamantyl radical;
e and h independently of one another are 0 or 1;
in all its stereoisomeric forms and mixtures thereof in all ratios, and its physiologically tolerable salts.

2. A compound of the formula I as claimed in claim 1, in which R¹ is an unsubstituted radical from the group consisting of phenyl, 2-thienyl, 3-thienyl and 4-pyridyl, in all its stereoisomeric forms and mixtures thereof in all ratios, and its physiologically tolerable salts.

3. A compound of the formula I as claimed in claim 1 and/or 2, in which R¹³ is (C₁-C₄)-alkyl, in all its stereoisomeric forms and mixtures thereof in all ratios, and its physiologically tolerable salts.

4. A process for the preparation of a compound of the formula I as claimed in one or more of claims 1 to 3, which comprises carrying out a fragment condensation of a compound of the formula II 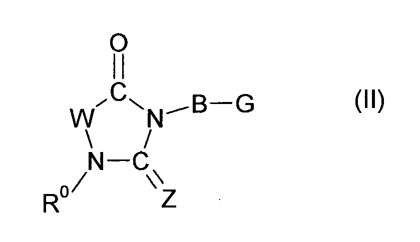 with a compound of the formula III, 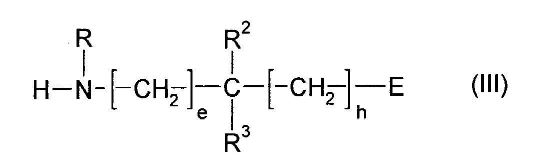 where, in the formulae II and III, the groups W, Z, B, E, R, R⁰, R² and R³ as well as e and h are as defined in claims 1 to 3 or alternatively functional groups can be contained in protected form or in the form of precursors, and where G is hydroxycarbonyl, (C₁-C₆)-alkoxycarbonyl or an activated carboxylic acid derivative.

5. A compound of the formula I as claimed in one or more of claims 1 to 3 and/or a physiologically tolerable salt thereof for use as a pharmaceutical.

6. A pharmaceutical preparation which contains one or more compounds of the formula I as claimed in one or more of claims 1 to 3 and/or its physiologically tolerable salts in addition to pharmaceutically innocuous excipients and/or additives.

7. A compound of the formula I as claimed in one or more of claims 1 to 3 and/or a physiologically tolerable salt thereof for use as an antiinflammatory.

8. A compound of the formula I as claimed in one or more of claims 1 to 3 and/or a physiologically tolerable salt thereof for use in the therapy or prophylaxis of rheumatoid arthritis, of inflammatory bowel disease, of systemic lupus erythematosus or of inflammatory disorders of the central nervous system.

9. A compound of the formula I as claimed in one or more of claims 1 to 3 and/or a physiologically tolerable salt thereof for use in the therapy or prophylaxis of asthma or allergies.

10. A compound of the formula I as claimed in one or more of claims 1 to 3 and/or a physiologically tolerable salt thereof for use in the therapy or prophylaxis of cardiovascular disorders, arteriosclerosis, of restenoses or of diabetes, for the prevention of damage to organ transplants, for the inhibition of tumor growth or formation of tumor metastases or for the therapy of malaria.

11. A compound of the formula I as claimed in one or more of claims 1 to 3 and/or a physiologically tolerable salt thereof for use as an inhibitor of the adhesion and/or migration of leucocytes or as an inhibitor of the VLA-4 receptor.

## Revendications

1. Composé de formule I 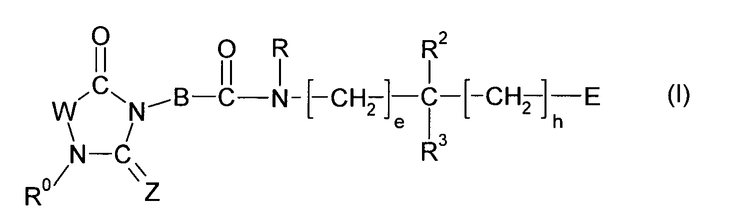 où
W représente R¹-A-C(R¹³) ;
Z représente oxygène ;
A représente une liaison directe ;
B représente un radical méthylène non substitué ou un radical méthylène, qui est substitué par un radical (C₁-C₈)-alkyle ;
E signifie R¹⁰CO ;
R signifie hydrogène ou (C₁-C₄)-alkyle ;
R⁰ représente un radical (C₆-C₁₄)-aryl-(C₁-C₄)-alkyle éventuellement substitué dans le radical aryle ;
R¹ représente un radical non substitué dans la série phényle, 2-thiényle, 3- thiényle, 3-pyridyle et 4-pyridyle ;
R² signifie hydrogène ou (C₁-C₄)-alkyle ;
R³ représente un radical phényle non substitué ou un radical phényle substitué par un, deux ou trois radicaux, identiques ou différents de la série (C₁-C₄)- alkyle, (C₁-C₄)-alcoxy, hydroxy, halogène, trifluorométhyle, nitro, méthylènedioxy, éthylènedioxy et cyano, ou R³ représente R¹¹NH, CON(CH₃)R⁴ ou CONHR⁴ ;
R⁴ signifie (C₁-C₆)-alkyle, qui est substitué par un ou deux radicaux identiques ou différents de la série hydroxy, (C₁-C₈)-alcoxy, phényle, benzyle, hydroxycarbonyle, aminocarbonyle et (C₁-C₆)-alcoxycarbonyle ;
R¹⁰ signifie hydroxy, (C₁-C₈)-alcoxy, (C₆-C₁₀)-aryl-(C₁-C₄)-alcoxy, qui peut être substitué dans le radical aryle, (C₆-C₁₀)-aryloxy éventuellement substitué, (C₁- C₈)-alkylcarbonyloxy-(C₁-C₄)-alcoxy, (C₆-C₁₀)-arylcarbonyloxy-(C₁-C₄)-alcoxy, amino ou mono-((C₁-C₈)-alkyl)-amino ou di-((C₁-C₈)-alkyl)-amino ;
R¹¹ représente R^{12a}-CO, R^{12a}-O-CO ou R^{12a}-NH-CO ;
R^{12a} signifie (C₁-C₁₀)-alkyle, (C₃-C₁₂)-cycloalkyle, (C₃-C₁₂)-cycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle ou le radical R¹⁵ ;
R¹³ signifie hydrogène ou (C₁-C₄)-alkyle ;
R¹⁵ représente R¹⁶-(C₁-C₃)-alkyle ou R¹⁶ ;
R¹⁶ représente un radical 2-norbornyle, 2-bicyclo[3.2.1]octyle, adamantyle, homoadamantyle ou noradamantyle ;
e et h représentent, indépendamment l'un de l'autre, 0 ou 1 ;
dans toutes ses formes stéréo-isomères et leurs mélanges dans tous les rapports, et ses sels physiologiquement acceptables.

2. Composé de formule I selon la revendication 1, où R¹ représente un radical non substitué de la série phényle, 2-thiényle, 3-thiényle et 4-pyridyle, dans toutes ses formes stéréo-isomères et leurs mélanges dans tous les rapports, et ses sels physiologiquement acceptables.

3. Composé de formule I selon la revendication 1 et/ou 2, où R¹³ représente (C₁-C₄)-alkyle, dans toutes ses formes stéréo-isomères et leurs mélanges dans tous les rapports, et ses sels physiologiquement acceptables.

4. Procédé pour la préparation d'un composé de formule I selon l'une ou plusieurs des revendications 1 à 3, **caractérisé en ce qu'**on réalise une condensation fragmentaire d'un composé de formule II 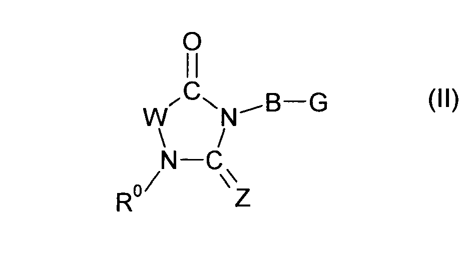 avec un composé de formule III 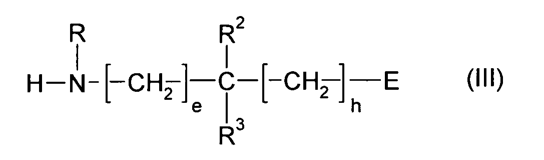 où, dans les formules II et III, les groupes W, Z, B, E, R, R⁰, R² et R³ ainsi que e et h sont définis comme dans les revendications 1 à 3 ou des groupes fonctionnels peuvent également être contenus sous forme protégée ou sous forme de précurseurs et où G représente hydroxycarbonyle, (C₁-C₆)-alcoxycarbonyle ou un dérivé activé d'acide carboxylique.

5. Composé de formule I selon l'une ou plusieurs des revendications 1 à 3 et/ou un sel physiologiquement acceptable de celui-ci destiné à une utilisation comme médicament.

6. Préparation pharmaceutique, **caractérisée en ce qu'**elle contient un ou plusieurs composés de formule I selon l'une ou plusieurs des revendications 1 à 3 et/ou ses/leurs sels physiologiquement acceptables, en plus de substances support et/ou d'additifs pharmaceutiquement irréprochables.

7. Composé de formule I selon l'une ou plusieurs des revendications 1 à 3 et/ou un sel physiologiquement acceptable de celui-ci destiné à une utilisation comme anti-inflammatoire.

8. Composé de formule I selon l'une ou plusieurs des revendications 1 à 3 et/ou un sel physiologiquement acceptable de celui-ci destiné à une utilisation dans la thérapie ou la prophylaxie de la arthrite rhumatoïde, de l'affection abdominale inflammatoire, du lupus érythémateux systémique ou de maladies inflammatoires du système nerveux central.

9. Composé de formule I selon l'une ou plusieurs des revendications 1 à 3 et/ou un sel physiologiquement acceptable de celui-ci destiné à une utilisation dans la thérapie ou la prophylaxie de l'asthme ou d'allergies.

10. Composé de formule I selon l'une ou plusieurs des revendications 1 à 3 et/ou un sel physiologiquement acceptable de celui-ci destiné à une utilisation dans la thérapie ou la prophylaxie de maladies cardiovasculaires, de l'artériosclérose, de resténoses ou du diabète, à empêcher la dégradation de transplants d'organes, à l'inhibition de la croissance de tumeurs ou de la formation de métastases de tumeurs ou à la thérapie de la malaria.

11. Composé de formule I selon l'une ou plusieurs des revendications 1 à 3 et/ou un sel physiologiquement acceptable de celui-ci destiné à une utilisation comme inhibiteur de l'adhérence et/ou de la migration de leucocytes ou comme inhibiteur du récepteur de VLA-4.
